(19) Europäisches Patentamt
European Patent Office
Office européen des brevets

(11) EP 4 434 974 A1

(12) EUROPEAN PATENT APPLICATION
published in accordance with Art. 153(4) EPC

(43) Date of publication:
**25.09.2024 Bulletin 2024/39**

(21) Application number: **22894177.9**

(22) Date of filing: **05.05.2022**

(51) International Patent Classification (IPC):
***C07D 307/20*** *(2006.01)* ***C07C 271/24*** *(2006.01)*
***C07D 295/182*** *(2006.01)* ***C07C 271/34*** *(2006.01)*
***C07D 265/32*** *(2006.01)* ***A61P 29/00*** *(2006.01)*

(86) International application number:
**PCT/CN2022/090947**

(87) International publication number:
**WO 2023/087632 (25.05.2023 Gazette 2023/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.11.2021 CN 202111365730**
**28.03.2022 CN 202210311555**

(71) Applicant: **Jiangsu Atom Bioscience and Pharmaceutical Co., Ltd.**
**Suzhou City, Jiangsu Province, 215123 (CN)**

(72) Inventors:
- **SHI, Dongfang**
  **Suzhou, Jiangsu Province 215123 (CN)**
- **FU, Changjin**
  **Suzhou, Jiangsu Province 215123 (CN)**
- **YANG, Yan**
  **Suzhou, Jiangsu Province 215123 (CN)**
- **LI, Haiming**
  **Suzhou, Jiangsu Province 215123 (CN)**

(74) Representative: **Cabinet Beau de Loménie**
**158, rue de l'Université**
**75340 Paris Cedex 07 (FR)**

# (54) ANTI-INFLAMMATORY ANALGESIC COMPOUNDS AND USE THEREOF

(57) The invention belongs to the field of medicinal chemistry, in particular discloses anti-inflammatory and analgesic compounds and use thereof. The compounds of the present invention are compounds, isomers or pharmaceutically acceptable salts as shown in formula (1) or formula (II). The compounds have significant curative effect on gouty arthritis in rats, and have excellent anti-inflammatory factor effect. Therefore, the compouds have potential application prospects in anti-inflammatory and analgesic drugs, especially in anti-acute gouty arthritis drugs, anti-rheumatoid arthritis drugs, drugs to reduce inflammatory factors, drugs to treat inflammatory storm or coronavirus pneumonia.

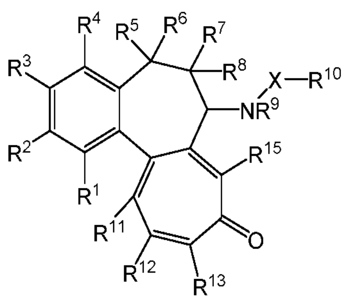

( I )

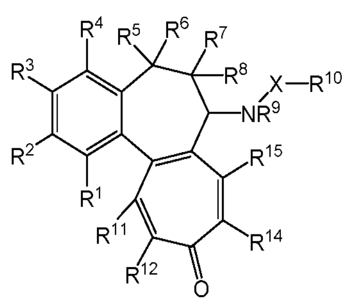

( II )

EP 4 434 974 A1

## Description

### Technical field

The invention belongs to the field of medicinal chemistry, in particular anti-inflammatory and analgesic compounds and use thereof.

### Background art

In recent years, with the continuous improvement of living standards, people's diet has also changed, and the number of gout patients has increased significantly. Gout has become the second largest metabolic disease after diabetes and has been listed by the United Nations as one of the twenty biggest diseases of the 21st century. According to the National Health and Nutrition Examination Survey, between 2007 and 2008, the prevalence of gout in the U.S. adult population was 3.9% (about 8.3 million) (Zhu Y, Pandya BJ, Choi HK. Prevalence of Gout and Hyperuricemia in the US General Population: The National Health and Nutrition Examination Survey 2007-2008[J]. Arthritis Rheum, 2011, 63(10): 3136-3141); The overall prevalence of hyperuricemia in China was 13.3% and that of gout was 1.1% (Liu R, Han C, Wu D, et al. Prevalence of hyperuricemia and gout in mainland China from 2000 to 2014: A systematic review and meta-analysis[J]. Biomed Research International, 2015: 1-12). In the past few decades, due to the prevalence of co-existing diseases that can promote hyperuricemia (e.g., hypertension, obesity, metabolic syndrome, type 2 diabetes, chronic kidney disease), the incidence of gout has also gradually increased (Khanna D, Fitzgerald JD, Khanna PP, et al. American College of Rheumatology Guidelines for Management of Gout. Part 1: Systematic Nonpharmacologic and Pharmacologic Therapeutic Approaches to Hyperuricemia[J]. Arthritis Care & Research, 2012, 64(10): 1432-1446).

Gout is caused by chronic hyperuricemia. Uric acid is the end product of human purine metabolism, and has poor solubility. Most uric acid in the body is excreted from the urine through the kidneys. The metabolism disorder in the body, or long-term intake of too much high-purine food leads to excessive uric acid production in the body. Human urate anion transporter 1 (URAT1) on the cell membrane of the kidney epithelium controls the reabsorption of most uric acid in the kidney. When there is a problem with uric acid transport and excretion system in the kidney, URAT1 reabsorbs excess uric acid into the body, causing the blood uric acid concentration to rise. 80-85% of patients with gout and hyperuricemia are caused by uric acid excretion disorder by the kidneys.

When the blood uric acid concentration in the human body is greater than 6.8-7.0 mg/dL and the uric acid concentration exceeds the maximum dissolution capacity in the blood, the blood uric acid concentration reaches saturation. Urate, which will produce crystalline deposits in synovial fluid of human tissues, cartilage of peripheral joints, pinna of ear, olecranon bursae of elbow, tendons, kidneys, etc. (Richette P, Bardin T. Gout. Lancet. 2010, 375(9711): 318-328), can lead to repeated inflammatory arthritis, produce gout flares, eventually will evolve into serious chronic joint disease, and even bone corrosion (Schlesinger N. Difficult-to-treat gouty arthritis: a disease warranting better management. Drugs, 2011, 71(11):1413-1439). When urate crystals form and deposit in the subcutaneous tissue, gouty tophi are formed, which can break the human epidermal tissue, and cause serious pains during acute attacks, and even cause infection. Gout can cause urate nephropathy and urate urinary calculi, as well as renal insufficiency. Gout is a serious condition that leads to a decrease in patient quality of life and an increase in medical costs.

When urate concentration in joint cavity of patients with gout changes dramatically, urate microcrystals will form, synovial endothelial cells will be activated to cause macrophage adhesion and exudation and secrete inflammatory factors to induce immune response after phagocytosis of urate microcrystals, which is called acute gouty arthritis. Acute gouty arthritis is the most common first symptom of gout, its typical attack is generally more sudden, sometimes violent, mostly in the middle of the night, leading to waking with foot pain, the pain feels like knife cutting and biting, while the joint and surrounding tissues appear reddness, swollenness, heat, pain and other symptoms. Most acute gouty arthritis only affects a single joint when it first occurs, of which the first metatarsophalangeal joint is the most common, followed by the dorsum of foot, heel, ankle, knee, wrist and elbow joints, and sometimes polyarthritis may occur simultaneously.

At present, there is no good treatment for acute gouty arthritis. The purpose of acute gouty arthritis treatment is to quickly control the symptoms of acute arthritis.

The guidelines and consensus recommendations for anti-inflammatory and analgesic treatment of acute attacks of gout are similar in different countries. During acute attacks of gout, it is recommended to use small doses of colchicine or non-steroidal anti-inflammatory drugs (NSAID) as soon as possible (sufficient amount, short course), and systemic glucocorticoids are recommended for patients with intolerance, poor efficacy or contraindications of these drugs. Selective cyclooxygenase-2 (COX-2) inhibitors are recommended for patients at risk of gastrointestinal bleeding or in need of long-term use of low-dose aspirin. For patients with acute gout involving multiple joints, large joints or combined with systemic symptoms, it is recommended to prioritize systemic glucocorticoid treatment. For patients with visual analogue pain scale (VAS) score ≥7, or two and more large joints involved, or polyarthritis, or poor response to one drug, it is recommended to combine two anti-inflammatory analgesic drugs, such as low-dose colchicine with NSAID or low-dose

colchicine with systemic glucocorticoids (Chinese Society of Endocrinology. Guidelines for the diagnosis and management of hyperuricemia and gout in China 2019. Chinese Journal Endocrinology and Metabolism, 2019, 36(1).

Colchicine is the first drug used for the anti-inflammatory and analgesic treatment of acute gouty arthritis, and is still the first-line drug for acute attacks of gout. Colchicine can (1) inhibit the accumulation of white blood cells in the inflammation site, weaken the effect of white blood cells phagocytosis of uric acid, reduce the reaction of acute gout, and achieve the purpose of rapid pain relief; (2) inhibit cell mitosis, by binding with tubulin, prevent microtubule formation, inhibit phospholipase A, and inhibit tyrosine phosphorylation and the production of leukotriene B4, so as to achieve anti-inflammatory and pain relief purposes; (3) inhibit local cells to produce interleukin-6 (IL-6), etc., so as to control inflammatory reactions such as local joint redness, swellenness, heat and pain. In the acute phase of gout, patients are traditionally given 0.5-1 mg of colchicine orally every 1-2 hours until joint symptoms are relieved or adverse reactions such as diarrhea and vomiting occur. The therapeutic dose is generally 3-5 mg, and should not exceed 6 mg within 24 hours, and dose per day is 0.5-1.5 mg 72 hours after drug withdrawal, and separate doses are required for 7 days. The therapeutic dose of colchicine is close to the toxic dose. It is generally believed that a dose of colchicine is toxic to the body at 0.5-0.8 mg/kg, and a dose higher than 0.8 mg/kg will be fatal, and there are reports of death cases caused 35 h after ingestion of 0.4 mg/kg colchicine abroad. There are a lot of clinical adverse reactions of colchicine, which was significantly correlated with the dose. Common gastrointestinal reactions such as nausea, vomiting, diarrhea, and abdominal pain are precursors to severe poisoning and colchicine administration should be discontinued immediately when these symptoms occur. Colchicine can also cause kidney damage, manifested as hematuria, oliguria; and it has a direct inhibitory effect on bone marrow and can cause agranulocytogenous aplastic anemia, and should be used with caution. Dose of colchicine should be reduced in patients with moderate to severe renal impairment (Keenan RT, O'Brien WR, Lee KH, et al. Prevalence of contraindications and prescription of pharmacologic therapies for gout[J]. American Journal of Medicine, 2011, 124(2): 155-163). Because acute gouty arthritis occurs frequently in patients with gout and requires long-term medication, the toxic side effects of colchicine often limit its use in this area of the disease. NSAID and COX-2 are also first-line drugs for acute gouty arthritis, but these drugs also have many toxic side effects, among which NSAID mainly causes gastrointestinal toxicity, such as gastrointestinal ulcer, gastrointestinal perforation and upper gastrointestinal bleeding. Glucocorticoids are mainly used for acute gout accompanied by severe acute attacks with severe systemic symptoms that do not respond to colchicine or NSAID treatment. Chinese gout guidelines recommend glucocorticoids as second-line analgesics, systemic glucocorticoid therapy is recommended only when acute gout attacks involve multiple joints, large joints, or systemic symptoms occur (Khanna D, Khanna PP, Fitzgerald JD, et al. 2012 American College of Rheumatology guidelines for management of gout. Part 2: therapy and antiinflammatory prophylaxis of acute gouty arthritis [J]. Arthritis care & research, 2012, 64(10): 1447-1461).

Inflammatory cytokines play an important role in the pathogenesis of gout and can be used as an auxiliary index for clinical diagnosis of acute gouty arthritis. Among them, IL-1$\beta$, IL-6 and tumor necrosis factor-$\alpha$ (TNF-$\alpha$) are the main factors inducing acute gouty arthritis, and play an important role in its pathogenesis. Some scholars suggest that IL-1$\beta$ and IL-6 should be used as indicators to evaluate the degree of inflammation in acute gout. Wang Siqi et al. evaluated the pain degree of 72 patients with acute gout who were treated in the gout outpatient department of Zhejiang Dermatology Hospital from January 2017 to December 2018 by using visual analog score method, and measured the expression levels of serum IL-1$\beta$, IL-6, TNF-$\alpha$, etc., and analyzed their correlation. The results showed that, serum IL-1$\beta$ and IL-6 levels have a good correlation with pain severity (Wang Siqi, Yu Bin, Zhang Xiaohai et al. Correlation analysis of pain and inflammatory factors in acute gout patients [J]. Medical Theory & Practice, 2021, 34(3): 371-373). In recent years, IL-1 antagonists have been gradually used in the treatment and prevention of acute gouty arthritis. The IL-1 antagonists that have been approved internationally for rheumatic diseases mainly include Anakinra, Canakinumab and Rilonacept, none of which have been marketed in China. In 2011 and 2012, the ACR recommended Anakinra and Canakinumab for the treatment of severe acute gouty arthritis. In 2013, Canakinumab was approved by the European Medicines Agency for use in gout where intolerance or contrainidations to conventional anti-inflammatory analgesics exist. TNF-$\alpha$ is also an important inflammatory factor involved in the occurrence and development of rheumatoid arthritis. TNF-$\alpha$ and its receptors are widely distributed in the serum and synovial fluid of patients with rheumatoid arthritis. TNF-$\alpha$ can inhibit important proteins in the inflammatory signaling pathway, manifested as promoting inflammatory response, and TNF-$\alpha$ inhibitors are also used in the clinical treatment of rheumatoid arthritis, such as adalimumab.

Inflammatory cytokines also play an important role in the development of the novel coronavirus pneumonia (COVID-19). In the clinic, it was found "inflammatory storm" may be a key factor in the sudden aggravation of disease induced by the new coronavirus. Inflammatory storm, also known as cytokine storm, is mainly due to the rapid release of a variety of cytokines such as IL-6, IL-1, IL-8, IFN, TNF-$\alpha$, etc., after immune system disorders, leading to extensive edema of lung tissue, causing acute lung injury, acute respiratory distress syndrome, multiple organ dysfunction syndrome, and even death (He Y, Gan W, Zhang M, et al. Evaluation of clinical biochemical indexes in the progression and treatment of COVID-19[J]. Journal of Laboratory Medicine, 2020, 41: 906-909). The symptom of "inflammatory storm" is actualy a key node of patients from mild to severe and critical illness, and is also a cause of death in severe and critical patients (Zhang Ximu, Li Tanshi, Cytokine storm and immunotherapy in post-influenza pneumonia [J]. Medical Review,

2020, 26(6): 1142-1146).

There is growing evidence that inflammatory storms are involved in the pathogenesis of COVID-19. Studies have found that in COVID-19 patients with severe hypoxia, serum cytokines such as IL-6 were significantly increased (Wilk AJ, Rustagi A, Zhao NQ, et al. A single cell atlas of the peripheral immune response in patients with severe COVID-19[J]. Nature medicine, 2020, 26(7): 1070-1076). HUANG et al. collected and analyzed the case data of 41 hospitalized patients with COVID-19 and found that the plasma levels of IL-2, IL-7, IL-10, granulocyte colony-stimulating factor, chemokine and TNF-$\alpha$ in ICU patients were higher than those in other patients (Huang C, Wang Y, Li X, et al. Clinical features of patients infected with 2019 novel coronavirus in Wuhan, China[J]. Lancet, 2020, 395(10223): 497-506). In conclusion, anti-inflammatory therapy may be an effective way to curb the inflammatory storm and prevent COVID-19.

There is no specific treatment for inflammatory storm in clinic, and the combination treatment of antiviral, glucocorticoid, targeted immunotherapy and traditional Chinese medicine is usually used. The most commonly used and effective drugs to directly reduce inflammatory storms are still glucocorticoids, which is the most commonly used immunomodulatory drugs that regulate immune activity and reduce inflammation in a variety of major diseases. But there is currently no clear evidence to support that patients with COVID-19 will benefit from glucocorticoid therapy, while patients with COVID-19 are more likely to be at risk from glucocorticoid therapy (Clark DR, Jonathan EM, Kenneth JB, et al. Clinical evidence does not support corticosteroid treatment for 2019-nCoV lung injury[J]. Lancet, 2020, 395: 473-475). Therefore, glucocorticoids should not be used to treat lung injury or shock caused by COVID-19 in the context of non-clinical trials. In addition to glucocorticoids, there are a variety of drugs such as immunosuppressants that can be used to treat inflammatory storms.

Colchicine has recently been reported to have positive effects in COVID-19 treatment trials and could be an effective oral drug against COVID-19. Because colchicine can not only inhibit the function of neutrophils, but also reduce the expression level of some cellular inflammatory factors. After novel coronavirus infection, these cellular inflammatory factors have increased, and "immune cell storm" caused by inhibiting these factors is also a current research direction for the treatment of novel coronavirus pneumonia. There are currently several ongoing clinical trials using colchicine to treat COVID-19 around the world. The Montreal Heart Institute in Canada released a news showing that colchicine has shown a "convincing" effect on the treatment of novel coronavirus pneumonia in clinical trials conducted. In the study, 4,488 patients with novel coronavirus infection who had not yet been hospitalized were enrolled, patients treated with colchicine had a 21% lower risk of hospitalization or death compared with those in the placebo group. A nasopharyngeal nucleic acid test of 4,159 patients found that colchicine reduced hospital admissions by 25%, the need for respiratory equipment by 50% and the number of deaths by 44% (Tardif JC, Bouabdallaoui N, L 'Allier PL, et al. Efficacy of Colchicine in Non-Hospitalized Patients with COVID-19[J]. MedRxiv, 2021).

Following the results of the study, the Expert Committee of the Greek Ministry of Health announced in early 2021 that it approved colchicine for inclusion in the oral drug regimen for patients with novel coronavirus. However, due to the toxic side effects of colchicine, the use need to be cautious.

In recent years, targeted therapy for a specific cytokine in the inflammatory storm has been applied to the clinic, although it has significant clinical effects, but the side effect of the therapy should not be underestimated, that is, cytokine release syndrome, which is a systemic immune storm involving activated immune cells to release a large number of cytokines, resulting in multiple system damage and even death.

## Summary of invention

One object of the invention is to provide a class of compounds with novel structure and anti-inflammatory and analgesic effects. These compounds can effectively relieve the symptoms of gouty arthritis, inhibit the level of inflammatory factors in the knee tissue, and have a good anti-inflammatory effect, which can be applied in the treatment of a variety of inflammation-related diseases.

The technical solution of the invention is as follows:

Compounds, isomers or pharmaceutically acceptable salts of the structure shown in formula (I) or formula (II),

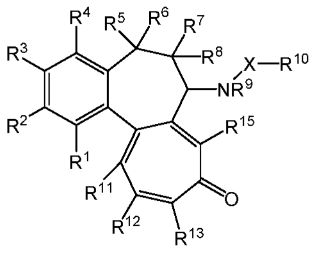

( I )

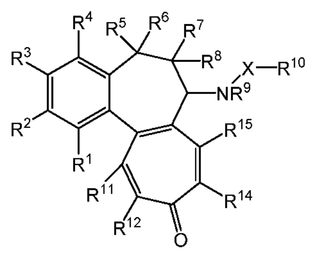

( II )

Wherein,

$R^1$, $R^2$, $R^3$, or $R^4$ are independently hydrogen, deuterium, halogen, cyano, $C_{1-3}$ alkoxy, hydroxyl, aldehyde group, carboxyl, $C_{2-6}$ ester, amide, substituted amide, $C_{1-3}$ alkyl, substituted $C_{1-3}$ alkoxy, or substituted $C_{1-3}$ alkyl, respectively, the said substituent is one or more selected from the group consisting of deuterium, halogen, hydroxyl, carboxyl, $C_{1-3}$ alkyl or cyano;

Alternatively, two adjacent groups in $R^1$, $R^2$, $R^3$ and $R^4$ are joined together to form -O-$R^{17}$-O- group, wherein $R^{17}$ is C1-6 alkyl;

$R^5$, $R^6$, $R^7$ or $R^8$ are independently hydrogen, deuterium, halogen, hydroxyl, cyano, carboxyl, $C_{2-6}$ ester group or substituted or non-substituted groups of amino, amide, $C_{1-3}$ alkoxy or $C_{1-3}$ alkyl, the said substituent is one or more selected from the group consisting of deuterium, halogen, cyano, $C_{1-3}$ alkyl or $C_{1-3}$ haloalkyl;

$R^9$ is hydrogen, $C_{1-4}$ alkyl, or $C_{1-4}$ substituted alkyl, the said substituent is one or more selected from the group consisting of deuterium, hydroxyl, $C_{1-2}$ alkoxy, or cyano;

X is -C(=O)-, -S(=O)$_2$-, -S(=O)-, -C(=NH)- or substituted -C(=NH)-, the substituent is selected from CN and $SO_2NH_2$;

$R^{10}$ is $C_{1-4}$ alkyl, substituted $C_{1-4}$ alkyl, $C_{3-6}$ cycloalkyl, substituted $C_{3-6}$ cycloalkyl, amino, $C_{1-4}$ alkylamino, substituted $C_{1-4}$ alkylamino, $C_{3-6}$ cycloalkylamino, $C_{1-4}$ alkoxy, substituted $C_{1-4}$ alkoxy, or vinyl; or $R^9$ is attached to $R^{10}$ so that $R^9$-N-X-$R^{10}$ forms a ring group together; the said substituent is selected from one or more of deuterium, halogen and cyano;

$R^{11}$ is hydrogen, deuterium, halogen, cyano, $C_{1-2}$ alkyl, $C_{1-2}$ alkoxy, carboxyl, or $C_{1-3}$ alkyl substituted amide.

$R^{12}$ is a hydrogen, deuterium, halogen, hydroxyl, cyano, carboxyl, or substituted or non-substituted group of the following: $C_{1-4}$ alkyl, $C_{3-6}$ cycloalkyl, $C_{1-4}$ alkoxy, morpholinyl, aminocarbonyl, $C_{1-4}$ alkylamino carbonyl, or $C_{1-4}$ alkoxy carbonyl; the substituent is selected from one or more of deuterium, halogen or cyano; and

(a) when $R^{12}$ is hydrogen, $R^{13}$ is cyano, aldehyde group, carboxyl, borono, substituted $C_{1-4}$ alkoxy, or substituted or non-substituted group of $C_{1-4}$ alkoxy carbonyl, amino carbonyl, phenyl amino carbonyl, pyridinyl amino carbonyl, $C_{1-4}$ alkylamino carbonyl, $C_{1-4}$ alkoxy carbonyl, $C_{1-4}$ alkylamino carbonyl amino, $C_{2-6}$ heterocycloalkyl carbonyl, $C_{3-6}$ heterocycloalkylketone group, $C_{1-2}$ alkyl carbonyl, sulfonamido, sulfonyloxy, amino sulfonyl, amino sulfonyloxy, $C_{1-4}$ alkyl sulfonamido, $C_{1-4}$ alkyl amino sulfonyl, $C_{1-4}$ alkyl amino sulfonamido, $C_{1-4}$ alkyl sulfonyloxy, $C_{1-4}$ alkyl amino sulfonyloxy, $C_{1-4}$ alkyl amino carbonyloxy, tetrazolyl, triazolyl, imidazolyl, morpholinyl, $C_{1-4}$ alkyl thio, $C_{3-6}$ cycloalkoxy or $C_{3-6}$ heterocycloalkyloxy, the substituent is selected from one or more of deuterium, halogen, cyano, hydroxyl, phenyl, pyridyl, $C_{1-3}$ alkyl, $C_{1-3}$ deuteroalkyl or $C_{1-3}$ haloalkyl;

(b) when $R^{12}$ is not hydrogen, $R^{13}$ is cyano, aldehyde group, carboxyl, borono, or substituted or non-substituted group of $C_{1-4}$ alkoxy carbonyl, amino carbonyl, phenyl amino carbonyl, pyridinyl amino carbonyl, $C_{1-4}$ alkylamino carbonyl, $C_{1-4}$ alkoxy carbonyl amino, $C_{1-4}$ alkylamino carbonyl amino, $C_{2-6}$ heterocycloalkyl carbonyl, $C_{3-6}$ heterocycloalkylketone group, $C_{1-4}$ alkyl carbonyl, sulfonamido, sulfonyloxy, sulfamoyl, sulfamoyloxy, $C_{1-4}$ alkyl sulfonamido, $C_{1-4}$ alkyl amino sulfonyl, $C_{1-4}$ alkyl amino sulfonamido, $C_{1-4}$ alkyl sulfonyloxy, $C_{1-4}$ alkyl amino sulfonyloxy, $C_{1-4}$ alkyl amino carbonyloxy, heterocyclic group, $C_{1-4}$ alkyl amino, $C_{3-6}$ cycloalkyl amino, $C_{3-6}$ heterocycloalkyloxy, $C_{3-6}$ heterocycloalkyl amino, $C_{1-4}$ alkyl carbonyl amino, $C_{1-4}$

alkoxy, $C_{1-4}$ alkylthio, $C_{3-6}$ cycloalkoxy, $C_{3-6}$ heterocyclo alkyl or $C_{1-4}$ alkyl, the substituent is selected from one or more of deuterium, halogen, cyano, hydroxyl, phenyl, pyridyl, $C_{1-3}$ alkyl, $C_{1-3}$ deuteroalkyl and $C_{1-3}$ haloalkyl;

$R^{14}$ is a cyano, carboxyl, or substituted or non-substituted group of $C_{1-4}$ alkoxy carbonyl, amino carbonyl, $C_{1-4}$ alkyl amino carbonyl, $C_{1-4}$ alkoxy carbonyl amino, $C_{1-4}$ alkyl amino carbonyl amino, $C_{2-6}$ heterocycloalkyl carbonyl, sulfamoyl, $C_{1-4}$ alkylaminosulfonyl, heterocyclic, $C_{3-6}$ heterocycloalkyl, $C_{3-6}$ heterocycloalkyloxy, $C_{1-4}$ alkoxy, $C_{3-6}$ cy-

cloalkoxy, $C_{3-6}$ heterocycloalkoxy or $C_{1-4}$ alkyl, the substituent is selected from one or more of deuterium, halogen, cyano, hydroxyl, $C_{1-3}$ alkyl and $C_{1-3}$ haloalkyl;
$R^{15}$ is hydrogen, deuterium, halogen, cyano, $C_{1-2}$ alkyl, $C_{1-2}$ alkoxy, carboxyl, or $C_{1-3}$ alkyl substituted amide.

Provided that the following conditions are excluded:

(i) In formula (I), when $R^1$-$R^3$ is methoxy, $R^4$-$R^9$ is hydrogen, $R^{10}$ is methyl, $R^{11}$-$R^{12}$ is hydrogen, and $R^{15}$ is hydrogen, $R^{13}$ is selected from the following groups: $C_{1-4}$ alkoxy, $C_{1-4}$ alkylthio, deutero $C_{1-4}$ alkylthio, $C_{1-4}$-alkoxy carbonyl amino, butyl carbonyl, or morpholinyl;
(ii) In formula (I), $R^1$-$R^3$ is methoxy, $R^4$-$R^9$ is hydrogen, $R^{10}$ is deuteromethyl, $R^{11}$ is hydrogen, $R^{12}$ is deuterium, $R^{13}$ is methoxy, and $R^{15}$ is deuterium;
(iii) In formula (I), $R^1$-$R^3$ is methoxy, $R^4$-$R^9$ is hydrogen, $R^{10}$ is methyl, $R^{11}$ is hydrogen, $R^{12}$ is bromine, $R^{13}$ is methoxy, and $R^{15}$ is bromine.

In a preferred embodiment, the compounds of the invention are selected from the compoundes in formula (III) or formula (IV),

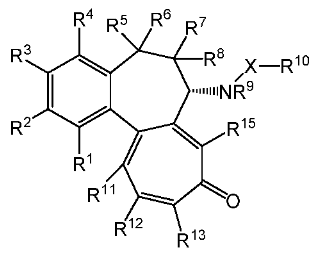
( III )

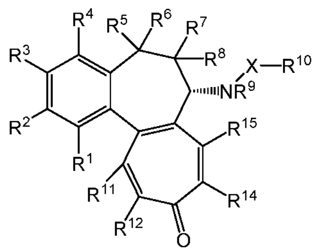
( IV )

In another preferred embodiment, the compound of the invention are selected from the compoundes in formula (V) or formula (VI),

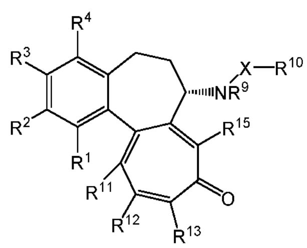
( V)

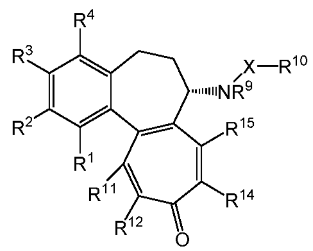
( VI )

In a preferred embodiment, $R^1$, $R^2$, $R^3$, or $R^4$ are independently hydrogen, deuterium, halogen, cyano, $C_{1-3}$ alkoxy, aldehyde group, $C_{1-3}$ alkyl, substituted $C_{1-3}$ alkoxy, or substituted $C_{1-3}$ alkyl, respectively, wherein the substituent is one or more selected from the group consisting of deuterium, halogen, $C_{1-3}$ alkyl, and cyano;

Alternatively, $R^1$ and $R^2$, $R^2$ and $R^3$, or $R^3$ and $R^4$ are joined together to form the -O-$R^{17}$-O- group, wherein $R^{17}$ is $C_{1-5}$ alkyl, preferably $C_{1-3}$ alkyl;
In a preferred embodiment, $R^1$ and $R^2$, $R^2$ and $R^3$ or $R^3$ and $R^4$ are joined together to form $-O-CH_2-O-$, $-O-CH_2CH_2-O-$ or $-O-CH_2CH_2CH_2-O-$, $-O-CH_2C(CH_3)_2CH_2-O-$, $-O-C(CH_3)_2C(CH_3)_2-O-$ group.

In a preferred embodiment, $R^5$, $R^6$, $R^7$, or $R^8$ is independently hydrogen, deuterium, halogen, cyano, carboxyl, hydroxyl, or amino, respectively;

In a preferred embodiment, $R^9$ is hydrogen, $C_{1-4}$ alkyl, or $C_{1-4}$ substituted alkyl, wherein the substituent is one or more selected from the group consisting of deuterium, hydroxyl, $C_{1-2}$ alkoxy, and cyano;
In a preferred embodiment, $R^{10}$ is a $C_{1-4}$ alkyl, substituted $C_{1-4}$ alkyl, $C_{3-6}$ cycloalkyl, substituted $C_{3-6}$ cycloalkyl, amino, $C_{1-4}$-alkyl amino, substituted $C_{1-4}$-alkyl amino, $C_{3-6}$ cycloalkyl amino, $C_{1-4}$ alkoxy, substituted $C_{1-4}$ alkoxy,

or vinyl, or $R^9$-N-X-$R^{10}$ forms

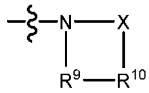,

the substituent is selected from one or more of deuterium and cyano;

In a preferred embodiment, $R^{11}$ is hydrogen, deuterium, halogen, cyano, $C_{1-2}$ alkyl, $C_{1-2}$ alkoxy, carboxyl, or $C_{1-3}$ alkyl substituted amide.

In a preferred embodiment, $R^{12}$ is hydrogen, deuterium, halogen, hydroxyl, cyano, or substituted or non-substituted group of the following: $C_{1-4}$ alkyl, $C_{3-6}$ cycloalkyl, $C_{1-4}$ alkoxy, morpholinyl, $C_{1-4}$ alkylamino carbonyl or $C_{1-4}$-alkoxy carbonyl; the substituent is selected from one or more of deuterium, halogen and cyano; and

In a preferred embodiment, when $R^{12}$ is hydrogen, $R^{13}$ is a cyano, aldehyde group, carboxyl, borono, substituted $C_{1-4}$ alkoxy, or substituted or non-substituted group of $C_{1-4}$ alkoxy carbonyl, amino carbonyl, phenyl amino carbonyl, pyridinyl amino carbonyl, $C_{1-4}$ alkyl amino carbonyl, $C_{1-4}$ alkoxy carbonyl amino, $C_{1-4}$ alkyl amino carbonyl amino, pyrrolidyl carbonyl, piperazinyl carbonyl, morpholinyl carbonyl, pyrrolidonyl, oxazolidinonyl, morpholinonyl, $C_{1-4}$ alkyl carbonyl, sulfonamido, sulfonyloxy, amino sulfonyl, amino sulfonyloxy, $C_{1-4}$ alkyl sulfonamido, $C_{1-4}$ alkyl amino sulfonyl, $C_{1-4}$ alkyl amino sulfonamido, $C_{1-4}$ alkyl amino sulfonyloxy, $C_{1-4}$ alkyl amino carbonyloxy, tetrazolyl, triazolyl, imidazolyl, morpholinyl, $C_{1-4}$ alkyl thio, $C_{3-6}$ cycloalkyloxy or tetrahydrofuranyloxy, the substituent is one or more selected from the group consisting of deuterium, halogen, cyano, hydroxyl, phenyl, $C_{1-3}$ alkyl, $C_{1-3}$ deuteroalkyl or $C_{1-3}$ haloalkyl.

In a preferred embodiment, when $R^{12}$ is not hydrogen, $R^{13}$ is cyano, aldehyde group, carboxyl, borono, or substituted or non-substituted group of $C_{1-4}$ alkoxy carbonyl, amino carbonyl, phenyl amino carbonyl, pyridinyl amino carbonyl, $C_{1-4}$ alkyl amino carbonyl, $C_{1-4}$ alkoxy carbonyl amino, $C_{1-4}$ alkyl amino carbonyl amino, $C_{2-6}$ heterocycloalkyl carbonyl, $C_{3-6}$ heterocycloalkyl ketone group, $C_{1-4}$ alkyl carbonyl, sulfonamido, sulfonyloxy, amino sulfonyl, amino sulfonyloxy, $C_{1-4}$ alkyl sulfonamido, $C_{1-4}$ alkyl amino sulfonyl, $C_{1-4}$ alkyl amino sulfonamido, $C_{1-4}$ alkyl sulfonyloxy, $C_{1-4}$ alkyl amino sulfonyloxy, $C_{1-4}$ alkyl amino carbonyloxy, heterocyclic, $C_{1-4}$ alkyl amino, $C_{3-6}$ cycloalkyl amino, tetrahydrofuranyloxy, tetrahydrofuranyl amino, $C_{1-4}$ alkyl carbonyl amino, $C_{1-4}$ alkoxy, $C_{1-4}$ alkylthio, $C_{3-6}$ cycloalkoxy, $C_{3-6}$ heterocycloalkyl or $C_{1-4}$ alkyl, wherein the substituent is one or more selected from the group consisting of deuterium, halogen, cyano, hydroxyl, phenyl, $C_{1-3}$ alkyl, $C_{1-3}$ deuteroalkyl or $C_{1-3}$ haloalkyl.

In a preferred embodiment, $R^{14}$ is a cyano, carboxyl, or substituted or non-substituted group of $C_{1-4}$ alkoxy carbonyl, aminocarbonyl, $C_{1-4}$ alkyl amino carbonyl, $C_{1-4}$ alkoxy carbonyl amino, $C_{1-4}$ alkyl amino carbonyl amino, *N*-pyrrolidinyl carbonyl, amino sulfonyl, $C_{1-4}$ alkyl amino sulfonyl, heterocyclic group, tetrahydrofuranyloxy, $C_{1-4}$ alkoxy, $C_{3-6}$ cycloalkyloxy, or $C_{1-4}$ alkyl, wherein the substituent is one or more selected from the group consisting of deuterium, halogen, cyano, hydroxyl, $C_{1-3}$ alkyl or $C_{1-3}$ haloalkyl.

In a preferred embodiment, $R^{15}$ is hydrogen, deuterium, halogen, cyano, $C_{1-2}$ alkyl, $C_{1-2}$ alkoxy, carboxyl, or $C_{1-3}$ alkyl substituted amide.

In a further preferred embodiment, $R^1$, $R^2$ or $R^3$ is independently $C_{1-3}$ alkoxy, respectively; or, $R^1$ and $R^2$ or $R^2$ and $R^3$ are joined together to form -O-$R^{17}$-O- group, wherein $R^{17}$ is $C_{1-2}$ alkyl;

In a further preferred embodiment, $R^4$ is hydrogen, deuterium, halogen, or cyano.

In a further preferred embodiment, $R^9$ is hydrogen or $C_{1-3}$ alkyl;

In a further preferred embodiment, $R^{10}$ is $C_{1-3}$ alkyl, substituted $C_{1-3}$ alkyl, $C_{3-6}$ cycloalkyl, amino, $C_{1-4}$ alkyl amino, $C_{1-3}$ alkoxy or vinyl, or $R^9$ forms

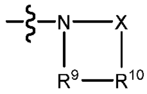

group with N-X-$R^{10}$; wherein the substituent is selected from one or more of deuterium and cyano;

In a further preferred embodiment, $R^{11}$ is hydrogen, deuterium or halogen;

In a further preferred embodiment, $R^{12}$ is hydrogen, deuterium, halogen, cyano, or substituted or non-substituted group of the following: $C_{1-3}$ alkyl, C3-5 cycloalkyl, $C_{1-3}$ alkoxy, morpholinyl, $C_{1-4}$ alkylamino carbonyl or $C_{1-4}$-alkoxy carbonyl; wherein the substituent is selected from one or more of deuterium and halogen; and

In a further preferred embodiment, when $R^{12}$ is hydrogen, $R^{13}$ is a cyano, aldehyde group, carboxyl, borono, substituted $C_{1-4}$ alkoxy, or substituted or non-substituted group of $C_{1-4}$ alkoxy carbonyl, amino carbonyl, phenyl amino

carbonyl, pyridinyl amino carbonyl, $C_{1-4}$ alkyl amino carbonyl, $C_{1-4}$ alkoxy carbonyl amino, $C_{1-4}$ alkyl amino carbonyl amino, N-pyrrolidyl carbonyl, piperazinyl carbonyl, morpholinyl carbonyl, pyrrolidonyl, oxazolidinonyl, 3-morpholinonyl, $C_{1-4}$ alkyl carbonyl, sulfonamido, sulfonyloxy, amino sulfonyl, amino sulfonyloxy, $C_{1-4}$ alkyl sulfonamido, $C_{1-4}$ alkyl amino sulfonyl, $C_{1-4}$ alkyl amino sulfonamido, $C_{1-4}$ alkyl amino sulfonyloxy, $C_{1-4}$ alkyl amino carbonyloxy, tetrazolyl, triazolyl, imidazolyl, pyrazolyl, morpholinyl, $C_{1-4}$ alkyl thio, $C_{3-6}$ cycloalkyloxy or tetrahydrofuranyloxy, the substituent is one or more selected from the group consisting of deuterium, halogen, cyano, hydroxyl, phenyl, $C_{1-3}$ alkyl, $C_{1-3}$ deuteroalkyl or $C_{1-3}$ haloalkyl.

In a further preferred embodiment, when $R^{12}$ is not hydrogen, $R^{13}$ is cyano, aldehyde group, carboxyl, borono, or substituted or non-substituted group of $C_{1-4}$ alkoxy carbonyl, amino carbonyl, phenyl amino carbonyl, pyridinyl amino carbonyl, $C_{1-4}$ alkyl amino carbonyl, $C_{1-4}$ alkoxy carbonyl amino, $C_{1-4}$ alkyl amino carbonyl amino, pyrrolidonyl, 2-oxazolidinonyl, 3-morpholinonyl, pyrrolidinyl carbonyl, methylpiperazinyl carbonyl, morpholinyl carbonyl, $C_{1-4}$ alkyl carbonyl, sulfonamido, sulfonyloxy, amino sulfonyl, amino sulfonyloxy, $C_{1-4}$ alkyl sulfonamido, $C_{1-4}$ alkyl amino sulfonyl, $C_{1-4}$ alkyl amino sulfonamido, $C_{1-4}$ alkyl sulfonyloxy, $C_{1-4}$ alkyl amino sulfonyloxy, $C_{1-4}$ alkyl amino carbonyloxy, heterocyclic, $C_{1-4}$ alkyl amino, $C_{3-6}$ cycloalkyl amino, tetrahydrofuranyloxy, tetrahydrofuranyl amino, $C_{1-4}$ alkyl carbonyl amino, $C_{1-4}$ alkoxy, $C_{1-4}$ alkylthio, $C_{3-6}$ cycloalkoxy, $C_{3-6}$ heterocycloalkyl or $C_{1-4}$ alkyl, wherein the substituent is one or more selected from the group consisting of deuterium, halogen, cyano, hydroxyl, phenyl, pyridyl, $C_{1-3}$ alkyl, $C_{1-3}$ deuteroalkyl or $C_{1-3}$ haloalkyl;

In another preferred embodiment, $R^{14}$ is cyano, carboxyl or substituted or non-substituted with the following groups: $C_{1-4}$ alkoxycarbonyl, aminocarbonyl, $C_{1-4}$ alkylaminocarbonyl, $C_{1-4}$ alkoxycarbonylamino, $C_{1-4}$ alkylaminocarbonylamino, N-pyrrolidinylcarbonyl, aminosulphonyl, $C_{1-4}$ alkylaminosulphonyl, heterocycloalkyl, tetrazolyl, triazolyl, tetrahydrofuroxy, $C_{1-4}$ alkoxy, $C_{3-6}$ cycloalkoxy, or $C_{1-4}$ alkyl, wherein the substituent is selected from one or more of deuterium, halogen, cyano, hydroxy, $C_{1-3}$ alkyl, or $C_{1-3}$ haloalkyl.

In a further preferred embodiment, $R^{14}$ is a cyano, carboxyl, or substituted or non-substituted group of $C_{1-4}$ alkoxy carbonyl, aminocarbonyl, $C_{1-4}$ alkyl amino carbonyl, $C_{1-4}$ alkoxy carbonyl amino, $C_{1-4}$ alkyl amino carbonyl amino, *N*-pyrrolidinyl carbonyl, amino sulfonyl, $C_{1-4}$ alkyl amino sulfonyl, pyrrolidyl, oxazolidyl, isooxazolidyl, piperazinyl, morpholinyl, piperidyl, tetrahydrofuranyl, tetrahydrothiophenyl, hexahydropyrimidyl, tetraazolidyl, triazolidyl, tetrahydrofuranyloxy, $C_{1-4}$ alkoxy, $C_{3-6}$ cycloalkyloxy, or $C_{1-4}$ alkyl, wherein the substituent is one or more selected from the group consisting of deuterium, halogen, cyano, hydroxyl, $C_{1-3}$ alkyl or $C_{1-3}$ haloalkyl.

In a further preferred embodiment, $R^{15}$ is hydrogen, deuterium, halogen, methyl, ethyl, halomethyl, or haloethyl.

In a further preferred embodiment, $R^9$ is hydrogen, methyl or ethyl;

In a further preferred embodiment, $R^{10}$ is methyl, ethyl, cyclopropyl, amino, methylamino, ethylamino, dimethylamino, diethylamino, methoxy, ethoxy, cyanomethyl, or cyanoethyl, or $R^9$ is joined together with N-X-$R^{10}$ to form

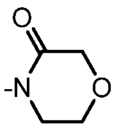
or
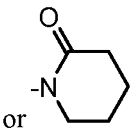
.

In a further preferred embodiment, $R^{12}$ is hydrogen, deuterium, methyl, ethyl, halogen, cyano, methoxy, ethoxy, morpholinyl, aminocarbonyl, phenyl aminocarbonyl, methyl aminocarbonyl, methyl formate or ethyl formate;

In a further preferred embodiment, when $R^{12}$ is hydrogen, $R^{13}$ is cyano, aldehyde group, carboxyl, borono, halomethoxyl, haloethoxyl, deutero methoxyl, deuteroethoxyl, methoxy carbonyl, ethoxy carbonyl, aminocarbonyl, phenyl aminocarbonyl, pyridinyl aminocarbonyl, methyl aminocarbonyl, deuteromethylaminocarbonyl, halomethyl aminocarbonyl, ethyl aminocarbonyl, dimethylaminocarbonyl, methoxy carbonyl amino, ethoxy carbonyl amino, (methoxy carbonyl)(methyl) amino, methyl amino carbonyl amino, ethyl amino carbonyl amino, dimethyl amino carbonyl, pyrrolidyl carbonyl, cyanopyrrolidyl carbonyl, piperazinyl carbonyl, methyl piperazinyl carbonyl, morpholinyl carbonyl, pyrrolidonyl, 2-oxazolidonyl, 3-morpholinonyl, methyl carbonyl, ethyl carbonyl, sulfonyl amino, sulfonyloxy, methyl sulfonyloxy, ethyl sulfonyloxy, hydroxyl sulfonyloxy, amino sulfonyl, amino sulfonyloxy, methyl sulfonyl amino, ethyl sulfonyl amino, methyl amino sulfonyl, methyl amino sulfonyl amino, methyl amino sulfonyloxy, N-ethyl methyl amino carbonyloxy, tetrazolyl, triazolyl, imidazolyl, pyrazolyl, morpholinyl, methylthio, ethylthio, halomethylthio, haloethylthio, deuteromethylthio, deuteroethylthio or tetrahydrofuranyloxy;

In a further preferred embodiment, when $R^{12}$ is not hydrogen, $R^{13}$ is cyano, aldehyde group, carboxyl, borono, methoxy carbonyl, ethoxy carbonyl, methylaminocarbonyl, dimethylaminocarbonyl, aminocarbonyl, phenylamino carbonyl, pyridyl amnio carbonyl, ethyl aminocarbonyl, halomethyl aminocarbonyl, deuteromethyl aminocarbonyl, (methoxycarbonyl)(methyl) amino, methyl aminocarbonyl amino, ethyl aminocarbonyl amino, dimethyl amino carbonyl amino, pyrrolidyl carbonyl, cyanopyrrolidyl carbonyl, piperazinyl carbonyl, methyl piperazinyl carbonyl, morpholinyl carbonyl, pyrrolidonyl, 2-oxazolidinonyl, 3-morpholinonyl, methyl carbonyl, ethyl carbonyl, sulfonyl amino, sulfonyloxy, hydroxyl sulfonyloxy, amino sulfonyl, amino sulfonyloxy, methyl sulfonyl amino, ethyl sulfonyl amino, methyl amino sulfonyl, ethyl amino sulfonyl, methyl amino sulfonyl amino, methyl amino sulfonyloxy, *N*-ethyl methyl amino carboxyloxy, tetrazolyl, triazolyl, methyl amino, ethyl amino, tetrahydrofuranyloxy, tetrahydrofuranyl amino, methyl carbonyl amino, methoxy, halomethoxy, deuteromethoxy, ethoxy, haloethoxy, deuteroethoxy, methylthio, halomethylthio, deuteromethylthio, ethylthio, haloethylthio, deuteroethylthio, tetrahydrofuranyl, piperazinyl, piperidyl, methylpiperidyl, imidazolyl, pyrazolyl, morpholinyl, pyrrolidinyl, tetrahydrocarbazolyl, morpholinyl, methyl, ethyl, propyl, hydroxymethyl, hydroxyethyl, halomethyl or haloethyl.

In a further preferred embodiment, $R^{11}$ is hydrogen or deuterium;

In a further preferred embodiment, $R^{14}$ is methylaminocarbonyl, dimethylaminocarbonyl, cyano, methoxy carbonyl, ethoxy carbonyl, carboxyl, *N*-pyrrolidyl carbonyl, pyrrolidinyl, oxazolidinyl, isoxazolidinyl, piperazinyl, morpholinyl, piperidinyl, tetrahydrofuranyl, tetrahydrothiophenyl, hexahydropyrimidinyl, tetrazolyl, triazolyl, tetrahydrofuranyloxy, methoxy, ethoxy, cyclopropoxy, halomethoxy, haloethoxy, deuteromethoxy, deuteroethoxy, methyl, ethyl, propyl, hydroxymethyl, hydroxyethyl, morpholinyl, halomethyl or haloethyl;
In a further preferred embodiment, $R^{15}$ is hydrogen, deuterium, methyl, ethyl, halomethyl, or haloethyl.

The compounds, isomers or pharmaceutically acceptable salts in the invention, wherein the compounds are specifically selected from:

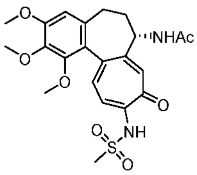
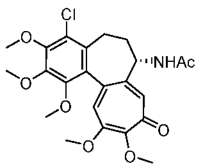
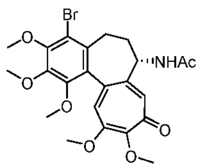
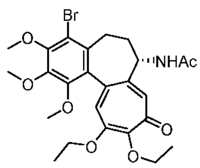

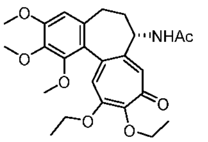
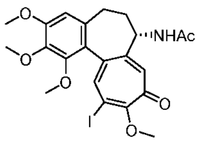
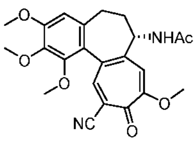
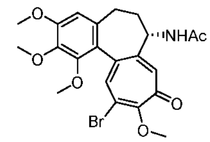

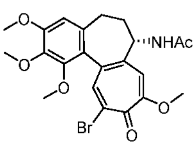
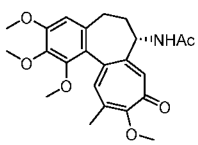
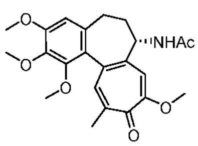
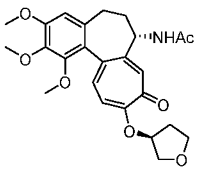

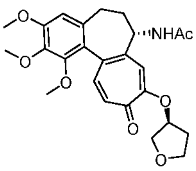
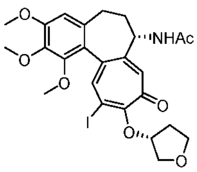
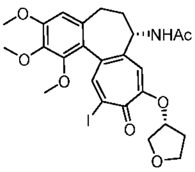
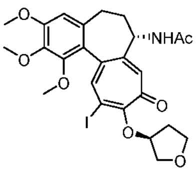

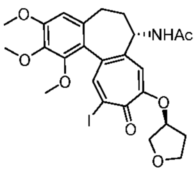
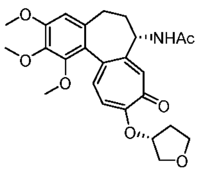
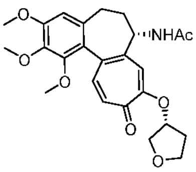
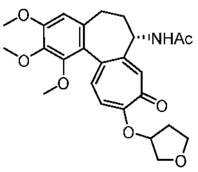

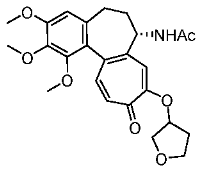 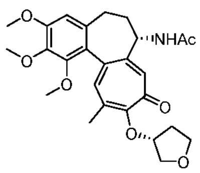 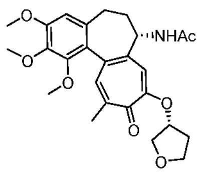 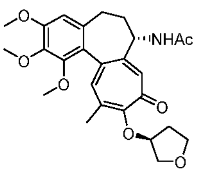

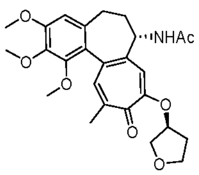 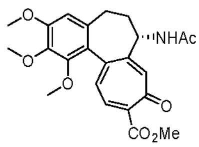 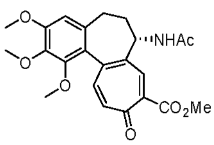 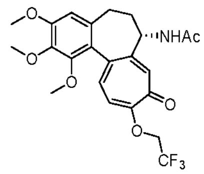

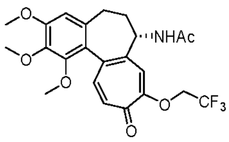 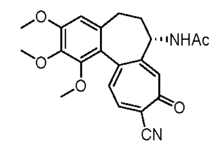 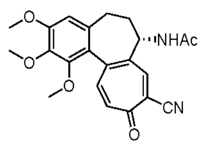 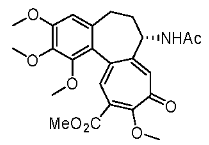

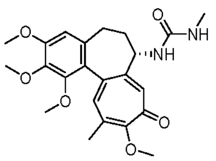 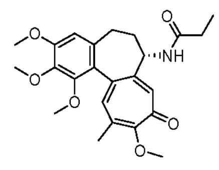 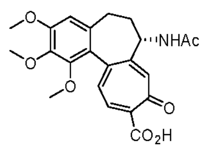 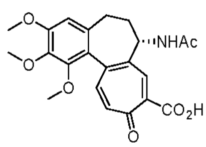

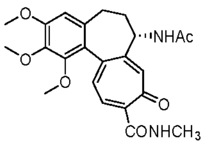 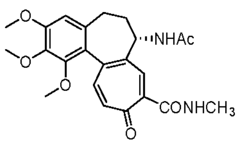 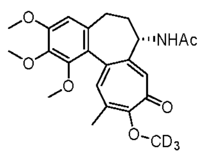 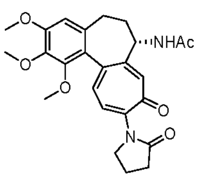

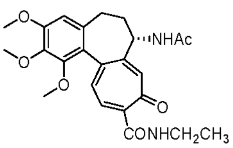 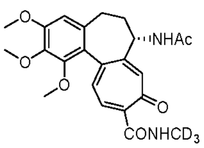 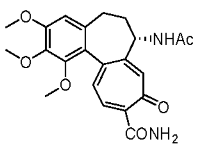 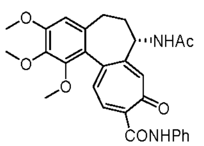

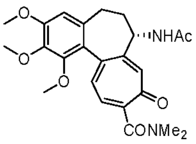 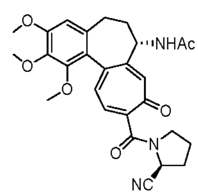 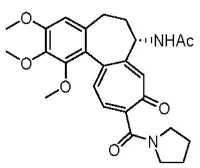 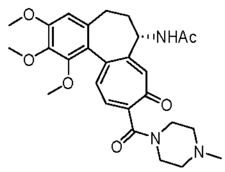

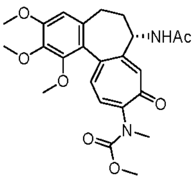 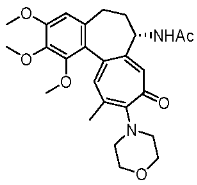 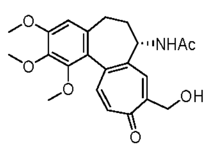 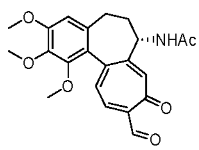

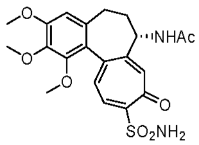
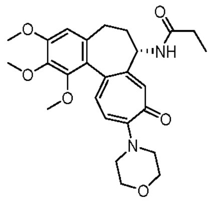
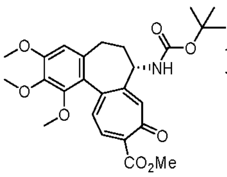
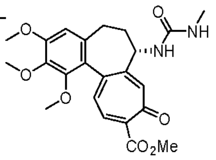

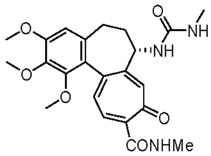
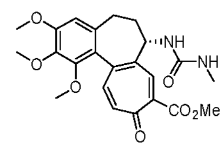
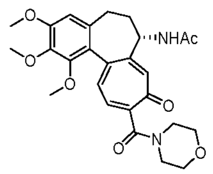
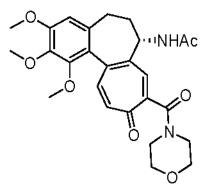

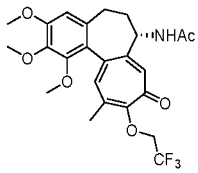
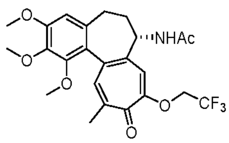
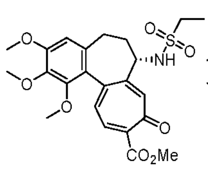
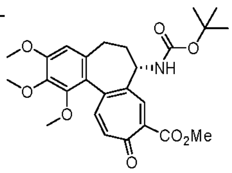

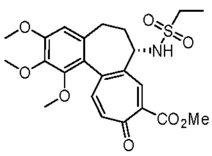
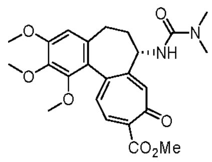
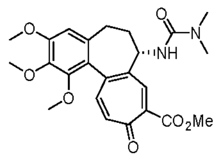
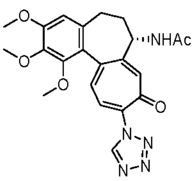

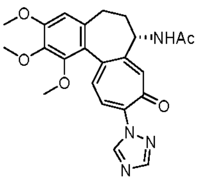
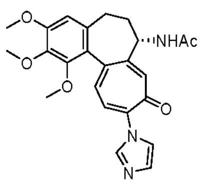
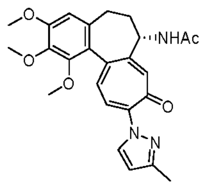
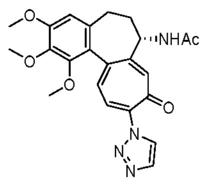

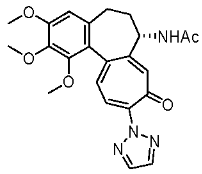
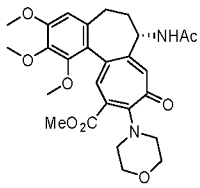
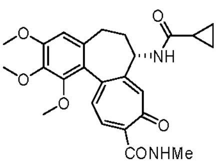
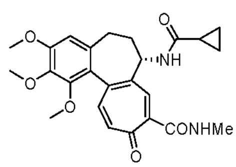

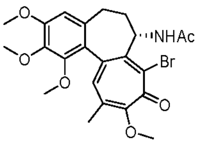 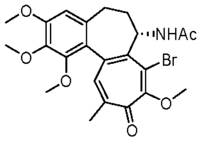 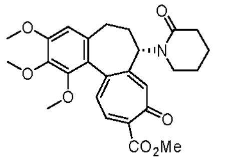 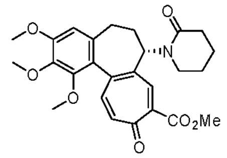

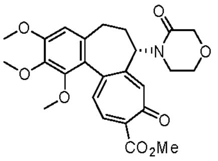 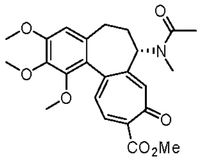 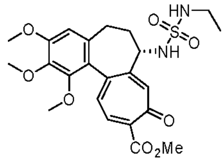 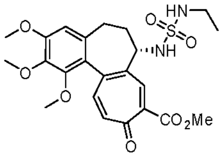

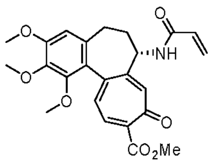 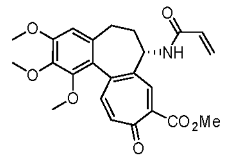 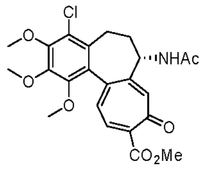 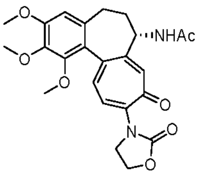

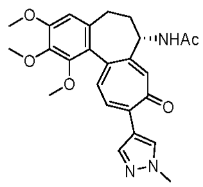 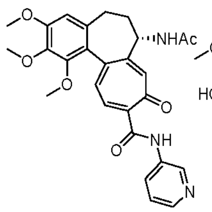 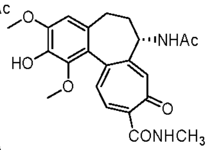 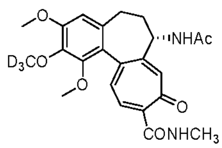

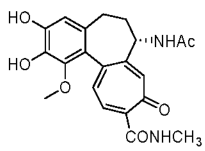 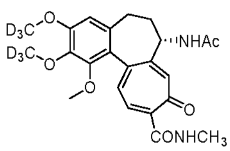 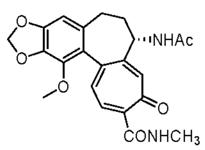 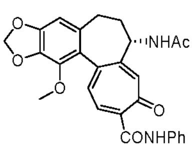

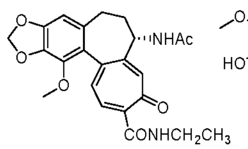 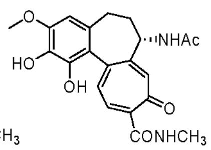 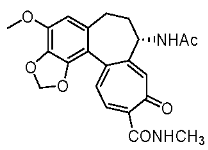 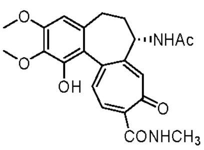

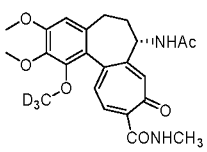 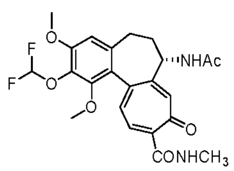 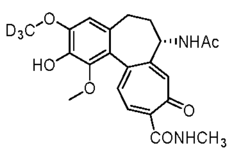

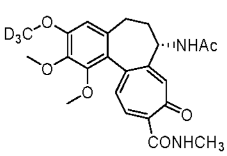 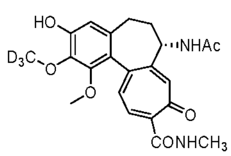 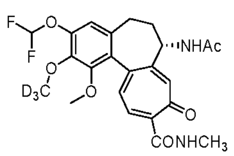

Unless otherwise stated, the following terms used in claims and specifications have the following meanings: "Hydrogen" means protium (1H), which is the main stable isotope of the element hydrogen.

"Deuterium" refers to a stable form isotope of hydrogen, also known as heavy hydrogen, whose element symbol

is D.

"Halogen" means an atom of fluorine, chlorine, bromine or iodine.

"Cyano", which means the -CN group.

"Alkyl" means a saturated alkyl group of 1-20 carbon atoms, including straight and branched groups (the range of numbers referred to in this application, e.g. "1-20", refers to the group, which in this case is an alkyl group, may contain 1 carbon atom, 2 carbon atoms, 3 carbon atoms, etc., up to 20 carbon atoms). Alkyl groups with 1-4 carbon atoms are called lower alkyl groups. When the lower alkyl group has no substituent, it is said to be an unsubstituted lower alkyl group. Preferably, an alkyl group is a medium-sized alkyl group with 2-5 carbon atoms. The alkyl groups in the invention are methyl, ethyl, propyl, 2-propyl, n-butyl, isobutyl, tert-butyl, amyl, etc. Preferably, an alkyl group is a lower alkyl group with 2-4 carbon atoms, such as ethyl, propyl, 2-propyl, n-butyl, isobutyl, or tert-butyl. Alkyl groups can be substituted or unsubstituted. In the interpretation of groups, R' is used to represent alkyl groups. Substituted alkyl groups are groups in which one or more H atoms in an alkyl group are replaced by other groups. Haloalkyl groups are groups in which one or more H atoms of alkyl groups are replaced by halogens.

"Alkoxy", denotes -OR 'group, i.e. -O- (unsubstituted alkyl) and -O- (unsubstituted cycloalkyl) groups, which further denotes -O- (unsubstituted alkyl). Representative examples include, but are not limited to, methoxy, ethoxy, propoxy, butoxy, cyclopropoxy, cyclobutoxy, cyclopentoxy, cyclohexyl, etc. Substituted alkoxy groups are groups in which one or more H atoms of an alkoxy group are replaced by other groups.

"Alkylthio", denotes -SR', i.e. -S- (unsubstituted alkyl) and -S- (unsubstituted cycloalkyl) groups, which further denotes -S- (unsubstituted alkyl). Representative examples include, but are not limited to, methylthio, ethylthio, propylthio, butylthio, cyclopropylthio, cyclobutylthio, cyclopentylthio, cyclohexylthio, etc. Substituted alkylthio groups are groups in which one or more H atoms of an alkylthio group are replaced by other groups.

"Alkyl amino", denotes -NHR' or $-N(R'^{1})(R'^{2})$ group, that is, the group in which one or both of the H atoms of the amino group are replaced by alkyl group. $C_{1-4}$ in $C_{1-4}$ alkyl amino group refers to the number of C atoms in the entire group. Representative examples include, but are not limited to, methylamino, ethylamino, propylamino, isopropylamino, butylamino, *N*,*N*-dimethylamino, *N,N*-diethylamino, *N*,*N*-dipropylamino, methyl ethyl amino, methyl propyl amino, ethyl propyl amino, etc.

"Cycloalkyl amino", denotes -NH-cycloalkyl or -N(cycloalkyl 1)(cycloalkyl 2) group, that is, one or two H atoms of the amino group are replaced by the cycloalkyl group. The $C_{3-6}$ in $C_{3-6}$ cycloalkyl amino group refers to the number of 3-6 C atoms in the entire group. Representative examples include, but are not limited to, cyclopropyl amino, cyclobutyl amino, cyclopentylamino, etc.

"Cycloalkyl" denoting monocyclic or bicyclic alkyl groups with more than 3 C atoms, includes, but are not limited to cyclopropyl, cyclopentyl, cyclohexyl, and dicycloheptyl.

"Carbonyl group", denotes C=O or -C(=O)- group.

"Hydroxyl", denotes -OH group.

"Aldehyde group", denotes -CH(=O) group.

"Carboxyl ", denotes -COOH group.

"Ester group", denotes -COOR' group, i.e. - COO-alkyl. $C_{2-6}$ in $C_{2-6}$ ester group means that the whole ester group contains 2-6 C atoms, such as $C_2$ ester group means methyl formate, $C_6$ ester group means pentyl formate; Common ester groups include but are not limited to methyl formate, ethyl formate, propyl formate, isopropyl formate, n-butyl formate, isobutyl formate, etc.

"Amide group", denotes -NH(C=O) group. A substituted amide group indicates that the H atom in the amide group is replaced by another group.

"Nitro", denotes $-NO_2$ group.

"Amino", denotes $-NH_2$ group.

"Vinyl", denotes $-CH=CH_2$ group.

"Alkyl amino carbonyl", denotes -C(=O)-NH-R' or $-CO-N(R')_2$ groups, namely -C(=O)-NH-alkyl or $-C(=O)-N(alkyl)_2$ group. When N has two alkyl groups, they can be the same or different. $C_{1-4}$ in $C_{1-4}$ alkyl amino carbonyl group means the number of C atoms of "$C_{1-4}$ alkyl amino group" as a carbonyl substituent in this group is 1 to 4. Representative examples include, but are not limited to, methylaminocarbonyl, ethylaminocarbonyl, propylaminocarbonyl, isopropylaminocarbonyl, butylaminocarbonyl, *N*,*N*-didimethylaminocarbonyl, *N*,*N*-diethylaminocarbonyl, *N*,*N*-dipropylaminocarbonyl, methylethyl aminocarbonyl, methylpropyl aminocarbonyl, ethylpropyl aminocarbonyl, etc.

"Alkoxy carbonyl", denotes -COOR' group, that is, -C(=O)-O-alkyl, which is also conventionally known as ester group. $C_{1-4}$ in the alkoxy carbonyl group means the number of C atoms of the "alkoxy group" as a carbonyl substituent in the group is 1 to 4. Representative examples include, but are not limited to, methoxycarbonyl (also known as methyl formate), ethoxycarbonyl (also known as ethyl formate), propoxycarbonyl (also known as propyl formate), etc.

"Aminocarbonyl", indicates $-C(=O)-NH_2$ group. "Phenylaminocarbonyl", indicates -C(=O)-NHPh or $-C(=O)-N(Ph)_2$ group, wherein Ph is phenyl.

"Alkyl carbonyl", indicates -C(=O)-R' group. $C_{1-4}$ in the alkyl carbonyl group means the number of C atoms of

the "alkyl" in the group as the substituent of the carbonyl group is 1 to 4. Representative examples include, but are not limited to, methyl carbonyl, ethyl carbonyl, propyl carbonyl, etc.

"Alkoxy carbonyl amino", denotes -NH-C(=O)-OR' group, i.e. -NH-C(=O)-O-alkyl group. $C_{1-4}$ in $C_{1-4}$ alkoxy carbonyl amino group means the number of C atoms of the "alkoxy group" in the group as the substituent of the carbonyl amino group is 1 to 4. Representative examples include, but are not limited to, methoxy carbonyl amino, ethoxy carbonyl amino, propoxy carbonyl amino, isopropoxy carbonyl amino, etc.

"Alkaminocarbonyl amino", indicates -NH-C(=O)-NHR' or NH-C(=O)-N(R')$_2$ group. When N has two alkyl groups, they can be the same or different. $C_{1-4}$ in the alkyl amino carbonyl amino group means the number of C atoms of the alkyl amino group in the group is 1 to 4. Representative examples include, but are not limited to, methylaminocarbonyl amino, ethylaminocarbonyl amino, propylaminocarbonyl amino, isopropylaminocarbonyl amino, butylaminocarbonyl amino, *N*,*N*-dimethylaminocarbonyl amino, *N*,*N*-diethylaminocarbonyl amino, *N,N*-dipropylaminocarbonyl amino, methyl ethyl aminocarbonyl amino, methyl propyl aminocarbonyl amino, and ethyl propyl aminocarbonyl amino, etc.

"Morpholinyl", denotes one of

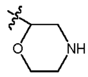 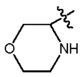 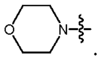.

"Pyrrolidyl", denotes one of

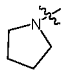 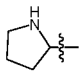 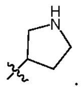.

"Oxazolidyl", denotes one of

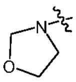 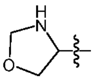 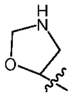 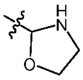.

"Isoxazolidyl", denotes one of

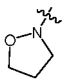 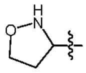 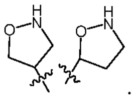.

"Piperidyl", denotes one of

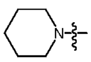 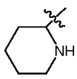.

"Tetrahydrofuranyl", denotes one of

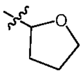 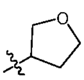.

"Tetrahydrothiophenyl", denotes one of

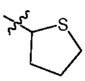 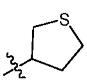.

"Hexahydropyrimidyl", denotes one of

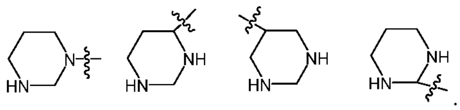

"Tetrazolyl", denotes one of

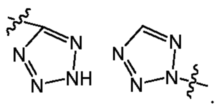

"Triazolyl", denotes one of

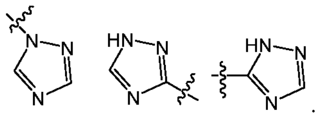

"Heterocycloalkyl" means a cyclic saturated alkyl having one or more O, N, S, P heteroatoms as ring atoms, representative examples include but are not limited to pyrrolidyl, oxazolidyl, isoxazolidyl, piperazinyl, morpholinyl, piperidyl, tetrahydrofuranyl, tetrahydrothiophenyl, hexahydropyrimidyl, etc.

"Heterocyclo alkyl carbonyl", means "-C(=O)-heterocyclo alkyl" group. $C_{2-6}$ in $C_{2-6}$ heterocyclo alkyl carbonyl group means that the number of C atoms in the heterocyclo alkane as a carbonyl substituent is 2 to 6. Representative examples include but are not limited to pyrrolidyl carbonyl, oxazolidyl carbonyl, isoxazolidyl carbonyl, piperazinyl carbonyl, morpholinyl carbonyl, piperidyl carbonyl, tetrahydrofuranyl carbonyl, tetrahydrothiophenyl carbonyl, hexahydropyrimidyl carbonyl, etc.

"Heterocycloalkyl ketone group" means a group having a ketone group (C=O) on the ring atom of a heterocyclo alkyl group, representative examples include, but are not limited to, pyrrolidonyl, oxazolidinonyl, isooxazolidinonyl, morpholinonyl, piperazinonyl, piperidonyl, tetrahydrothiophenonyl, etc.

"Sulfamido", indicates $-NH-S(O)_2-OH$ group.

"Sulfonyloxy", indicates $-O-S(O)_2-OH$ group.

"Sulfamoyl", indicates $-S(O)_2-NH_2$ group.

"Sulfamoyloxy", indicates $-O-S(O)_2-NH_2$ group.

"Alkyl sulfonamido", indicates $-NH-S(O)_2-R'$ group. $C_{1-4}$ in "$C_{1-4}$ alkyl sulfonamido" means the number of C atoms of the alkyl group that acts as a substituent for a sulfonamido group. Representative examples include, but are not limited to, methyl sulfonamido, ethyl sulfonamido, propyl sulfonamido, etc.

"Alkyl sulfamoyl", indicates $-S(O)_2-NHR'$ or $-S(O)_2-N(R')_2$ group, when N has two alkyl groups, the two alkyl groups may or may not be the same. $C_{1-4}$ in "$C_{1-4}$ alkyl sulfamoyl" means the number of C atoms of the alkyl group that acts as a substituent for the sulfamoyl group is 1 to 4. Representative examples include, but are not limited to, methylsulfamoyl, ethylsulfamoyl, propylsulfamoyl, isopropylsulfamoyl, butylsulfamoyl, *N,N*-dimethylsulfamoyl, *N,N*-diethylsulfamoyl, methyl ethyl sulfamoyl, etc.

"Alkyl amino sulfonyl amino", indicates $-NH-S(O)_2-NHR'$ or $-NH-S(O)_2-N(R')_2$ group, when N has two alkyl groups, the two alkyl groups may or may not be the same. The $C_{1-4}$ in "$C_{1-4}$ alkyl amino sulfonyl amino" means the number of C atoms of the alkyl group as the substituent of the amino sulfonyl amino group is 1 to 4. Representative examples include, but are not limited to, methyl amino sulfonyl amino, ethyl amino sulfonyl amino, propyl amino sulfonyl amino, isopropyl amino sulfonyl amino, butyl amino sulfonyl amino, *N,N*-dimethyl amino sulfonyl amino, *N,N*-diethyl amino sulfonyl amino, methyl ethyl amino sulfonyl amino, etc.

"Alkyl sulfonyloxy", indicates $-O-S(O)_2-R'$ group. $C_{1-4}$ in "$C_{1-4}$ alkyl sulfonyloxy" means the number of C atoms of the alkyl group that acts as a substituent for the sulfonyloxy group is 1 to 4. Representative examples include, but are not limited to, methyl sulfonyloxy, ethyl sulfonyloxy, and propyl sulfonyloxy.

"Alkyl sulfamoyloxy", indicates $-O-S(O)_2-NHR'$ or $-O-S(O)_2-N(R')_2$ group. Where N has two alkyl groups, they can be the same or different. $C_{1-4}$ in "$C_{1-4}$ alkyl sulfamyloxy" means the number of C atoms of the alkyl group that acts as a substituent for the amino sulfamyloxy group is 1 to 4. Representative examples include, but are not limited to, methyl amino sulfamoyloxy, ethyl amino sulfamoyloxy, propyl amino sulfamoyloxy, isopropyl amino sulfamoyloxy, butyl amino sulfamoyloxy, *N,N*-dimethylsulfamoyloxy, N,*N*-diethylsulfamoyloxy, methylethyl sulfamoyloxy, etc.

"Alkaminocarbonyloxy", indicates $-O-C(=O)-NHR'$ or $-O-C(=O)-N(R')_2$ group. Where N has two alkyl groups, they can be the same or different. $C_{1-4}$ in "$C_{1-4}$-alkyl aminocarbonyloxy" means the number of C atoms of the alkyl group

that acts as the substituent of the aminocarbonyloxy group is 1 to 4. Representative examples include, but are not limited to, methylaminocarbonyloxy, ethylaminocarbonyloxy, propylaminocarbonyloxy, isopropylaminocarbonyloxy, butylaminocarbonyloxy, *N,N*-dimethylaminocarbonyloxy, *N,N*-diethylaminocarbonyloxy, methylethyl aminocarbonyloxy, etc.

"Cycloalkoxy", means "-O-cycloalkyl" group. $C_{3-6}$ in "$C_{3-6}$ cycloalkoxy" means that the number of C atoms contained in this group is 3 to 6. Representative examples include, but are not limited to, cyclopropyloxy, cyclobutyloxy, cyclopentyloxy, etc.

"Heterocyclo alkyloxy", means "-O-heterocyclo alkyl" group. The $C_{3-6}$ in the $C_{3-6}$ heterocyclo alkyloxy group means that the number of C atoms contained in the group is 3 to 6. Representative examples include, but are not limited to, pyrrolidyloxy, oxazolidyloxy, isoxazolidyloxy, piperazinyloxy, morpholinyloxy, piperidyloxy, tetrahydrofuranyloxy, tetrahydrothiophenyloxy, hexahydropyrimidyloxy, etc.

"Heterocyclo alkyl amino ", means -NH-heterocyclo alkyl or -N(heterocyclo alkyl)$_2$ group. $C_{3-6}$ heterocyclo alkylamino group in $C_{3-6}$ means that the number of C atoms contained in the group is 3 to 6. Representative examples include, but are not limited to, pyrrolidyl amino, oxazolidyl amino, isoxazolidyl amino, piperazinyl amino, morpholinyl amino, piperidyl amino, tetrahydrofuranyl amino, tetrahydrothiophenyl amino, hexahydropyrimidyl amino, etc.

"Alkyl carbonyl amino ", indicates -NH-C(=O)-R' group. $C_{1-4}$ in the alkyl carbonyl amino group means that the number of C atoms contained in the alkyl group as a carbonyl amino group is 3 to 6. Representative examples include, but are not limited to, methyl carbonyl amino, ethyl carbonyl amino, propyl carbonyl amino, isopropyl carbonyl amino, butyl carbonyl amino, isobutyl carbonyl amino, etc.

"Heterocyclic group", indicates that in addition to the C atom, there are N, O, S, P and other heteroatoms as ring groups of ring atoms, including heterocyclo alkyl and heterocyclo aryl group. According to the number of ring atoms, it can include three-membered, four-membered, five-membered, six-membered heterocyclic, benzoheterocyclic group and so on. Common three-membered heterocyclic groups include oxiranyl, thiiranyl and so on. The four-membered heterocyclic groups include tetrazolyl, β-propiolactonyl, β-azetidinonyl, etc. The five-membered heterocyclic groups include furanyl, thiophenyl, pyrrolyl, thiazolyl, imidazolyl, tetrazolyl, triazolyl, etc. The six-membered heterocyclic groups are pyridinyl, pyrazinyl, pyrimidinyl, pyridazinyl and so on. The polyheterocyclic groups include indolyl, quinolinyl, pteridinyl, acridinyl and so on.

"Borono group", indicates -B(OH)$_2$ group.

"Pharmaceutically acceptable salts" are salts containing compounds of general formula (I) formed with organic or inorganic acids, indicating those salts that retain the biological availability and properties of the parent compound. These salts include:

(1) acid addition salts, obtained by the reaction of the free base of the parent compound with inorganic or organic acids, inorganic acids include, such as (but not limited to) hydrochloric acid, hydrobromic acid, nitric acid, phosphoric acid, metaphosphoric acid, sulfuric acid, sulfite and perchloric acid, etc. Organic acids include, such as (but not limited to) acetic acid, propionic acid, acrylic acid, oxalic acid, (D) or (L) malic acid, fumaric acid, maleic acid, hydroxybenzoic acid, γ-hydroxybutyric acid, methoxybenzoic acid, phthalic acid, methanesulfonic acid, ethanesulfonic acid, naphthalene-1-sulfonic acid, naphthalene-2-sulfonic acid, p-toluenesulfonic acid, salicylic acid, tartaric acid, citric acid, lactic acid, mandelic acid, succinic acid or malonic acid, etc.

(2) salts obtained by replacing the acid protons present in the parent compound with metal ions or by coordination with organic bases, metal ions are, such as alkali metal ions, alkaline earth metal ions or aluminum ions, and organic bases are, such as ethanolamine, diethanolamine, triethanolamine, tromethamine, n-methylglucosamine, etc.

"Pharmaceutical composition" means a mixture of one or more of the compounds described herein or their pharmaceutically acceptable salts and prodrugs with other chemical components, such as pharmaceutically acceptable carriers and excipients. The pharmaceutical composition intends to facilitate the delivery of a compound to an organism.

In the following, unless specifically limited, compounds of formula (I) as active ingredients of therapeutic agents include all their pharmaceutically acceptable salts, which shall be understood to fall within the scope of the present invention. In this specification, for convenience only, they are referred to simply as "compounds of formula (I)".

The invention comprises a pharmaceutical composition comprising any of the compounds, isomers, pharmaceutically acceptable salts of the invention or prodrugs or esters of the invention which are readily hydrolyzed as active ingredients, supplemented by pharmaceutically acceptable excipients.

The compounds, isomers or pharmaceutically acceptable salts thereof may be used in the preparation of anti-inflammatory drugs, in particular drugs for the treatment of acute gouty arthritis, gout or coronavirus pneumonia.

The experimental results showed that the compounds had a significant effect on gouty arthritis in rats, the compounds could significantly improve the gait of rats after modeling, and significantly reduce the swelling rate of ankles. The compounds can also reduce the levels of TNF-α, IL-1β and IL-6 in the synovial tissue of knee joint after modeling, and had excellent anti-inflammatory factors effect. The compounds of the invention can be applied in the treatment of various inflammation-related diseases. Therefore, this application compounds had potential application prospects in

anti-inflammatory drugs, including drugs for the treatment of arthritis, especially acute gouty arthritis drugs, rheumatoid arthritis drugs, drugs to reduce inflammatory factors, drugs for the treatment of inflammatory storm or coronavirus pneumonia.

## Specific mode for carrying out the embodiments

The present invention is further illustrated in combination with Examples below, but the scope of protection of the present invention is not limited to the following Examples.

### Example 1: Synthesis of

**(S)-N-(1,2,3-trimethoxy-10-methanesulfonylamino-9-oxo-5,6,7,9-tetrahydrobenzo[a]heptalen-7-yl)acetamid e (2)**

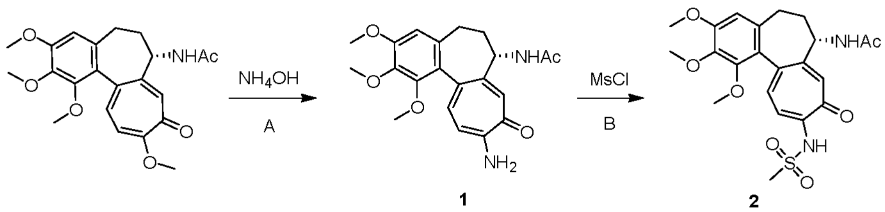


1 2

Step A: A mixture containing colchicine (800 mg, 2.0 mmol), concentrated ammonia (15 mL) and methanol (5 mL) was stirred at 30°C for 40 hours. Saturated saline (30 mL) was added, and the resulting mixture was extracted with ethyl acetate (40 mL×4) and dried over anhydrous sodium sulfate. The solvent was evaporated under reduced pressure to give (S)-N-(10-amino-1,2,3-trimethoxy-9-oxo-5,6,7,9-tetrahydrobenzo[a]heptalen-7-yl)acetamide (1) (699 mg). The yield was 90.9%.

Step B: To a solution of compound 1 (140 mg, 0.364 mmol) in pyridine (7 mL) was added methanesulfonyl chloride (9 drops), and after addition, the resulting mixture was stirred at 55°C for 3 hours. The solvent was evaporated under reduced pressure, water (20 mL) was added, the product was extracted with ethyl acetate (30 mL × 3), and the combined organic phases were washed with saturated brine (20 mL) and dried over anhydrous sodium sulfate. The solvent was evaporated under reduced pressure and the product was purified by column chromatography (200 ~ 300 mesh silica gel, eluted with petroleum ether:ethyl acetate = 1:1 ~ 1:6) to give (S)-N-(1,2,3-trimethoxy-10-methanesulfonylamino-9-oxo-5,6,7,9-tetrahydrobenzo[a]heptalen-7-yl)acetamide (2) (101 mg). The yield was 60.0%. $^1H$ NMR (DMSO-$d_6$, 400 MHz) δ 9.72 (s, 1H), 8.81 (d, J = 10.8 Hz, 1H), 8.65 (d, J = 6.8 Hz, 1H), 7.34 (s, 1H), 7.27 (d, J = 10.8 Hz, 1H), 6.80 (s, 1H), 4.41-4.35 (m, 1H), 3.86 (s, 3H), 3.81 (s, 3H), 3.55 (s, 3H), 2.65-2.61 (m, 1H), 2.28-2.18 (m, 4H), 2.10-2.04 (m, 1H), 1.93-1.81 (m, 4H). MS (ESI, m/z): 461.1 $[M-H]^-$.

### Example 2: Synthesis of

**(S)-N-(4-chloro-1,2,3,10,11-pentamethoxy-9-oxo-5,6,7,9-tetrahydrobenzo[a]heptalen-7-yl)acetamide (4)**

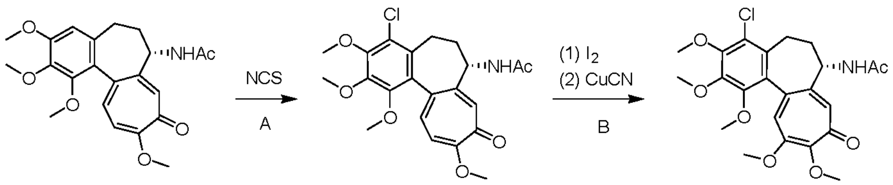


3 4

Step A: A mixture containing colchicine (3.0 g, 7.51 mmol), NCS (1.30 g, 9.73 mmol) and acetic acid (30 mL) was stirred under nitrogen at 70°C for 3.5 hours. Most of the solvent was evaporated under reduced pressure, water (60 mL) was added, the product was extracted with ethyl acetate (60 mL ×3), the combined organic phases were washed with saturated brine (40 mL) and dried over anhydrous sodium sulfate. The solvent was evaporated under reduced

pressure and the product was purified by column chromatography (200 ~ 300 mesh silica gel, eluted with ethyl acetate:methanol = 40:1 ~ 10:1) to afford (S)-N-(4-chloro-1,2,3,10-tetramethoxy-9-oxo-5,6,7,9-tetrahydrobenzo[a]heptalen-7-yl)acetamide (3) (2.50 g). The yield was 76.7%.

Step B: A mixture containing compound 3 (2.70 g, 6.22 mmol), iodine (2.39 g, 9.42 mmol), silver nitrate (1.60 g, 9.42 mmol) and methanol (30 mL) was stirred at 30°C overnight. Water (90 mL) was added and then saturated sodium thiosulfate solution was added dropwise until the reddish brown color disappeared. Extracted with ethyl acetate (60 mL ×3), the combined organic phases were washed with saturated brine (40 mL) and dried over anhydrous sodium sulfate. The solvent was evaporated under reduced pressure and the product was purified by column chromatography (200 ~ 300 mesh silica gel, eluted with ethyl acetate:methanol = 40:1 ~ 10:1) to give a yellow solid (1.48 g). The solid (600 mg) was taken and dissolved in DMF (6 mL), then cuprous cyanide (144 mg, 1.61 mmol) was added, and the resulting mixture was stirred at 100°C for 3 hours. Water (25 mL) was added and the product was extracted with ethyl acetate (30 mL ×3), the combined organic phases were washed sequentially with water (15 mL ×2) and saturated saline (15 mL) and dried over anhydrous sodium sulfate. The solvent was evaporated under reduced pressure and the product was purified by column chromatography (200 ~ 300 mesh silica gel, eluted with ethyl acetate: methanol = 50:1 ~ 20:1) to afford (S)-N-(4-chloro-1,2,3,10,11-pentamethoxy-9-oxo-5,6,7,9-tetrahydrobenzo[a]heptalen-7-yl)acetamide **(4)** (240 mg). The yield was 20.5%. $^1$H NMR (DMSO-$d_6$, 400 MHz) δ 8.63 (d, J = 7.6 Hz, 1H), 7.18 (s, 1H), 7.08 (s, 1H), 4.19-4.12 (m, 1H), 3.91 (s, 3H), 3.87 (s, 3H), 3.86 (s, 3H), 3.80 (s, 3H), 3.58 (s, 3H), 3.13-3.08 (m, 1H), 2.18-2.10 (m, 1H), 1.98-1.89 (m, 1H), 1.84-1.79 (m, 4H). MS (ESI, m/z): 464.1 $[M+H]^+$.

**Example 3: Synthesis of (S)-N-(4-bromo-1,2,3,10,11-pentamethoxy-9-oxo-5,6,7,9-tetrahydrobenzo[a]heptalen-7-yl)acetamide (6)**

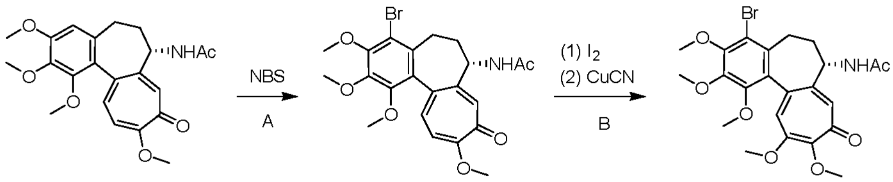


5

6

The experimental operations for synthesizing compound **6** was described in Example 2, wherein NCS in step A of Example 2 was replaced with NBS. $^1$H NMR (DMSO-$d_6$, 400 MHz) δ 8.63 (d, J = 7.2 Hz, 1H), 7.19 (s, 1H), 7.10 (s, 1H), 4.20-4.13 (m, 1H), 3.93 (s, 3H), 3.89 (s, 3H), 3.88 (s, 3H), 3.83 (s, 3H), 3.61 (s, 3H), 3.16-3.12 (m, 1H), 2.30-2.22 (m, 1H), 1.98-1.76 (m, 5H). MS (ESI, m/z): 508.0 $[M+H]^+$.

**Example 4: Synthesis of (S)-N-(4-bromo-10,11-diethoxy-1,2,3,-trimethoxy-9-oxo-5,6,7,9-tetrahydrobenzo[a]heptalen-7-yl)acetamide (7) and (S)-N-(10,11-diethoxy-1,2,3,-trimethoxy-9-oxo-5,6,7,9-tetrahydrobenzo[a]heptalen-7-yl)acetamide (8).**

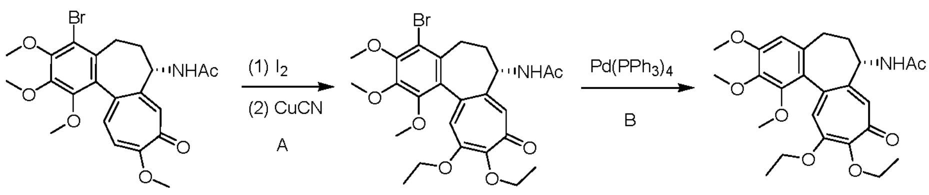


5

7

8

Step A: The experimental operations of synthesizing compound 7 using compound 5 as raw material were described in Step B of Example 2, wherein methanol and silver nitrate in Step B of Example 2 were replaced with ethanol and silver sulfate, respectively. $^1$H NMR (DMSO-$d_6$, 400 MHz) δ 8.69 (d, J = 7.2 Hz, 1H), 7.18 (s, 1H), 7.14 (s, 1H), 4.26-4.08 (m, 5H), 3.98 (s, 3H), 3.92 (s, 3H), 3.63 (s, 3H), 3.20-3.16 (m, 1H), 2.34-2.26 (m, 1H), 2.00-1.91 (m, 1H), 1.88 (s, 3H), 1.86-1.81 (m, 1H), 1.39-1.32 (m, 6H). MS (ESI, m/z): 536.0 $[M+H]^+$.

Step B: Diisopropylethylamine (120 mg, 0.929 mmol) and tetrakis(triphenylphosphine)palladium (93 mg, 0.0805 mmol) were added to a mixture containing Compound 7 (200 mg, 0.373 mmol), ethanol (4 mL), water (2 mL) and toluene

(12 mL), and after addition, the resulting mixture was stirred at reflux under nitrogen overnight. Water (20 mL) was added and the product was extracted with ethyl acetate (20 mL × 3). The combined organic phases were washed sequentially with water (15 mL × 2) and saturated saline (10 mL) and dried over anhydrous sodium sulfate. The solvent was evaporated under reduced pressure and the product was purified by column chromatography (200 ~ 300 mesh silica gel, eluted with ethyl acetate:methanol = 100:1 ~ 30:1) to give (S)-N-(10,11-diethoxy-1,2,3,-trimethoxy-9-oxo-5,6,7,9-tetrahydrobenzo[a]heptalen-7-yl)acetamide **(8).** $^1$H NMR (DMSO-$d_6$, 400 MHz) δ 8.57 (d, J = 7.6 Hz, 1H), 7.10 (s, 1H), 7.09 (s, 1H), 6.78 (s, 1H), 4.29-4.07 (m, 5H), 3.84 (s, 3H), 3.79 (s, 3H), 3.56 (s, 3H), 2.63-2.58 (m, 1H), 2.30-2.21 (m, 1H), 2.03-1.97 (m, 1H), 1.83-1.76 (m, 4H), 1.33-1.26 (m, 6H). MS (ESI, m/z): 458.2 $[M+H]^+$.

**Example 5: Synthesis of (S)-N-(11-iodo-1,2,3,10-tetramethoxy-9-oxo-5,6,7,9-tetrahydrobenzo[a]heptalen-7-yl)acetamide (12) and (S)-N-(11-cyano-1,2,3,9-tetramethoxy-10-oxo-5,6,7,10-tetrahydrobenzo[a]heptalen-7-yl)acetamide (13)**

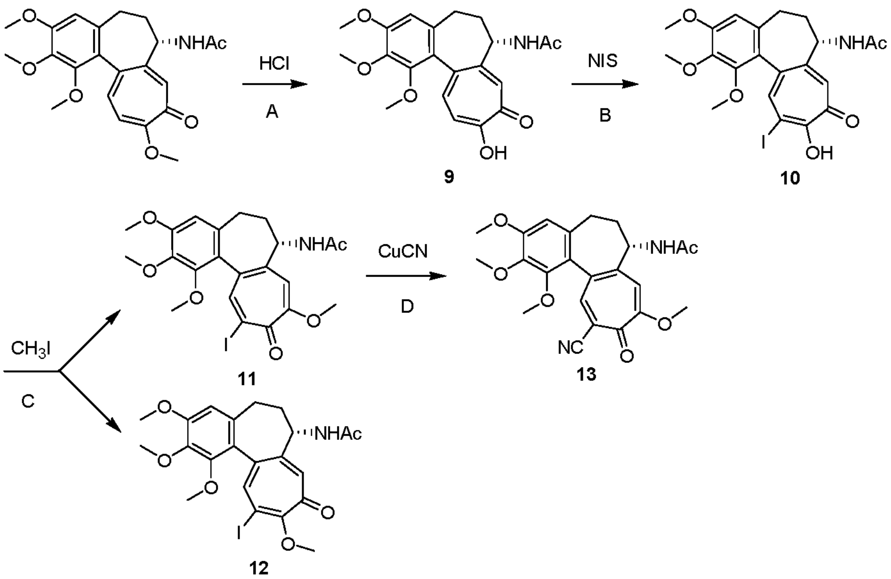


Step A: A mixture containing colchicine (6.0 g, 15.0 mmol), water (165 mL), concentrated hydrochloric acid (1.5 mL) and acetic acid (27.3 mL) was stirred at 100°C for 5 h. The mixture was extracted with ethyl acetate (200 mL ×3). The pH was adjusted to 5 ~ 6 with saturated sodium bicarbonate solution. The resulting mixture was extracted with ethyl acetate (200 mL ×3), the combined organic phases were washed with saturated brine (100 mL) and dried over anhydrous sodium sulfate. The solvent was evaporated under reduced pressure to afford (S)-N-(10-hydroxy-1,2,3-trimethoxy-9-oxo-5,6,7,9-tetrahydrobenzo[a]heptalen-7-yl)acetamide (9) crude (6.40 g). The compound was used directly in the next step of the reaction without purification.

Step B: NIS (3.59 g, 16.0 mmol) was added to a solution of crude compound 9 (5.60 g) in acetonitrile (70 mL) and after addition, the resulting mixture was stirred at room temperature overnight. Water (200 mL) was added and the excess NIS was quenched with 2 M sodium thiosulfate solution. The mixture was extracted with ethyl acetate (200 mL ×3), the combined organic phases were washed with saturated brine (100 mL) and dried over anhydrous sodium sulfate. The solvent was evaporated under reduced pressure and the product was purified by column chromatography (200 ~ 300 mesh silica gel, eluted with ethyl acetate:methanol = 80:1 ~ 20:1) to afford (S)-N-(10-hydroxy-11-iodo-1,2,3-trimethoxy-9-oxo-5,6,7,9-tetrahydrobenzo[a]heptalen-7-yl)acetamide **(10)** (4.0 g). The total yield of the two-step reaction of steps A and B was 59.6%.

Step C: Potassium carbonate (1.40 g, 10.1 mmol) and iodomethane (1.19 g, 8.38 mmol) were added to a solution of compound **10** (3.50 g, 6.85 mmol) in DMF (35 mL), and after addition, the resulting mixture was stirred at room temperature overnight. Water (140 mL) was added and the product was extracted with ethyl acetate (70 mL ×3). The combined organic phases were washed sequentially with water (40 mL × 2) and saturated saline (40 mL) and dried over anhydrous sodium sulfate. The solvent was evaporated under reduced pressure and the product was purified by column chromatography (200 ~ 300 mesh silica gel, eluted with petroleum ether:ethyl acetate:methanol = 1:1:0 ~ 0:30:1) to afford (S)-N-(11-iodo-1,2,3,9-tetramethoxy-10-oxo-5,6,7,10-tetrahydrobenzo[a]heptalen-7-yl)acetamide (11) (1.20 g, ethyl acetate:methanol=5:1, $R_f$ = 0.3) and (S)-N-(11-iodo-1,2,3,10-tetramethoxy-9-oxo-5,6,7,9-tetrahydrobenzo[a]heptalen-7-yl)acetamide **(12)** (270 mg, ethyl acetate:methanol = 5:1, $R_f$ = 0.5). The yields were 33.3% and 7.50%, respectively. Compound **12:** $^1$H NMR (DMSO-$d_6$, 400 MHz) δ 8.61 (d, J = 7.2 Hz, 1H), 7.78 (s, 1H), 7.07 (s, 1H), 6.79 (s, 1H), 4.29-4.23 (m, 1H), 3.90 (s, 3H), 3.85 (s, 3H), 3.81 (s, 3H), 3.64 (s, 3H), 2.64-2.59 (m, 1H), 2.29-2.25 (m, 1H), 2.07-2.03 (m, 1H), 1.85-1.79 (m, 4H). MS (ESI, m/z): 526.0 $[M+H]^+$.

Step D: To a solution of compound **11** (220 mg, 0.419 mmol) in DMF (5 mL) was added cuprous cyanide (100 mg, 1.12 mmol), and after addition, the resulting mixture was stirred at 100°C for 3 hours. Water (20 mL) was added and the product was extracted with ethyl acetate (20 mL ×3). The combined organic phases were washed sequentially with water (15 mL × 2) and saturated saline (10 mL) and dried over anhydrous sodium sulfate. The solvent was evaporated under reduced pressure and the product was purified by column chromatography (200 ~ 300 mesh silica gel, eluted with ethyl acetate:methanol = 40:1 ~ 10:1) to afford (S)-N-(11-cyano-1,2,3,9-tetramethoxy-10-oxo-5,6,7,10-tetrahydrobenzo[a]heptalen-7-yl)acetamide **(13).** $^1$H NMR (DMSO-$d_6$, 400 MHz) δ 8.78 (d, J = 7.6 Hz, 1H), 7.86 (s, 1H), 7.19 (s, 1H), 6.86 (s, 1H), 4.40-4.35 (m, 1H), 4.00 (s, 3H), 3.87 (s, 3H), 3.83 (s, 3H), 3.59 (s, 3H), 2.62-2.60 (m, 1H), 2.26-2.10 (m, 3H), 1.91 (s, 3H). MS (ESI, m/z): 425.1 $[M+H]^+$.

**Example 6: Synthesis of (S)-N-(11-bromo-1,2,3,10-tetramethoxy-9-oxo-5,6,7,9-tetrahydrobenzo[a]heptalen-7-yl)acetamide (14) and (S)-N-(11-bromo-1,2,3,9-tetramethoxy-10-oxo-5,6,7,10-tetrahydrobenzo[a]heptalen-7-yl)acetamide (15)**

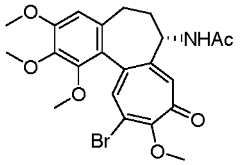

14

+

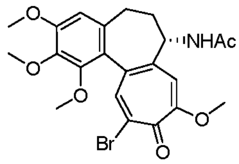

15

The experimental operations for the synthesis of compounds **14** and **15** using compound **9** as a raw material were described in Steps B and C of Example 5, wherein NIS in Step B of Example 5 was replaced with NBS. Compound **14:** $^1$H NMR (DMSO-$d_6$, 400 MHz) δ 8.61 (d, J = 7.6 Hz, 1H), 7.49 (s, 1H), 7.08 (s, 1H), 6.79 (s, 1H), 4.29-4.23 (m, 1H), 3.92 (s, 3H), 3.84 (s, 3H), 3.79 (s, 3H), 3.61 (s, 3H), 2.64-2.59 (m, 1H), 2.30-2.23 (m, 1H), 2.05-1.99 (m, 1H), 1.86-1.80 (m, 4H). MS (ESI, m/z): 478.0 $[M+H]^+$. Compound **15:** $^1$H NMR (DMSO-$d_6$, 400 MHz) δ 8.72 (d, J = 7.2 Hz, 1H), 8.19 (s, 1H), 7.14 (s, 1H), 6.84 (s, 1H), 4.35-4.30 (m, 1H), 3.94 (s, 3H), 3.85 (s, 3H), 3.80 (s, 3H), 3.59 (s, 3H), 2.59-2.56 (m, 1H), 2.22-2.05 (m, 3H), 1.89 (s, 3H). MS (ESI, m/z): 478.0 $[M+H]^+$.

**Example 7: Synthesis of (S)-N-(1,2,3,10-tetramethoxy-11-methyl-9-oxo-5,6,7,9-tetrahydrobenzo[a]heptalen-7-yl)acetamide (16)**

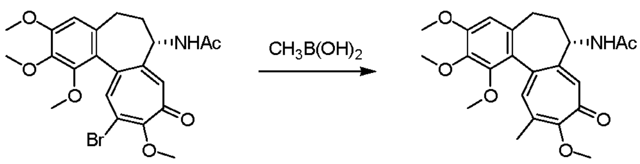


14 16

To a mixture containing compound **14** (150 mg, 0.314 mmol), methylboronic acid (38 mg, 0.635 mmol), potassium carbonate (216 mg, 1.57 mmol) and toluene (10 mL) was added

1,1'-bis(diphenylphosphino)ferrocene]dichloropalladium(II) (23 mg, 0.0314 mmol), after addition, the resulting mixture was stirred at 100°C under nitrogen overnight. The solvent was evaporated under reduced pressure and the product was purified by column chromatography (200 ~ 300 mesh silica gel, eluted with ethyl acetate:dichloromethane:methanol = 1:1:0 ~ 30:10:1) to afford

(S)-N-(1,2,3,10-tetramethoxy-11-methyl-9-oxo-5,6,7,9-tetrahydrobenzo [a]heptalen-7-yl)acetamide **(16).** $^1$H NMR ($CDCl_3$, 400 MHz) δ 7.63 (s, 1H), 7. 02 (s, 1H), 6.56 (s, 1H), 6.31 (d, J = 6.0 Hz, 1H), 4.64-4.59 (m, 1H), 3.95-3.91 (m, 9H), 3.66 (s, 3H), 2.53-2.48 (m, 1H), 2.40-2.27 (m, 5H), 2.06 (s, 3H), 2.00-1.93 (m, 1H). MS (ESI, m/z): 414.1

[M+H]+.

**Example 8: Synthesis of (S)-N-(1,2,3,9-tetramethoxy-11-methyl-10-oxo-5,6,7,10-tetrahydrobenzo[a]heptalen-7-yl)acetamide (17)**

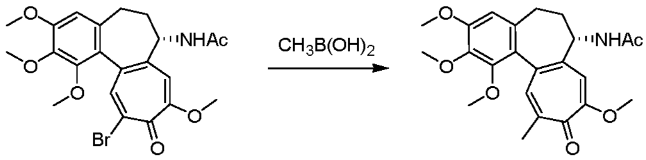


**15** **17**

See Example 7 for experimental operations to synthesize compound **17** using compound **15** as a raw material. $^1$H NMR (DMSO-$d_6$, 400 MHz) δ 8.56 (d, J = 7.6 Hz, 1H), 7.08 (s, 1H), 7. 07 (s, 1H), 6.78 (s, 1H), 4.32-4.26 (m, 1H), 3.85-3.80 (m, 9H), 3.58 (s, 3H), 2.63-2.58 (m, 1H), 2.30-2.21 (m, 4H), 2.04-1.98 (m, 1H), 1.85-1.77 (m, 4H). MS (ESI, m/z): 414.1 [M+H]+.

**Example 9: Synthesis of N-{(S)-1,2,3-trimethoxy-9-oxo-10-{[(S)-tetrahydrofuran-3-yl]oxy}-5,6,7,9-tetrahydrobenzo[a]heptalen-7-yl} acetamide (18) and N-{(S)-1,2,3-trimethoxy-10-oxo-9-{[(S)-tetrahydrofuran-3-yl]oxy}-5,6,7,10-tetrahydrobenzo[a]heptalen-7-yl }acetamide** (19)

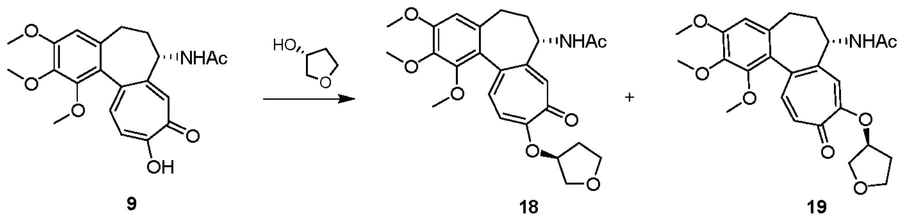

**9** **18** **19**

Diisopropyl azodicarboxylate (787 mg, 3.89 mmol) was added to a solution of compound **9** (500 mg, 1.30 mmol), triphenylphosphine (1.02 g, 3.89 mmol) and (R)-3-hydroxytetrahydrofuran (137 mg, 1.56 mmol) in THF (5 mL) under nitrogen. After addition, the resulting mixture was stirred overnight at room temperature. The solvent was evaporated under reduced pressure and the product was purified by preparative HPLC and SFC to afford N-{(S)-1,2,3-trimethoxy-9-oxo-10-{[(S)-tetrahydrofuran-3-yl]oxy}-5,6,7,9-tetrahydrobenzo[a]heptalen-7-yl}aceta mide **(18)** and N-{(S)-1,2,3-trimethoxy-10-oxo-9-{[(S)-tetrahydrofuran-3-yl]oxy}-5,6,7,10-tetrahydrobenzo[a]heptalen-7-yl}acetamide **(19).** Compound **18:** $^1$H NMR (DMSO-$d_6$, 400 MHz) δ 8.57 (d, J = 7.6 Hz, 1H), 7.13 (s, 1H), 7.11-7.03 (m, 2H), 6.77 (s, 1H), 5.15-5.13 (m, 1H), 4.36-4.29 (m, 1H), 3.95-3.91 (m, 1H), 3.87-3.82 (m, 5H), 3.78-3.73 (m, 4H), 3.52 (s, 3H), 2.61-2.57 (m, 1H), 2.33-2.20 (m, 2H), 2.07-1.95 (m, 2H), 1.88-1.86 (m, 4H). MS (ESI, m/z): 456.2 [M+H]+. Compound **19:** $^1$H NMR (DMSO-$d_6$, 400 MHz) δ 8.67 (d, J = 6.8 Hz, 1H), 7.22 (d, J = 12.8 Hz, 1H), 7.02 (s, 1H), 6.94 (d, J = 12.8 Hz, 1H), 6.80 (s, 1H), 5.12 (s, 1H), 4.34-4.30 (m, 1H), 3.99-3.95 (m, 1H), 3.91-3.78 (m, 9H), 3.56 (s, 3H), 2.59-2.56 (m, 1H), 2.35-2.26 (m, 1H), 2.21-2.03 (m, 3H), 1.93-1.87 (m, 4H). MS (ESI, m/z): 456.3 [M+H]+.

**Example 10: Synthesis of N-{(S)-11-iodo-1,2,3-trimethoxy-9-oxo-10-{[(R)-tetrahydrofuran-3-yl]oxy}-5,6,7,9-tetrahydrobenzo[a]heptal en-7-yl}acetamide (20), N-{(S)-11-iodo-1,2,3-trimethoxy-10-oxo-9-{[(R)-tetrahydrofuran-3-yl]oxy}-5,6,7,10-tetrahydrobenzo[a]hept ahydro-7-yl}acetamide (21), N-{(S)-11-iodo-1,2,3-trimethoxy-9-oxo-10-{[(S)-tetrahydrofuran-3-yl]oxy}-5,6,7,9-tetrahydrobenzo[a]heptal en-7-yl}acetamide (22) and N-{(S)-11-iodo-1,2,3-trimethoxy-10-oxo-9-{[(S)-tetrahydrofuran-3-yl]oxy}-5,6,7,10-tetrahydrobenzo[a]hepta len-7-yl}acetamide (23).**

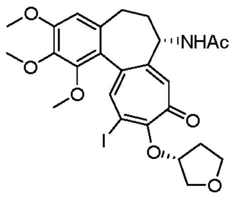
20

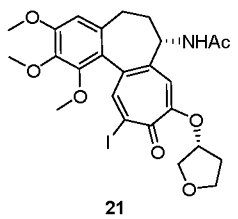
21

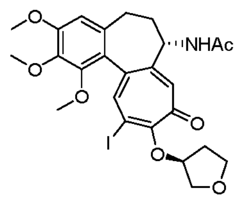
22

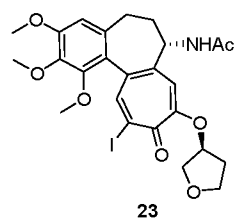
23

See Example 9 for experimental operations to synthesize compounds **20** and **21, 22** and **23** using compound 10 and (S)-3-hydroxytetrahydrofuran (or (R)-3-hydroxytetrahydrofuran). compound **20:** $^1$H NMR ($CDCl_3$, 400 MHz) δ 8.08 (s, 1H), 7.17 (s, 1H), 6.50 (s, 1H), 6.31 (d, J = 6.8 Hz, 1H), 5.71-5.68 (m, 1H), 4.60-4.53 (m, 1H), 4.20-4.14 (m, 1H), 4.02-3.83 (m, 9H), 3.73 (s, 3H), 2.55-2.50 (m, 1H), 2.46-2.34 (m, 2H), 2.27-2.14 (m, 2H), 2.00 (s, 3H), 1.79-1.72 (m, 1H). MS (ESI, m/z): 582.2 $[M+H]^+$. compound **21:** $^1$H NMR ($CDCl_3$, 400 MHz) δ 8.70 (s, 1H), 7.16 (s, 1H), 6.56 (s, 1H), 6.18 (d, J = 6.0 Hz, 1H), 5.35-5.33 (m, 1H), 4.53-4.47 (m, 1H), 4.01-3.89 (m, 10H), 3.72 (s, 3H), 2.54-2.47 (m, 1H), 2.38-2.27 (m, 2H), 2.22-2.13 (m, 2H), 2.04-1.92 (m, 4H). MS (ESI, m/z): 582.2 $[M+H]^+$. compound **22:** $^1$H NMR ($CDCl_3$, 400 MHz) δ 8.09 (s, 1H), 7.19 (s, 1H), 6.50 (s, 1H), 6.36 (d, J = 6.8 Hz, 1H), 5.64-5.62 (m, 1H), 4.58-4.52 (m, 1H), 4.18-4.08 (m, 3H), 3.97-3.89 (m, 7H), 3.74 (s, 3H), 2.55-2.50 (m, 1H), 2.45-2.37 (m, 1H), 2.27-2.18 (m, 2H), 2.10-2.00 (m, 5H). MS (ESI, m/z): 582.1 $[M+H]^+$. compound **23:** $^1$H NMR ($CDCl_3$, 400 MHz) δ 8.70 (s, 1H), 7.20 (s, 1H), 6.56 (s, 1H), 6.31 (s, 1H), 5.25 (s, 1H), 4.51-4.48 (m, 1H), 4.06-3.85 (m, 10H), 3.72 (s, 3H), 2.53-2.51 (m, 1H), 2.38-2.29 (m, 2H), 2.23-2.16 (m, 2H), 2.05-1.96 (m, 4H). MS (ESI, m/z): 582.1 $[M+H]^+$.

**Example 11: Synthesis of N-{(S)-1,2,3-trimethoxy-9-oxo-10-{[(R)-tetrahydrofuran-3-yl]oxy}-5,6,7,9-tetrahydrobenzo[a]heptalen-7-yl} acetamide (24), N-{(S)-1,2,3-trimethoxy-10-oxo-9-{[(R)-tetrahydrofuran-3-yl]oxy}-5,6,7,10-tetrahydrobenzo[a]heptalen-7-yl }acetamide (25), (S)-N-(1,2,3-trimethoxy-9-oxo-10-[(tetrahydrofuran-3-yl)oxy]-5,6,7,9-tetrahydrobenzo[a]heptalen-7-yl)acet amide (26) and (S)-N-(1,2,3-trimethoxy-10-oxo-9-[( tetrahydrofuran-3-yl)oxy]-5,6,7,10-tetrahydrobenzo[a]heptalen-7-yl)ace tamide (27)**

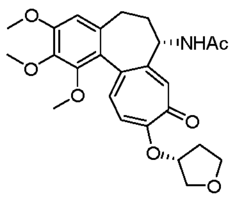
24

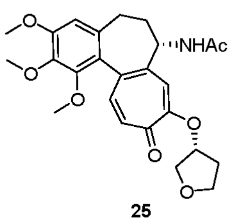
25

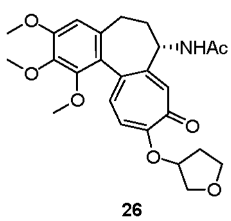
26

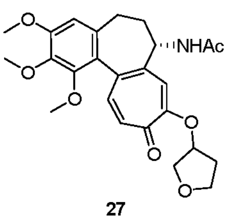
27

See Example 9 for experimental operations to synthesize compounds **24, 25, 26** and **27** using compound **9** and (S)-3-hydroxytetrahydrofuran (or 3-hydroxytetrahydrofuran) as raw materials, compound **24:** $^1$H NMR (DMSO-$d_6$, 400 MHz) δ 8.57 (d, J = 7.6 Hz, 1H), 7.13 (s, 1H), 7.10-7.02 (m, 2H), 6.77 (s, 1H), 5.16-5.13 (m, 1H), 4.35-4.30 (m, 1H), 3.95-3.91 (m, 1H), 3.86-3.82 (m, 5H), 3.79-3.75 (m, 4H), 3.53 (s, 3H), 2.61-2.57 (m, 1H), 2.34-2.18 (m, 2H), 2.04-2.00 (m, 2H), 1.92-1.84 (m, 4H). MS (ESI, m/z): 456.4 $[M+H]^+$. compound **25:** $^1$H NMR (DMSO-$d_6$, 400 MHz) δ 8.65 (d, J = 7.2 Hz, 1H), 7.22 (d, J = 12.8 Hz, 1H), 6.96-6.93 (m, 2H), 6.81 (s, 1H), 5.16-5.13 (m, 1H), 4.34-4.29 (m, 1H), 3.99-3.95 (m, 1H), 3.93-3.83 (m, 4H), 3.81-3.74 (m, 5H), 3.56 (s, 3H), 2.58-2.55 (m, 1H), 2.36-2.28 (m, 1H), 2.22-2.02 (m, 4H), 1.88 (s, 3H). MS (ESI, m/z): 456.4 $[M+H]^+$. compound **26:** $^1$H NMR (DMSO-$d_6$, 400 MHz) δ 8.58 (d, J = 7.2 Hz, 1H), 7.13-7.02 (m, 3H), 6.77 (s, 1H), 5.14 (s, 1H), 4.34-4.29 (m, 1H), 3.96-3.91 (m, 1H), 3.87-3.73 (m, 9H), 3.52 (s, 3H), 2.61-2.57 (m, 1H), 2.33-2.18 (m, 2H), 2.04-1.96 (m, 2H), 1.87-1.79 (m, 4H). MS (ESI, m/z): 456.1 $[M+H]^+$. compound **27:** $^1$H NMR (DMSO-$d_6$, 400 MHz) δ 8.68-8.65 (m, 1H), 7.24-7.21 (m, 1H), 7.02-6.93 (m, 2H), 6.81 (s, 1H), 5.14 (s, 1H), 4.33-4.28 (m, 1H), 3.99-3.95 (m, 1H), 3.91-3.74 (m, 10H), 3.56 (s, 3H), 2.60-2.54 (m, 1H), 2.34-2.30 (m, 1H), 2.19-2.03 (m, 2H), 1.92-1.84 (m, 4H). MS (ESI, m/z): 456.2 $[M+H]^+$.

**Example 12: Synthesis of N-{(S)-1,2,3-trimethoxy-11-methyl-9-oxo-10-{[(R)-tetrahydrofuran-3-yl]oxy}-5,6,7,9-tetrahydrobenzo[a]hep talen-7-yl}acetamide (28), N-{(S)-1,2,3-trimethoxy-11-methyl-10-oxo-9-{[(R)-tetrahydrofuran-3-yl]oxy}-5,6,7,10-tetrahydrobenzo[a]he ptalen-7-yl}acetamide (29), N-{(S)-1,2,3-trimethoxy-11-methyl-9-oxo-10-{[(S)-tetrahydrofuran-3-yl]oxy}-5,6,7,9-tetrahydrobenzo[a]hep talen-7-yl}acetamide (30) and N-{(S)-1,2,3-trimethoxy-11-methyl-10-oxo-9-{[(S)-tetrahydrofuran-3-yl]oxy}-5,6,7,10-tetrahydrobenzo[a]he ptalen-7-yl}acetamide (31).**

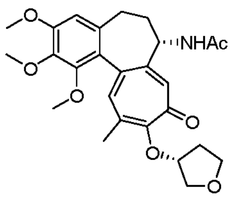
**28**

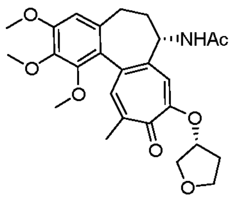
**29**

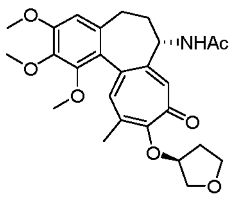
**30**

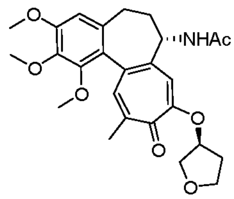
**31**

See Example 7 for experimental operations to synthesize compound **28** (or **29, 30, 31)** using compound **20** (or 21, 22, 23) as a raw material, respectively. compound **28:** $^1$H NMR (DMSO-d6, 400 MHz) δ 8.56 (d, J = 7.6 Hz, 1H), 7.09 (d, J = 4.4 Hz, 2H), 6.77 (s, 1H), 5.48-5.46 (m, 1H), 4.33-4.27 (m, 1H), 3.93-3.63 (m, 10H), 3.58 (s, 3H), 2.62-2.57 (m, 1H), 2.34-2.20 (m, 4H), 2.15-1.98 (m, 3H), 1.85-1.77 (m, 4H). MS (ESI, m/z): 470.1 $[M+H]^+$. compound **29:** $^1$H NMR (DMSO-d6, 400 MHz) δ 8.62 (d, J = 7.6 Hz, 1H), 7.45 (d, J = 0.8 Hz, 1H), 6.98 (s, 1H), 6.79 (s, 1H), 5.13-5.10 (m, 1H), 4.32-4.26 (m, 1H), 3.97-3.74 (m, 10H), 3.57 (s, 3H), 2.56-2.54 (m, 1H), 2.32-2.24 (m, 1H), 2.21 (s, 3H), 2.18-1.97 (m, 4H), 1.87 (s, 3H). MS (ESI, m/z): 470.1 $[M+H]^+$. compound **30:** $^1$H NMR (DMSO-d6, 400 MHz) δ 8.57 (d, J = 7.6 Hz, 1H), 7.09 (d, J = 5.6 Hz, 2H), 6.76 (s, 1H), 5.45-5.42 (m, 1H), 4.32-4.26 (m, 1H), 3.90-3.73 (m, 10H), 3.58 (s, 3H), 2.61-2.56 (m, 1H), 2.28-2.20 (m, 4H), 2.07-1.90 (m, 3H), 1.84-1.76 (m, 4H). MS (ESI, m/z): 470.4 $[M+H]^+$. compound **31:** $^1$H NMR (DMSO-d6, 400 MHz) δ 8.65 (d, J = 7.6 Hz, 1H), 7.45 (d, J = 1.2 Hz, 1H), 7.05 (s, 1H), 6.80 (s, 1H), 5.12-5.09 (m, 1H), 4.34-4.28 (m, 1H), 3.98-3.95 (m, 1H), 3.91-3.78 (m, 9H), 3.58 (s, 3H), 2.57-2.53 (m, 1H), 2.34-2.27 (m, 1H), 2.22 (s, 3H), 2.18-2.00 (m, 3H), 1.94-1.88 (m, 4H). MS (ESI, m/z): 470.1 $[M+H]^+$.

**Example 13: Synthesis of methyl (S)-7-acetamido-1,2,3-trimethoxy-9-oxo-5,6,7,9-tetrahydrobenzo[a]heptalen-10-carboxylate (34) and methyl (S)-7-acetamido-1,2,3-trimethoxy-10-oxo-5,6,7,10 tetrahydrobenzo[a]heptalen-9-carboxylate (35)**

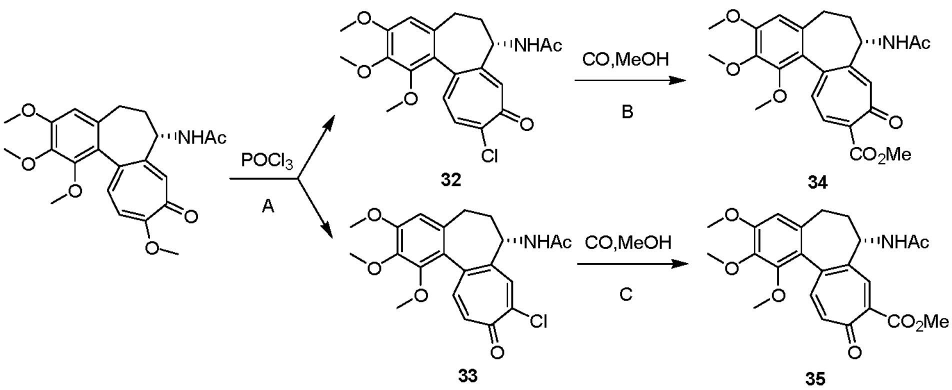

**32** **34** **33** **35**

Step A: A mixture containing phosphorus oxychloride (800 mg, 5.22 mmol), colchicine (2.0 g, 5.01 mmol) and DMF (40 mL) was stirred at room temperature for 4 hours. The reaction was quenched with cold water (100 mL) and then extracted with ethyl acetate (50 mL ×3). The combined organic phases were washed with saturated saline (30 mL ×3) and dried over anhydrous sodium sulfate. The solvent was evaporated under reduced pressure and the product was purified by column chromatography (200 ~ 300 mesh silica gel, eluted with dichloromethane: methanol = 30:1 ~10:1) to afford

(S)-N-(10-chloro-1,2,3-trimethoxy-9-oxo-5,6,7,9-tetrahydrobenzo[a]heptalen-7-yl)acetatnide **(32)** and
(S)-N-(9-chloro-1,2,3-trimethoxy- 10-oxo-5,6,7,10-tetrahydrobenzo[a]heptalen-7-yl)acetamide **(33).**

Step B: To a solution of compound **32** (2.40 g, 5.94 mmol) in methanol (40 mL) was added diisopropylethylamine (768 mg, 5.94 mmol) and 1,1'-bis(diphenylphosphino)ferrocene]dichloropalladium(II) (1.0 g, 1.37 mmol), and after addition the resulting mixture was stirred at 80°C under carbon monoxide (50 psi) at 80°C with stirring overnight. Methyl (S)-7-acetamido-1,2,3-trimethoxy-9-oxo-5,6,7,9-tetrahydrobenzo[a]heptalen-10-carboxylate **(34)** was then obtained by preparative HPLC and SFC separation. $^1$H NMR (DMSO-d6, 400 MHz) δ 8.61 (d, J = 7.2 Hz, 1H), 7.65 (d, J = 9.2 Hz, 1H), 7.13 (d, J = 9.2 Hz, 1H), 7.09 (s, 1H), 6.80 (s, 1H), 4.29-4.23 (m, 1H), 3.85 (s, 3H), 3.79 (s, 3H), 3.78 (s, 3H), 3.57 (s, 3H), 2.68-2.61 (m, 1H), 2.29-2.24 (m, 1H), 2.06-1.96 (m, 1H), 1.85-1.76 (m, 4H). MS (ESI, m/z): 428.0 [M+H]$^+$.

The experimental operations of step C was described in step B. Methyl (S)-7-acetamido-1,2,3-trimethoxy-10-oxo-5,6,7,10 tetrahydrobenzo[a]heptalen-9-carboxylate (35) was obtained. $^1$H NMR (DMSO-d6, 400 MHz) δ 8.59 (d, J = 7.2 Hz, 1H), 7.65 (s, 1H), 7.24 (d, J = 12.8 Hz, 1H), 6.96 (d, J = 12.8 Hz, 1H), 6.85 (s, 1H), 4.26-4.19 (m, 1H), 3.86 (s, 3H), 3.80 (s, 3H), 3.79 (s, 3H), 3.61 (s, 3H), 2.63-2.58 (m, 1H), 2.26-2.05 (m, 3H), 1.84 (s, 3H). MS (ESI, m/z): 428.1 [M+H]$^+$.

**Example 14: Synthesis of (S)-N-{1,2,3-trimethoxy-9-oxo-10-(2,2,2-trifluoroethoxy)-5,6,7,9-tetrahydrobenzo[a]heptalen-7-yl}acetamide (36) and (S)-N-{1,2,3-trimethoxy-10-oxo-9-(2,2,2-trifluoroethoxy)-5,6,7,10-tetrahydrobenzo[a]heptalen-7-yl}acetami de (37)**

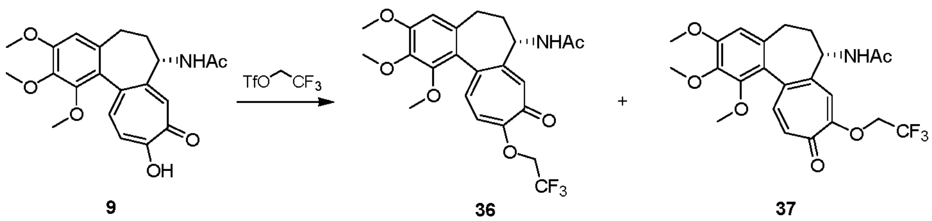


9 36 37

A mixture containing compound 9 (500 mg, 1.30 mmol), potassium carbonate (717 mg, 5.19 mmol), acetonitrile (1.5 mL), 2,2,2-trifluoroethyl trifluoromethanesulfonate (602 mg, 2.59 mmol) and DMF (3.5 mL) was reacted for 1.5 h at 150°C in microwave. Water (20 mL) was added and the resulting mixture was extracted with ethyl acetate (30 mL ×3). The combined organic phases were washed with saturated saline (20 mL ×3) and dried over anhydrous sodium sulfate. The solvent was evaporated under reduced pressure and the product was separated by preparative HPLC and SFC to afford

(S)-N-{1,2,3-trimethoxy-9-oxo-10-(2,2,2-trifluoroethoxy)-5,6,7,9-tetrahydrobenzo[a]heptalen-7-yl}acetamide (36) (290 mg) and
(S)-N-{1,2,3-trimethoxy-10-oxo-9-(2,2,2-trifluoroethoxy)-5,6,7,10-tetrahydrobenzo[a]heptalen-7-yl}acetamide **(37)** (46 mg). The yields were 47.7% and 7.57%, respectively. Compound **36:** $^1$H NMR (DMSO-d6, 400 MHz) δ 8.61 (d, J = 7.6 Hz, 1H), 7.19-7.17 (m, 2H), 7.08 (d, J = 10.4 Hz, 1H), 6.79 (s, 1H), 4.94-4.78 (m, 2H), 4.36-4.30 (m, 1H), 3.84 (s, 3H), 3.79 (s, 3H), 3.53 (s, 3H), 2.63-2.58 (m, 1H), 2.26-2.17 (m, 1H), 2.07-1.97 (m, 1H), 1.87-1.78 (m, 4H). MS (ESI, m/z): 468.1 [M+H]$^+$. compound 37: $^1$H NMR (DMSO-d6, 400 MHz) δ 8.59 (d, J = 7.6 Hz, 1H), 7.30 (d, J = 12.8 Hz, 1H), 7.21 (s, 1H), 7.03 (d, J = 12.8 Hz, 1H), 6.83 (s, 1H), 4.93-4.76 (m, 2H), 4.38-4.31 (m, 1H), 3.85 (s, 3H), 3.79 (s, 3H), 3.58 (s, 3H), 2.60-2.56 (m, 1H), 2.21-2.02 (m, 3H), 1.87 (s, 3H). MS (ESI, m/z): 468.0 [M+H]$^+$.

**Example 15: Synthesis of (S)-N-(10-cyano-1,2,3-trimethoxy-9-oxo-5,6,7,9-tetrahydrobenzo[a]heptalen-7-yl)acetamide (38)**

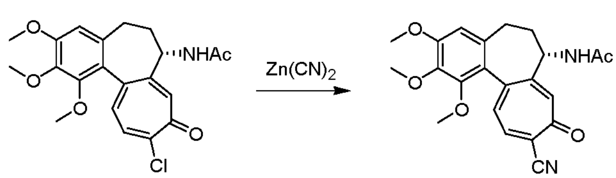


32 38

A solution containing compound **32** (1.0 g, 2.48 mmol), zinc cyanide (880 mg, 7.49 mmol), tetrakis(triphenylphosphine)palladium (572 mg, 0.495 mmol) and DMF (20 mL) was stirred for 1 h at 100°C under nitrogen. After the mixture was cooled to room temperature, water (100 mL) was added and the mixiture was extracted with ethyl acetate (100 mL ×2). The combined organic phases were washed with saturated saline (50 mL ×2) and dried over anhydrous sodium sulfate. The solvent was evaporated under reduced pressure and the product was separated by preparative HPLC and SFC to give (S)-N-(10-cyano-1,2,3-trimethoxy-9-oxo-5,6,7,9-tetrahydrobenzo[a]heptalen-7-yl)acetamide **(38).** $^1$H NMR (DMSO-d6, 400 MHz) δ 8.63 (d, J = 7.2 Hz, 1H), 8.07 (d, J = 9.6 Hz, 1H), 7.16-7.12 (m, 2H), 6.81 (s, 1H), 4.30-4.23 (m, 1H), 3.82 (s, 3H), 3.79 (s, 3H), 3.59 (s, 3H), 2.67-2.62 (m, 1H), 2.33-2.28 (m, 1H), 2.03-2.00 (m, 1H), 1.84-1.80 (m, 4H). MS (ESI, m/z): 395.4 [M+H]$^+$.

**Example 16: Synthesis of (S)-N-(9-cyano-1,2,3-trimethoxy-10-oxo-5,6,7,10-tetrahydrobenzo[a]heptalen-7-yl)acetamide (39)**

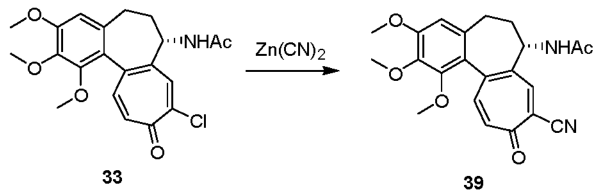


33 39

See Example 15 for experimental operations to synthesize compound **39** using compound **33** as a raw material. $^1$H NMR (DMSO-d6, 400 MHz) δ 8.57 (d, J = 7.2 Hz, 1H), 7.94 (s, 1H), 7.34 (d, J = 12.8 Hz, 1H), 7.04 (d, J = 12.8 Hz, 1H), 6.87 (s, 1H), 4.28-4.22 (m, 1H), 3.86 (s, 3H), 3.79 (s, 3H), 3.64 (s, 3H), 2.61-2.58 (m, 1H), 2.24-2.08 (m, 3H), 1.87 (s, 3H). MS (ESI, m/z): 395.3 [M+H]$^+$.

**Example 17: Synthesis of methyl (S)-7-acetamido-1,2,3,10-tetramethoxy-9-oxo-5,6,7,9-tetrahydrobenzo[a]heptalen-11-carboxylate (40)**

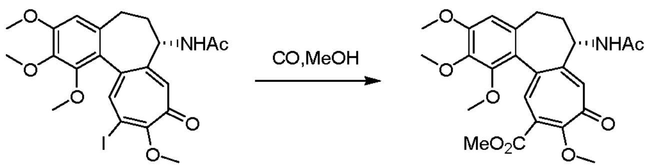


12 40

See step B in Example 13 for experimental operations to synthesize compound **40** using compound **12** as a raw material. $^1$H NMR (DMSO-d6, 400 MHz) δ 8.62 (d, J = 7.6 Hz, 1H), 7.18 (s, 1H), 6.94 (s, 1H), 6.79 (s, 1H), 4.30-4.26 (m, 1H), 3.88 (s, 3H), 3.84 (s, 6H), 3.78 (s, 3H), 3.56 (s, 3H), 2.62-2.59 (m, 1H), 2.26-2.24 (m, 1H), 2.03-2.01 (m, 1H), 1.85-1.77 (m, 4H). MS (ESI, m/z): 458.4 [M+H]$^+$.

**Example 18: Synthesis of (S)-1-methyl-(1,2,3,10-tetramethoxy-11-methyl-9-oxo-5,6,7,9-tetrahydrobenzo[a]heptalen-7-yl)urea (44)**

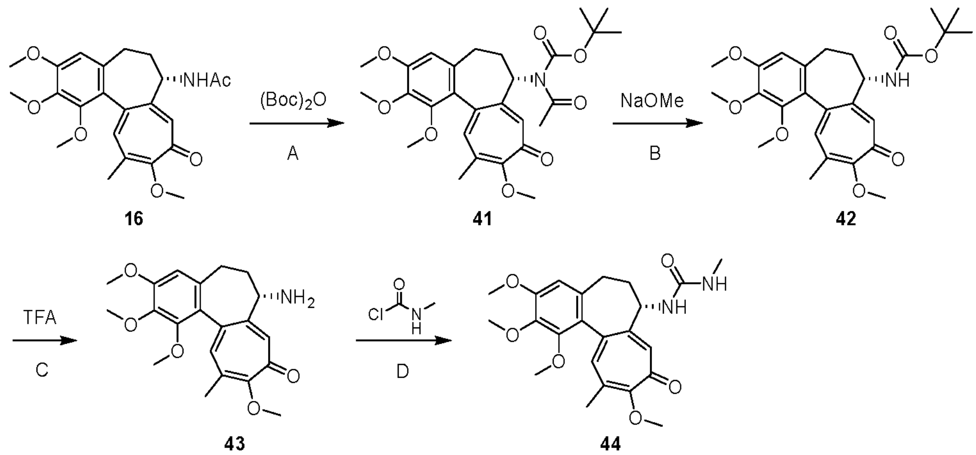


16 41 42 43 44

Step A: A mixture containing compound **16** (1.35 g, 3.27 mmol), triethylamine (330 mg, 3.27 mmol), DMAP (800 mg, 6.55 mmol), di-tert-butyl dicarbonate (2.30 g, 10.5 mmol) and acetonitrile (13 mL) was stirred for 2 hours at 80°C, and then stirred at reflux overnight after di-tert-butyl dicarbonate ( 1.40 g, 6.41 mmol) was added. The solvent was evaporated under reduced pressure and the product was purified by column chromatography (200 ~ 300 mesh silica gel, eluted with petroleum ether:ethyl acetate = 2:3) to give tert-butyl (S)-acetyl (1,2,3,10-tetramethoxy-11-methyl-9-oxo-5,6,7,9-tetrahydrobenzo[a]heptalen-7-yl)carbamate **(41)** (921 mg). The yield was 54.8 %. $^1$H NMR (DMSO-d6, 400 MHz) $\delta$ 7.18 (s, 1H), 7.06 (s, 1H), 6.78 (s, 1H), 4.91-4.87 (m, 1H), 3.84 (s, 6H), 3.78 (s, 3H), 3.57 (s, 3H), 2.72-2.67 (m, 1H), 2.54-2.47 (m, 1H), 2.36-2.31 (m, 1H), 2.28 (s, 3H), 2.24 (s, 3H), 1.95-1.87 (m, 1H), 1.49 (s, 9H).

Step B: A solution of compound **41** (1.10 g, 2.14 mmol) and sodium methanolate (463 mg, 8.57 mmol) in methanol (8 mL) was stirred at 40°C for 1 hour. Saturated ammonium chloride solution (25 mL) was added, the mixture was extracted with ethyl acetate (30 mL ×3) and dried over anhydrous sodium sulfate. The solvent was evaporated under reduced pressure to give tert-butyl (S)-(1,2,3,10-tetramethoxy-11-methyl-9-oxo-5,6,7,9-tetrahydrobenzo[a]heptalen-7-yl)carbamate **(42)** (900 mg). The yield was 89.2%.

Step C: A mixture containing compound 42 (900 mg, 1.91 mmol), trifluoroacetic acid (3 mL) and dichloromethane (10 mL) was stirred at room temperature for 4 hours. Water (30 mL) was added and the pH was adjusted to 7 ~ 8 with saturated sodium bicarbonate solution, the mixture was extracted with ethyl acetate (30 mL ×3) and dried over anhydrous sodium sulfate. The solvent was evaporated under reduced pressure and the product was purified by column chromatography (200 ~ 300 mesh silica gel, eluted with dichloromethane:methanol = 200:3, supplemented by 0.2% triethylamine) to give (S)-7-amino-1,2,3,10-tetramethoxy-11-methyl-6,7-dihydrobenzo[a]heptalen-9(SH)-one **(43)** (490 mg). The yield was 69.1%.

Step D: Methylcarbamoyl chloride (102 mg, 1.09 mmol) was added dropwise to a solution of compound **43** (170 mg, 0.458 mmol) and triethylamine (139 mg, 1.38 mmol) in dichloromethane (5 mL) in an ice-water bath, and after addition, the resulting mixture was stirred at room temperature overnight, then heated up to 35 °C and continued stirring for 2 hours. Water (15 mL) was added, and the mixture was extracted with dichloromethane (30 mL ×2) and dried over anhydrous sodium sulfate. The solvent was evaporated under reduced pressure and the product was purified by column chromatography (200 ~ 300 mesh silica gel, eluted with petroleum ether: ethyl acetate = 1:1 ~ 2:3) to give (S)-1-methyl-(1,2,3,10-tetramethoxy-11-methyl-9-oxo-5,6,7,9-tetrahydrobenzo[a]heptalen-7-yl)urea **(44).** $^1$H NMR (DMSO-d6, 400 MHz) $\delta$ 7.83 (s, 1H), 7.25 (s, 1H), 6.81 (s, 1H), 6.61 (d, J = 8.4 Hz, 1H), 5.77-5.73 (m, 1H), 4.43-4.36 (m, 1H), 3.85 (s, 6H), 3.79 (s, 3H), 3.50 (s, 3H), 2.57-2.53 (m, 7H), 2.21-2.16 (m, 1H), 2.05-1.96 (m, 1H), 1.81-1.73 (m, 1H). MS (ESI, m/z): 429.1 [M+H]$^+$.

Example 19: Synthesis of (S)-N-(1,2,3,10-tetramethoxy-11-methyl-9-oxo-5,6,7,9-tetrahydrobenzo[a]heptalen-7-yl)propanamide (45)

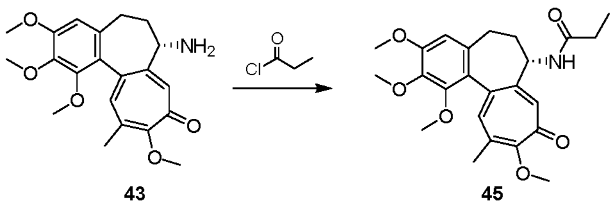

43 45

The experimental operations for the synthesis of compound **45** were described in Step D of Example 18, wherein methylcarbamoyl chloride was replaced with propionyl chloride in Step D of Example 18. $^1$H NMR (DMSO-d6, 400 MHz) δ 8.45 (d, J = 8.4 Hz, 1H), 7.83 (s, 1H), 7.29 (s, 1H), 6.84 (s, 1H), 4.56-4.49 (m, 1H), 3.87 (s, 6H), 3.81 (s, 3H), 3.53 (s, 3H), 2.58-2.55 (m, 4H), 2.22-2.15 (m, 3H), 2.09-2.01 (m, 1H), 1.96-1.88 (m, 1H), 1.02 (t, J = 7.6 Hz, 3H). MS (ESI, m/z): 428.1 [M+H]$^+$.

**Example 20: (S)-7-acetamido-1,2,3-trimethoxy-9-oxo-5,6,7,9-tetrahydrobenzo[a]heptalen-10-carboxylic acid (46), (S)-7-acetamido-1,2,3-trimethoxy-10-oxo-5,6,7,10-tetrahydrobenzo[a]heptalen-9-carboxylic acid (47), (S)-7-acetamido-1,2,3-trimethoxy-N-methyl-9-oxo-5,6,7,9-tetrahydrobenzo[a]heptalene-10-carboxamide (48) and (S)-7-acetamido-1,2,3-trimethoxy-N-methyl-10-oxo-5,6,7,10-tetrahydrobenzo[a]heptalen-9-carboxamide (49).**

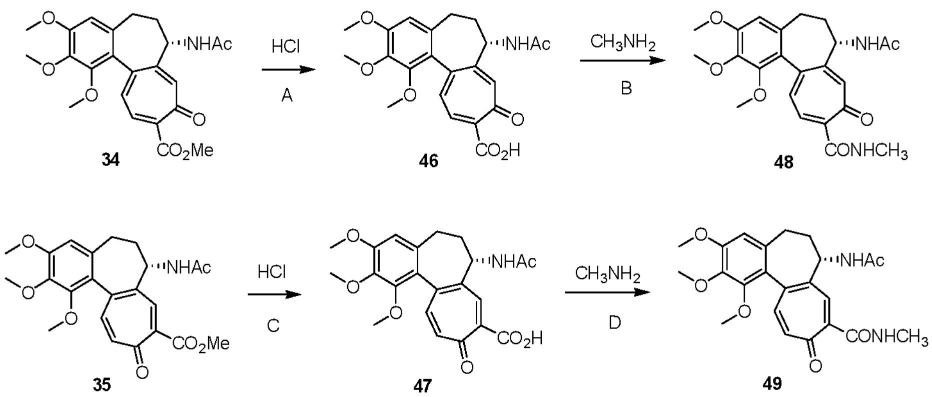


34 46 48

35 47 49

Step A: A mixture containing compound **34** (2.03 g, 2.34 mmol), 6 M hydrochloric acid (8.83 mL) and acetic acid (10 mL) was stirred for 4 hours at room temperature. Water (40 mL) was added, and the mixture was extracted with dichloromethane (20 mL ×3) and dried over anhydrous sodium sulfate. The solvent was evaporated under reduced pressure and the product was purified by preparative HPLC to give (S)-7-acetamido-1,2,3-trimethoxy-9-oxo-5,6,7,9-tetrahydrobenzo[a]heptalen-10-carboxylic acid **(46).** $^1$H NMR (DMSO-d6, 400 MHz) δ 8.68 (d, J = 7.2 Hz, 1H), 8.26 (d, J = 10.0 Hz, 1H), 8.41 (d, J = 10.0 Hz, 1H), 7.38 (s, 1H), 6.83 (s, 1H), 4.36-4.30 (m, 1H), 3.86 (s, 3H), 3.80 (s, 3H), 3.59 (s, 3H), 2.67-2.63 (m, 1H), 2.26-2.23 (m, 1H), 2.06-2.03 (m, 1H), 1.90-1.82 (m, 4H). MS (ESI, m/z): 414.0 [M+H]$^+$.

Step B: To a mixture containing Compound **46** (1.0 g, 2.42 mmol), diisopropylethylamine (1.56 g, 12.1 mmol) and DMF (10 mL) was added HATU (1.38 g, 3.63 mmol) under an ice-water bath, and after addition, it was stirred for 0.5 h at room temperature. Then methylamine hydrochloride (196 mg, 2.90 mmol) was added and then stirring was continued for 2 hours. Water (40 mL) was added and the product was extracted with ethyl acetate (20 mL ×3), the combined organic phases were washed with saturated saline (20 mL ×3) and dried over anhydrous sodium sulfate. The solvent was evaporated under reduced pressure and the product was purified by preparative HPLC and SFC to give (S)-7-acetamido-1,2,3-trimethoxy-N-methyl-9-oxo-5,6,7,9-tetrahydrobenzo[a]heptalen-10-carboxatnide **(48).** $^1$H NMR (DMSO-d6, 400 MHz) δ 9.24 (q, J = 4.4 Hz, 1H), 8.63 (d, J = 7.2 Hz, 1H), 8.19 (d, J = 10.0 Hz, 1H), 7.29 (d, J = 10.0 Hz, 1H), 7.23 (s, 1H), 6.83 (s, 1H), 4.33-4.27 (m, 1H), 3.85 (s, 3H), 3.79 (s, 3H), 3.58 (s, 3H), 2.81 (d, J = 4.4 Hz, 3H), 2.67-2.62 (m, 1H), 2.29-2.20 (m, 1H), 2.08-2.00 (m, 1H), 1.88-1.78 (m, 4H). MS (ESI, m/z): 427.4 [M+H]$^+$.

The experimental operations of Steps C and D are described in turn in Steps A and B, wherein Compound **34** in Step A was replaced with Compound **35** to give (S)-7-acetamido-1,2,3-trimethoxy-10-oxo-5,6,7,10-tetrahydrobenzo[a]heptalen-9-carboxylic acid **(47)** and (S)-7-acetamido-1,2,3-trimethoxy-N-methyl-10-oxo-5,6,7,10-tetrahydrobenzo[a]heptalen-9-carboxyfic acid **(49).** Compound **47:** $^1$H NMR (DMSO-d6, 400 MHz) δ 8.78 (d, J = 7.2 Hz, 1H), 8.42 (s, 1H), 7.50 (d, J = 12.8 Hz, 1H), 7.26 (d, J = 12.8 Hz, 1H), 6.88 (s, 1H), 4.29-4.23 (m, 1H), 3.87 (s, 3H), 3.80 (s, 3H), 3.62 (s, 3H), 2.62-2.58 (m, 1H), 2.26-2.07 (m, 3H), 1.85 (s, 3H). MS (ESI, m/z): 414.0 [M+H]$^+$. Compound 49: $^1$H NMR (DMSO-

d6, 400 MHz) δ 9.31 (q, J = 4.4 Hz, 1H), 8.72 (d, J = 6.8 Hz, 1H), 8.42 (s, 1H), 7.28 (d, J = 12.8 Hz, 1H), 7.07 (d, J = 12.8 Hz, 1H), 6.85 (s, 1H), 4.29-4.22 (m, 1H), 3.86 (s, 3H), 3.80 (s, 3H), 3.60 (s, 3H), 2.82 (d, J = 4.4 Hz, 3H), 2.62-2.57 (m, 1H), 2.21-2.06 (m, 3H), 1.84 (s, 3H). MS (ESI, m/z): 427.4 $[M+H]^+$.

**Example 21: Synthesis of (S)-N-(1,2,3-trimethoxy-10-trideutero-methoxy-11-methyl-9-oxo-5,6,7,9-tetrahydrobenzo[a]heptalen-7-yl)ac etamide (51)**

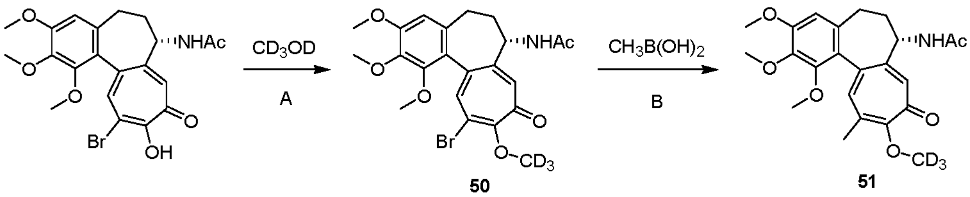


50

51

Step A: To a solution of (S)-N-(11-bromo-10-hydroxy-1,2,3-trimethoxy-9-oxo-5,6,7,9-tetrahydrobenzo[a]heptalen-7-yl)acetamide (1.60 g, 3.43 mmol), deuterated methanol (0.5 mL) and triphenylphosphine (2.70 g, 10.3 mmol) in THF (32 mL) was added diisopropyl azodicarboxylate (2.08 g, 10.3 mmol) under an ice-water bath, and after addition, the resulting mixture was stirred under nitrogen at room temperature overnight. The solvent was evaporated under reduced pressure and the product was purified by column chromatography (200 ~ 300 mesh silica gel, eluted with petroleum ether: ethyl acetate = 1:1 ~ 0:1) to afford (S)-N-(11-bromo-1,2,3-trimethoxy-10-trideutero-methoxy-9-oxo-5,6,7,9-tetrahydrobenzo[a]heptalen-7-yl)acetami de (50) (589 mg). The yield was 35.7%.

The experimental operations of step B were described in Example 7 to afford (S)-N-(1,2,3-trimethoxy-10-trideutero-methoxy-11-methyl-9-oxo-5,6,7,9-tetrahydrobenzo[a]heptalen-7-yl)acetam ide (51). $^1H$ NMR (DMSO-$d_6$, 400 MHz) δ 8.54 (d, J = 7.6 Hz, 1H), 7.07 (s, 1H), 7.06 (s, 1H), 6.76 (s, 1H), 4.31-4.25 (m, 1H), 3.83 (s, 3H), 3.79 (s, 3H), 3.57 (s, 3H), 2.61-2.56 (m, 1H), 2.29-2.21 (m, 4H), 2.05-1.95 (m, 1H), 1.84-1.76 (m, 4H). MS (ESI, m/z): 417.2 $[M+H]^+$.

**Example 22: Synthesis of (S)-N-[1,2,3-trimethoxy-9-oxo-10-(2-oxo-pyrrolidin-1-yl)-5,6,7,9-tetrahydrobenzo[a]heptalen-7-yl]acetamid e (52)**

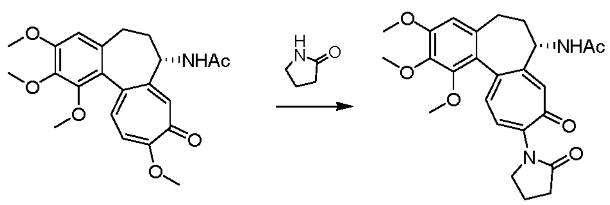

52

Under an ice-water bath, 60% sodium hydride (120 mg, 3.00 mmol) was added in batches to asolution of pyrrolidone (320 mg, 3.76 mmol) in THF (10 mL), then stirring was continued at this temperature for 0.5 h. Colchicine (1.0 g, 2.50 mmol) was then added. After addition, the resulting mixture was stirred at room temperature for 3 hours. Saturated ammonium chloride solution (20 mL) was added to the reaction mixture, which was extracted with ethyl acetate (20 mL ×3). The combined organic phases were washed with saturated saline (20 mL ×2) and dried over anhydrous sodium sulfate. The solvent was evaporated under reduced pressure and the product was isolated and purified by preparative HPLC to give (S)-N-[1,2,3-trimethoxy-9-oxo-10-(2-oxo-pyrrolidin-1-yl)5,6,7,9-tetrahydrobenzo[a]heptalen-7-yl]acetamide (52). $^1H$ NMR (DMSO-$d_6$, 400 MHz) δ 8.59 (d, J = 7.6 Hz, 1H), 7.56 (d, J = 10.0 Hz, 1H), 7.14 (s, 1H), 7.10 (d, J = 10.0 Hz, 1H), 6.79 (s, 1H), 4.35-4.29 (m, 1H), 3.85 (s, 3H), 3.79 (s, 3H), 3.67-3.61 (m, 2H), 3.57 (s, 3H), 2.67-2.61 (m, 1H), 2.42 (t, J = 8.0 Hz, 2H), 2.33-2.22 (m, 1H), 2.11-1.99 (m, 3H), 1.88-1.79 (m, 4H). MS (ESI, m/z): 453.4 $[M+H]^+$.

**Example 23: Synthesis of methyl (S)-(7-acetamido-1,2,3-trimethoxy-9-oxo-5,6,7,9-tetrahydrobenzo[a]heptalen-10-yl)methylcarbamate (54)**

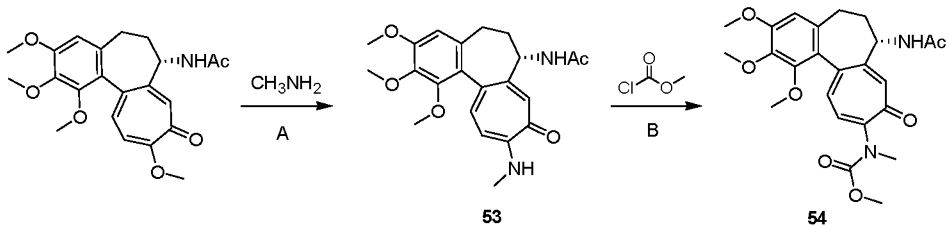


53 54

Step A: A mixture containing colchicine (5.0 g, 12.5 mmol), ethanol (50 mL) and 30% aqueous methylamine (25 mL) was stirred at 80°C for 1 hour. The solvent was evaporated under reduced pressure and then the mixture was pulped with petroleum ether to give (S)-N-(10-methylamino-1,2,3-trimethoxy-9-oxo-5,6,7,9-tetrahydrobenzo[a]heptalen-7-yl)acetamide **(53)** (4.90 g). The yield was 98.2%.

Step B: 60% sodium hydride (602 mg, 15.1 mmol) was added to a solution of compound **53** (1.0 g, 2.51 mmol) in THF (10 mL) in batches under an ice-water bath and then stirring was continued for 30 min at this temperature. Methyl chloroformate (2.57 g, 27.2 mmol) was then added, and after addition, the resulting mixture was stirred at room temperature for 1 hour. Water (50 mL) was added and the product was extracted with ethyl acetate (50 mL ×2). The combined organic phases were washed with saturated saline (30 mL) and dried over anhydrous sodium sulfate. The solvent was evaporated under reduced pressure and the product was purified by preparative HPLC to give methyl (S)-(7-acetamido-1,2,3-trimethoxy-9-oxo-5,6,7,9-tetrahydrobenzo[a]heptalen-10-yl)methylcarbatnate **(54).** $^1H$ NMR (DMSO-d6, 400 MHz) δ 8.62 (d, J = 7.6 Hz, 1H), 7.57 (d, J = 10.4 Hz, 1H), 7.17 (s, 1H), 7.05 (d, J = 10.4 Hz, 1H), 6.80 (s, 1H), 4.36-4.30 (m, 1H), 3.85 (s, 3H), 3.79 (s, 3H), 3.59 (s, 3H), 3.57 (s, 3H), 3.07 (s, 3H), 2.66-2.61 (m, 1H), 2.30-2.22 (m, 1H), 2.08-1.99 (m, 1H), 1.90-1.79 (m, 4H). MS (ESI, m/z): 457.4 $[M+H]^+$.

**Example 24: Synthesis of (S)-7-acetamido-1,2,3-trimethoxy-N-ethyl-9-oxo-5,6,7,9-tetrahydrobenzo[a]heptalen-10-carboxamide (55), (S)-7-acetamido-1,2,3-trimethoxy-N-(trideuterio)methyl-9-oxo-5,6,7,9-tetrahydrobenzo[a]heptalen-10-carbo xamide (56), (S)-7-acetamido-1,2,3-trimethoxy-9-oxo-5,6,7,9-tetrahydrobenzo[a]heptalen-10-carboxamide (57), (S)-7-acetamido-1,2,3-trimethoxy-N-phenyl-9-oxo-5,6,7,9-tetrahydrobenzo[a]heptalen-10-carboxamide (58), (S)-7-acetamido-1,2,3-trimethoxy-N,N-dimethyl-9-oxo-5,6,7,9-tetrahydrobenzo[a]heptalen-10-carboxamide (59), N-{(S)-10-[(S)-2-cyanopyrrolidin-1-carbonyl]-1,2,3-trimethoxy-9-oxo-5,6,7,9-tetrahydrobenzo[a]heptalen-7-yl} acetamide (60), (S)-N-{1,2,3-trimethoxy-9-oxo-10-(pyrrolidin-1-carbonyl)-5,6,7,9-tetrahydrobenzo[a]heptalen-7-yl}acetami de (61) and (S)-N-{1,2,3-trimethoxy-10-(4-methylpiperazine-1-carbonyl)-9-oxo-5,6,7,9-tetrahydrobenzo[a] heptalen-7-yl}acetamide (62)**

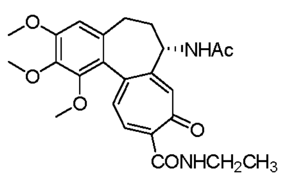
55

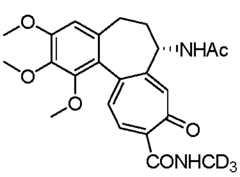
56

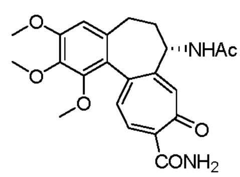
57

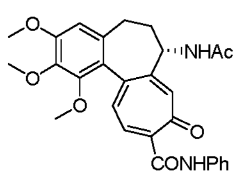
58

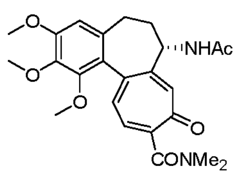
59

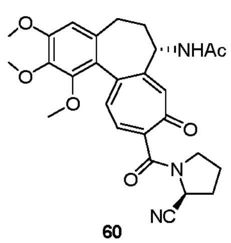
60

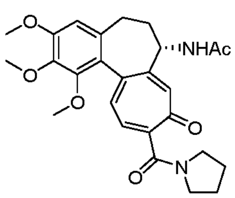
61

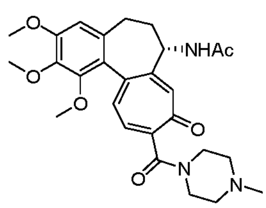
62

The experimental procedures for the synthesis of compound **55** (or 56, 57, 58, 59, 60, 61, 62) using compound **46** and ethylamine (or tri-deuteromethylamine, ammonium chloride, aniline, dimethylamine, (S)-pyrrolidine-2-carbonitrile, pyrrolidine, N-methylpiperazine) as raw materials were described in Step B of Example 20. Compound **55:** $^1$H NMR (DMSO-d6, 400 MHz) δ 9.35 (s, 1H), 8.65 (d, J = 6.0 Hz, 1H), 8.18 (d, J = 10.0 Hz, 1H), 7.29 (d, J = 10.0 Hz, 1H), 7.22 (s, 1H), 6.81 (s, 1H), 4.30-4.28 (m, 1H), 3.84 (s, 3H), 3.79 (s, 3H), 3.58 (s, 3H), 3.51-3.46 (m, 2H), 2.67-2.63 (m, 1H), 2.25-2.24 (m, 1H), 2.03-2.00 (m, 1H), 1.84-1.82 (m, 4H), 1.12-1.10 (m, 3H). MS (ESI, m/z): 441.4 [M+H]$^+$. compound **56:** $^1$H NMR (DMSO-d6, 400 MHz) δ 9.22 (s, 1H), 8.64 (d, J = 7.2 Hz, 1H), 8.19 (d, J = 10.0 Hz, 1H), 7.29 (d, J = 10.0 Hz, 1H), 7.22 (s, 1H), 6.81 (s, 1H), 4.33-4.26 (m, 1H), 3.85 (s, 3H), 3.79 (s, 3H), 3.58 (s, 3H), 2.67-2.62 (m, 1H), 2.26-2.24 (m, 1H), 2.03-1.98 (m, 1H), 1.84-1.82 (m, 4H). MS (ESI, m/z): 430.4 [M+H]$^+$. compound **57:** $^1$H NMR (DMSO-d6, 400 MHz) δ 8.67 (d, J = 2.4 Hz, 1H), 8.62 (d, J = 7.6 Hz, 1H), 8.18 (d, J = 10.0 Hz, 1H), 7.72 (d, J = 2.4 Hz, 1H), 7.27 (d, J = 10.0 Hz, 1H), 7.22 (s, 1H), 6.81 (s, 1H), 4.33-4.26 (m, 1H), 3.85 (s, 3H), 3.79 (s, 3H), 3.58 (s, 3H), 2.65-2.62 (m, 1H), 2.28-2.25 (m, 1H), 2.05-2.01 (m, 1H), 1.85-1.80 (m, 4H). MS (ESI, m/z): 413.0 [M+H]$^+$. compound **58:** $^1$H NMR (DMSO-d6, 400 MHz) δ 11.31 (s, 1H), 8.67 (d, J = 7.6 Hz, 1H), 8.12 (d, J = 10.0 Hz, 1H), 7.71-7.69 (m, 2H), 7.38-7.29 (m, 4H), 7.13-7.10 (m, 1H), 6.82 (s, 1H), 4.36-4.29 (m, 1H), 3.86 (s, 3H), 3.80 (s, 3H), 3.60 (s, 3H), 2.69-2.64 (m, 1H), 2.29-2.26 (m, 1H), 2.07-2.04 (m, 1H), 1.86-1.84 (m, 4H). MS (ESI, m/z): 489.2 [M+H]$^+$. compound **59:** $^1$H NMR (DMSO-d6, 400 MHz) δ 8.60 (d, J = 7.6 Hz, 1H), 7.32 (d, J = 9.2 Hz, 1H), 7.11-7.08 (m, 2H), 6.79 (s, 1H), 4.33-4.27 (m, 1H), 3.84 (s, 3H), 3.78 (s, 3H), 3.57 (s, 3H), 2.94 (s, 3H), 2.83 (s, 3H), 2.64-2.61 (m, 1H), 2.31-2.29 (m, 1H), 2.02-1.99 (m, 1H), 1.85-1.81 (m, 4H). MS (ESI, m/z): 441.1 [M+H]$^+$. compound **60:** $^1$H NMR (DMSO-d6, 400 MHz) δ 8.61 (d, J = 7.6 Hz, 1H), 7.32 (d, J = 9.2 Hz, 1H), 7.11-7.08 (m, 2H), 6.80 (s, 1H), 4.90-4.87 (m, 1H), 4.32-4.27 (m, 1H), 3.84 (s, 3H), 3.79 (s, 3H), 3.59 (s, 3H), 3.35-3.29 (m, 2H), 2.67-2.66 (m, 1H), 2.33-2.21 (m, 3H), 1.98-1.97 (m, 3H), 1.85-1.81 (m, 4H). MS (ESI, m/z): 492.4 [M+H]$^+$. compound **61:** $^1$H NMR (DMSO-d6, 400 MHz) δ 8.60 (d, J = 7.6 Hz, 1H), 7.36 (d, J = 9.2 Hz, 1H), 7.13-7.09 (m, 2H), 6.80 (s, 1H), 4.32-4.29 (m, 1H), 3.86 (s, 3H), 3.79 (s, 3H), 3.58 (s, 3H), 3.43-3.38 (m, 2H), 3.23-3.20 (m, 2H), 2.63-2.62 (m, 1H), 2.30-2.28 (m, 1H), 2.05-2.01 (m, 1H), 1.87-1.83 (m, 8H). MS (ESI, m/z): 467.4 [M+H]$^+$. compound **62:** $^1$H NMR (DMSO-d6, 400 MHz) δ 8.60 (d, J = 7.2 Hz, 1H), 7.31 (d, J = 9.6 Hz, 1H), 7.10-7.07 (m, 2H), 6.79 (s, 1H), 4.31-4.28 (m, 1H), 3.85 (s, 3H), 3.78 (s, 3H), 3.58 (s, 5H), 3.20 (s, 2H), 2.63-2.62 (m, 1H), 2.33-2.28 (m, 5H), 2.19 (s, 3H), 2.03-1.98 (m, 1H), 1.85-1.81 (m, 4H). MS (ESI, m/z): 496.4 [M+H]$^+$.

**Example 25: Synthesis of (S)-N-(1,2,3-trimethoxy-11-methyl-10-morpholino-9-oxo-5,6,7,9-tetrahydrobenzo[a]heptalen-7-yl)acetamide (63)**

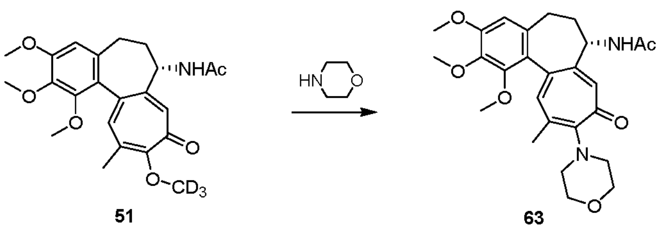

51 63

A solution of compound **51** (137 mg, 0.329 mmol) and morpholine (143 mg, 1.64 mmol) in acetonitrile (5 mL) was stirred at 85 °C for 48 hours. The solvent was evaporated under reduced pressure. The product was purified by column chromatography (200 ~ 300 mesh silica gel, eluted with dichloromethane:methanol = 100:1) to give (S)-N-(1,2,3-trimethoxy-11-methyl-10-morpholino-9-oxo-5,6,7,9-tetrahydrobenzo[a]heptalen-7-yl)acetamide (63). $^1$H NMR (DMSO-d6, 400 MHz) δ 8.50 (d, J = 6.0 Hz, 1H), 7.03 (s, 1H), 6.86 (s, 1H), 6.76 (s, 1H), 4.27-4.22 (m, 1H), 3.84 (s, 3H), 3.79 (s, 3H), 3.68-3.67 (m, 4H), 3.58 (s, 3H), 3.13-3.09 (m, 2H), 3.00-2.96 (m, 2H), 2.62-2.58 (m, 1H), 2.39 (s, 3H), 2.30-2.23 (m, 1H), 2.02-1.97 (m, 1H), 1.86-1.75 (m, 4H). MS (ESI, m/z): 469.2 [M+H]$^+$.

**Example 26: Synthesis of (S)-N-(9-hydroxymethyl-1,2,3-trimethoxy-10-oxo-5,6,7,10-tetrahydrobenzo[a]heptadien-7-yl)acetamide (66)**

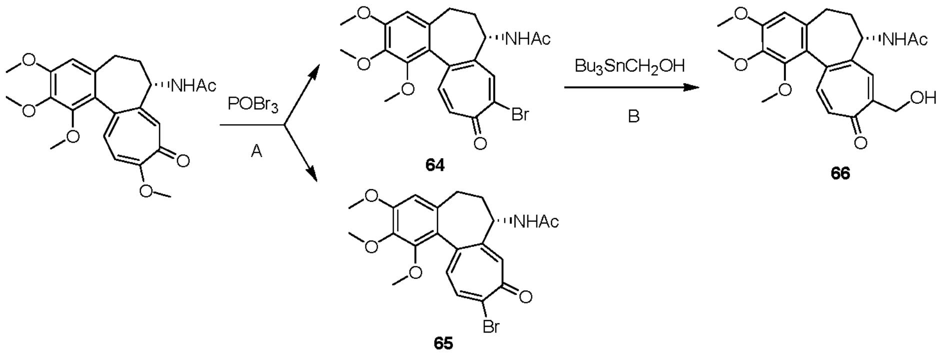


64 65 66

The experimental operations of Step A were described in Step A of Example 13, wherein phosphorus oxychloride in Step A of Example 13 was replaced with phosphorus oxybromide to give Compounds **64** and **65.**

Step B: A mixture containing compound **64** (1.0 g, 2.23 mmol), THF (10 mL) and tetrakis(triphenylphosphine)palladium (258 mg, 0.223 mmol) was stirred under nitrogen at room temperature for 30 minutes, and then tributyltin methanol (716.22 mg, 2.23 mmol) was added. After addition, the resulting mixture was stirred at 65 °C overnight. It was cooled to room temperature and filtered to remove insolubles. The solvent was evaporated under reduced pressure and the product was separated by preparative HPLC and SFC to give (S)-N-(9-hydroxymethyl-1,2,3-trimethoxy-10-oxo-5,6,7,10-tetrahydrobenzo[a]heptadien-7-yl)acetamide **(66).** $^1$H NMR (DMSO-d6, 400 MHz) δ 8.66 (d, J = 6.8 Hz, 1H), 7.84 (s, 1H), 7.21 (d, J = 12.8 Hz, 1H), 6.84-6.81 (m, 2H), 5.41-5.38 (m, 1H), 4.50-3.36 (m, 2H), 4.25-4.24 (m, 1H), 3.85 (s, 3H), 3.78 (s, 3H), 3.57 (s, 3H), 2.59-2.56 (m, 1H), 2.17-2.04 (m, 3H), 1.84 (s, 3H). MS (ESI, m/z): 400.0 [M+H]$^+$.

**Example 27: Synthesis of (S)-N-(10-formyl-1,2,3-trimethoxy-9-oxo-5,6,7,9-tetrahydrobenzo[a]heptadien-7-yl)acetamide (68)**

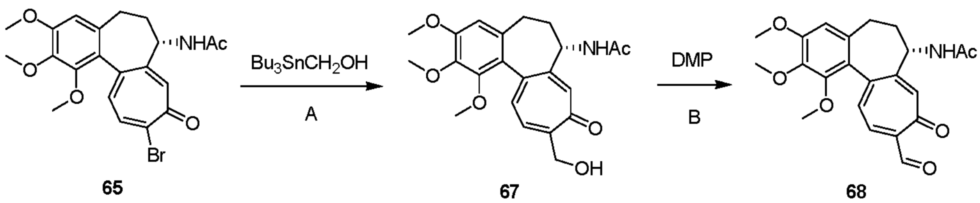


65 67 68

The experimental operations of Step A were described in Step B of Example 26, wherein Compound **64** in Step B of Example 26 was replaced with Compound **65** to afford (S)-N-(10-hydroxymethyl-1,2,3-trimethoxy-9-oxo-5,6,7,9-tetrahydrobenzo[a]heptadien-7-yl)acetamide **(67).** $^1$H NMR (DMSO-d6, 400 MHz) δ 8.58 (d, J = 7.2 Hz, 1H), 7.58 (d, J = 9.6 Hz, 1H), 7.19 (d, J = 9.6 Hz, 1H), 7.02 (s, 1H), 6.78 (s, 1H), 5.36 (t, J = 5.6 Hz, 1H), 4.39-4.33 (m, 2H), 4.31-4.28 (m, 1H), 3.84 (s, 3H), 3.79 (s, 3H), 3.54 (s, 3H), 2.62-2.56 (m, 1H), 2.27-2.24 (m, 1H), 2.02-1.99 (m, 1H), 1.84-1.79 (m, 4H). MS (ESI, m/z): 400.0 [M+H]$^+$.

Step B: A mixture containing Compound **67** (100 mg, 0.250 mmol), Dess-Martin oxidant (127 mg, 0.30 mmol) and dichloromethane (10 mL) was stirred at room temperature for 2 hours. The insolubles was removed by filtration. The solvent was evaporated under reduced pressure and the product was purified by preparative HPLC to afford (S)-N-(10-formyl-1,2,3-trimethoxy-9-oxo-5,6,7,9-tetrahydrobenzo[a]heptadien-7-yl)acetamide (68). $^1$H NMR (DMSO-d6, 400 MHz) δ 10.12 (s, 1H), 8.66 (d, J = 7.2 Hz, 1H), 7.82 (d, J = 9.2 Hz, 1H), 7.28-7.24 (m, 2H), 6.81 (s, 1H), 4.31-4.25 (m, 1H), 3.85 (s, 3H), 3.79 (s, 3H), 3.59 (s, 3H), 2.67-2.63 (m, 1H), 2.29-2.27 (m, 1H), 2.03-2.00 (m, 1H), 1.85-1.80 (m, 4H). MS (ESI, m/z): 398.3 [M+H]$^+$.

**Example 28: Synthesis of (S)-N-[1,2,3-trimethoxy-9-oxo-10-aminosulfonyl-5,6,7,9-tetrahydrobenzo[a]heptalen-7-yl]acetamide (69)**

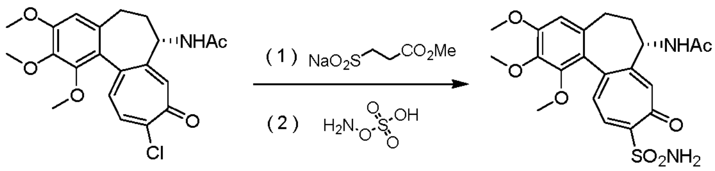


32 69

A solution of compound **32** (300 mg, 0.743 mmol) and sodium 3-methoxy-3-oxopropanesulfmate (155 mg, 0.891 mmol) in DMSO (6 mL) was stirred at room temperature for 2 h. Sodium methanol (40 mg, 0.740 mmol) was then added, and stirring was continued for 15 min at this temperature. Then a solution of hydroxylamine sulfonic acid (392 mg, 3.46 mmol) and sodium acetate (227 mg, 2.77 mmol) in water (1 mL) was added sequentially. After addition, the resulting mixture was stirred overnight at room temperature. Water (30 mL) was added and the product was extracted with ethyl acetate (50 mL ×2). The combined organic phases were washed with saturated saline (20 mL ×2) and dried over anhydrous sodium sulfate. The solvent was evaporated under reduced pressure and the product was purified by preparative HPLC to give (S)-N-[1,2,3-trimethoxy-9-oxo-10-aminosulfonyl-5,6,7,9-tetrahydrobenzo[a]heptalen-7-yl]acetamide (69). $^1H$ NMR (DMSO-d6, 400 MHz) δ 8.65 (d, J = 7.2 Hz, 1H), 8.19 (d, J = 10.0 Hz, 1H), 7.30 (d, J = 10.0 Hz, 1H), 7.20 (s, 1H), 7.09 (s, 2H), 6.82 (s, 1H), 4.30-4.27 (m, 1H), 3.85 (s, 3H), 3.79 (s, 3H), 3.59 (s, 3H), 2.67-2.52 (m, 1H), 2.33-2.27 (m, 1H), 2.06-1.99 (m, 1H), 1.85-1.83 (m, 4H). MS (ESI, m/z): 449.4 $[M+H]^+$.

**Example 29: Synthesis of (S)-N-[1,2,3-trimethoxy-10-morpholinyl-9-oxo-5,6,7,9-tetrahydrobenzo[a]heptalen-7-yl]propanamide (74)**

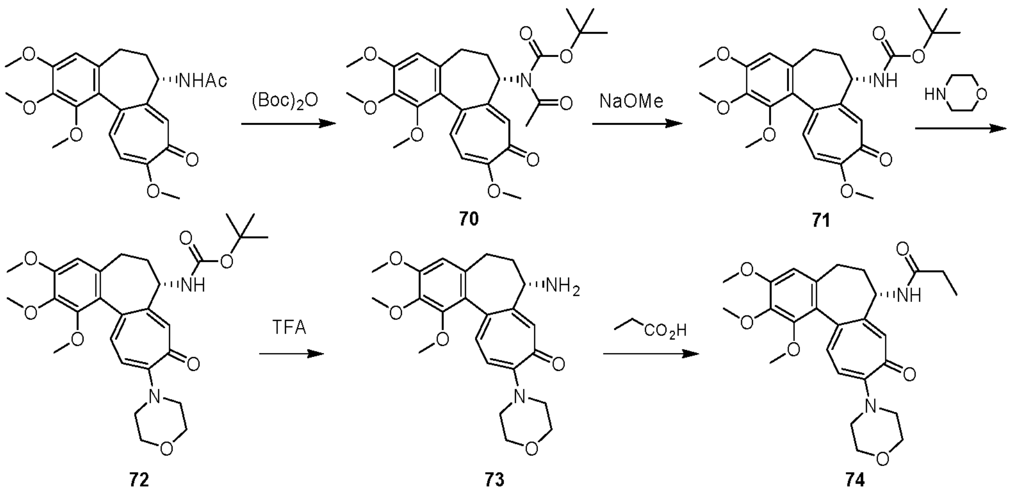


70 71

72 73 74

The experimental procedures for the synthesis of compound 74 using colchicine as a raw material were described in turn in Steps A and B of Example 18, Example 25, Step C of Example 18, and Step B of Example 20, wherein Compound **46** in Step B of Example 20 was substituted with propionic acid to give (S)-N-[1,2,3-trimethoxy-10-morpholinyl-9-oxo-5,6,7,9- tetrahydrobenzo[a]heptalen-7-yl]propanamide **(74).** $^1H$ NMR (DMSO-$d_6$, 400 MHz) δ 8.52 (d, J = 7.6 Hz, 1H), 7.13 (d, J = 10.8 Hz, 1H), 7.02 (s, 1H), 6.91 (d, J = 10.8 Hz, 1H), 6.81 (s, 1H), 4.41-4.37 (m, 1H), 3.89 (s, 3H), 3.84 (s, 3H), 3.80-3.77 (m, 4H), 3.58 (s, 3H), 3.47-3.41 (m, 2H), 3.36-3.33 (m, 2H), 2.59-2.57 (m, 1H), 2.21-2.18 (m, 3H), 2.16-2.05 (m, 1H), 1.95-1.90 (m, 1H), 1.01 (t, J = 7.2 Hz, 3H). MS (ESI, m/z): 469.4 $[M+H]^+$.

**Example 30: Synthesis of methyl (S)-7-(tert-butoxycarbonyl)amino-1,2,3-trimethoxy-9-oxo-5,6,7,9-tetrahydrobenzo[a]heptalen-10-carboxyla te (77), methyl (S)-1,2,3-trimethoxy-7-(3-methylureido)-9-oxo-5,6,7,9-tetrahydrobenzo[a]heptalen-10-carboxylate (79) and (S)-1,2,3-trimethoxy-N-methyl-7-(3-methylureido)-9-oxo-5,6,7,9-tetrahydrobenzo[a]heptalen-10-carboxami de (81)**

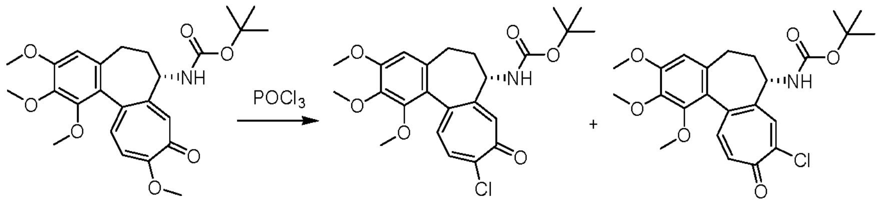


71 75 76

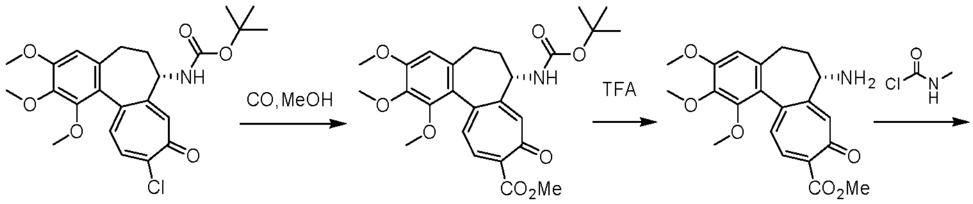


75 77 78

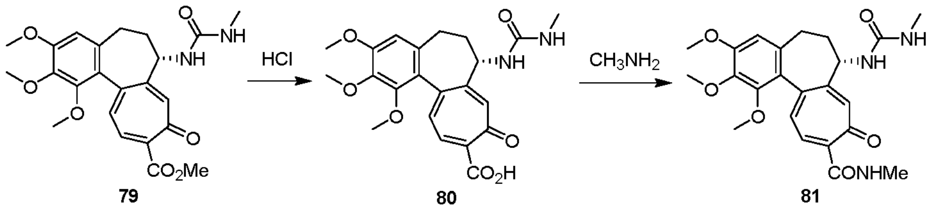


79 80 81

Experimental operations for the synthesis of compounds **77, 78, 79,** and 81 using compound **71** as a raw material were described in turn in steps A and B of Example 13, steps C and D of Example 18, and Example 20. compound **77:** $^1H$ NMR (DMSO-d6, 400 MHz) δ 7.75 (d, J = 7.6 Hz, 1H), 7.64 (d, J = 9.6 Hz, 1H), 7.17 (s, 1H), 7.11 (d, J = 9.6 Hz, 1H), 6.79 (s, 1H), 4.06-4.00 (m, 1H), 3.85 (s, 3H), 3.79 (s, 3H), 3.78 (s, 3H), 3.60 (s, 3H), 2.63-2.58 (m, 1H), 2.25-2.20 (m, 1H), 2.01-1.98 (m, 1H), 1.81-1.79 (m, 1H), 1.33 (s, 9H). MS (ESI, m/z): 486.3 $[M+H]^+$. compound **78:** MS (ESI, m/z): 386.0 $[M+H]^+$. compound **79:** $^1H$ NMR (DMSO-d6, 400 MHz) δ 7.62 (d, J = 9.6 Hz, 1H), 7.15 (s, 1H), 7.11 (d, J = 9.6 Hz, 1H), 6.80-6.78 (m, 2H), 5.79 (q, J = 4.4 Hz, 1H), 4.19-4.12 (m, 1H), 3.84 (s, 3H), 3.79 (s, 3H), 3.78 (s, 3H), 3.56 (s, 3H), 2.64-2.59 (m, 1H), 2.47 (d, J = 4.4 Hz, 3H), 2.26-2.24 (m, 1H), 2.03-2.00 (m, 1H), 1.74-1.71 (m, 1H). MS (ESI, m/z): 443.1 $[M+H]^+$. compound **81:** $^1H$ NMR (DMSO-d6, 400 MHz) δ 9.30 (q, J = 4.4 Hz, 1H), 8.20 (d, J = 10.0 Hz, 1H), 7.30-7.27 (m, 2H), 6.82-6.80 (m, 2H), 5.80 (q, J = 4.4 Hz, 1H), 4.23-4.16 (m, 1H), 3.85 (s, 3H), 3.79 (s, 3H), 3.57 (s, 3H), 2.81 (d, J = 4.4 Hz, 3H), 2.63-2.60 (m, 1H), 2.47 (d, J = 4.4 Hz, 3H), 2.23-2.21 (m, 1H), 2.05-2.04 (m, 1H), 1.74-1.73 (m, 1H). MS (ESI, m/z): 442.4 $[M+H]^+$.

**Example 31: Synthesis of methyl (S)-1,2,3-trimethoxy-7-(3-methylureido)-10-oxo-5,6,7,10-tetrahydrobenzo[a]heptalen-9-carboxylate (82)**

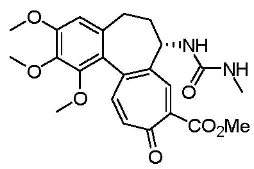

82

The experimental operations for the synthesis of compound **82,** using compound **76** as a raw material, wer e described in turn in step B of Example 13 and steps C and D of Example 18. $^1H$ NMR (DMSO-d6, 400 MHz) δ 7.71(s, 1H), 7.23 (d, J = 12.8 Hz, 1H), 6.94 (d, J = 12.8 Hz, 1H), 6.84 (s, 1H), 6.74 (d, J = 6.8 Hz, 1H), 5.80 (q, J = 4.4 Hz, 1H), 4.17-4.10 (m, 1H), 3.85 (s, 3H), 3.79 (s, 3H), 3.77 (s, 3H), 3.60 (s, 3H), 2.58-2.55 (m, 1H), 2.47 (q, J = 4.4 Hz, 3H), 2.21-2.16 (m, 2H), 2.14-1.99 (m, 1H). MS (ESI, m/z): 443.1 $[M+H]^+$.

**Example 32: Synthesis of (S)-N-{1,2,3-trimethoxy-10-(morpholine-4-carbonyl)-9-oxo-5,6,7,9-tetrahydrobenzo[a]heptalen-7-yl}acetam ide (83) and (S)-N-{1,2,3-trimethoxy-9-(morpholine-4-carbonyl)-10-oxo-5,6,7,10-tetrahydrobenzo[a]heptalen-7-yl}aceta** mide (84).

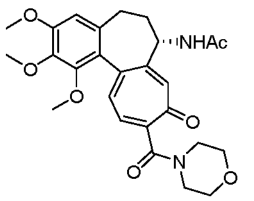

83

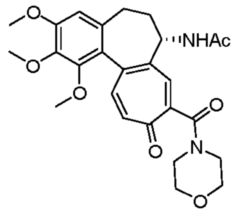

84

See Step B in Example 20 for experimental operations to synthesize compounds **83** or 84 using compound **46** (or 47) and morpholine as raw materials, compound **83:** $^1$H NMR (DMSO-d6, 400 MHz) δ 8.60 (d, J = 7.6 Hz, 1H), 7.35 (d, J = 9.6 Hz, 1H), 7.11-7.08 (m, 2H), 6.79 (s, 1H), 4.31-4.28 (m, 1H), 3.85 (s, 3H), 3.78 (s, 3H), 3.63-3.62 (m, 2H), 3.57-3.54 (m, 7H), 3.24-3.22 (m, 2H), 2.68-2.66 (m, 1H), 2.33-2.32 (m, 1H), 2.01-1.98 (m, 1H), 1.85-1.80 (m, 4H). MS (ESI, m/z): 483.4 [M+H]$^+$. Compound **84:** $^1$H NMR (DMSO-d6, 400 MHz) δ 8.54 (d, J = 7.2 Hz, 1H), 7.33 (s, 1H), 7.25 (d, J = 12.8 Hz, 1H), 6.94 (d, J = 12.8 Hz, 1H), 6.84 (s, 1H), 4.26-4.23 (m, 1H), 3.85 (s, 3H), 3.79 (s, 3H), 3.66-3.52 (m, 9H), 3.19 (s, 2H), 2.63-2.58 (m, 1H), 2.26-2.23 (m, 1H), 2.21-2.14 (m, 1H), 2.03-2.01 (m, 1H), 1.83 (s, 3H). MS (ESI, m/z): 483.2 [M+H]$^+$.

**Example 33: Synthesis of (S)-N-{1,2,3-trimethoxy-11-methyl-9-oxo-10-(2,2,2-trifluoroethoxy)-5,6,7,9-tetrahydrobenzo[a]heptalen-7-yl }acetamide (87) and (S)-N-{1,2,3-trimethoxy-11-methyl-10-oxo-9-(2,2,2-trifluoroethoxy)-5,6,7,10-tetrahydrobenzo[a]heptalen-7-yl}acetamide (88)**

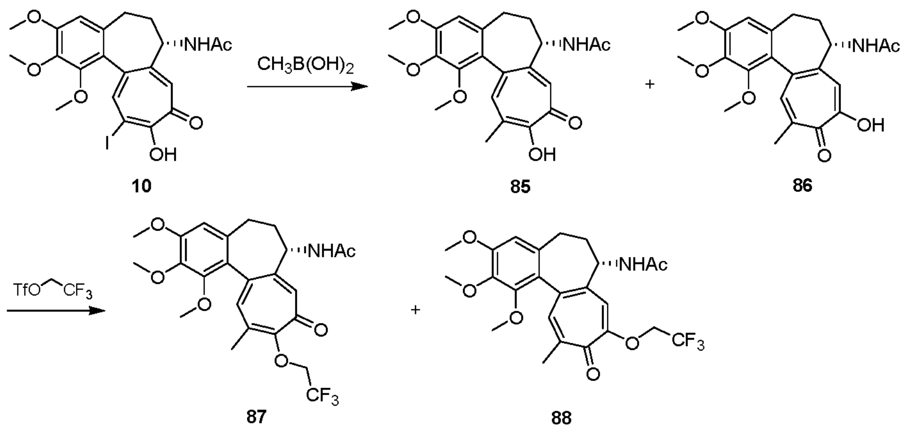


10 85 86 87 88

The experimental operations for the synthesis of compounds **87** and **88** using compound **10** as a raw material were described in turn in Example 7 and Example 14, wherein toluene in Example 7 was replaced with a solvent mixture of dioxane and water. Compound **87:** $^1$H NMR (DMSO-d6, 400 MHz) δ 8.58 (d, J = 7.2 Hz, 1H), 7.15 (s, 1H), 7.11 (s, 1H), 6.78 (s, 1H), 4.89-4.84 (m, 1H), 4.75-4.70 (m, 1H), 4.31-4.28 (m, 1H), 3.84 (s, 3H), 3.79 (s, 3H), 3.58 (s, 3H), 2.63-2.58 (m, 1H), 2.33 (s, 3H), 2.24-2.22 (m, 1H), 2.03-1.99 (m, 1H), 1.84-1.80 (m, 4H). MS (ESI, m/z): 482.4 [M+H]$^+$. compound **88:** $^1$H NMR (DMSO-d6, 400 MHz) δ 8.57 (d, J = 7.2 Hz, 1H), 7.52 (s, 1H), 7.27 (s, 1H), 6.81 (s, 1H), 4.83-4.76 (m, 2H), 4.34-4.31 (m, 1H), 3.85 (s, 3H), 3.80 (s, 3H), 3.59 (s, 3H), 2.57-2.55 (m, 1H), 2.25 (s, 3H), 2.16-1.98 (m, 3H), 1.86 (s, 3H). MS (ESI, m/z): 482.4 [M+H]$^+$.

**Example 34: Synthesis of methyl (S)-7-(ethylsulfonamido)-1,2,3-trimethoxy-9-oxo-5,6,7,9-tetrahydrobenzo[a]heptalen-10-carboxylate (89)**

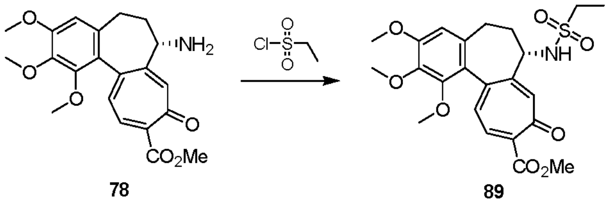

78 89

The experimental procedures for the synthesis of compound **89** using compound **78** and ethylsulfonyl chloride as raw material were described in step D of Example 18. $^1$H NMR (DMSO-d6, 400 MHz) δ 8.08 (d, J = 7.6 Hz, 1H), 7.66 (d, J = 9.2 Hz, 1H), 7.40 (s, 1H), 7.12 (d, J = 9.2 Hz, 1H), 6.80 (s, 1H), 3.95-3.91 (m, 1H), 3.85 (s, 3H), 3.79 (s, 6H), 3.52 (s, 3H), 2.89-2.80 (m, 2H), 2.65-2.60 (m, 1H), 2.28-2.20 (m, 1H), 2.12-2.07 (m, 1H), 1.86-1.79 (m, 1H), 1.09 (t, J = 7.2 Hz, 3H). MS (ESI, m/z): 478.4 [M+H]$^+$.

**Example 35: Synthesis of methyl (S)-7-(tert-butoxycarbonyl)amino-1,2,3-trimethoxy-10-oxo-5,6,7,10-tetrahydrobenzo[a]heptalen-9-carboxyl ate (90) and methyl (S)-7-(ethylsulfonamido)-1,2,3-trimethoxy-10-oxo-5,6,7,10-tetrahydrobenzo[a]heptalen-9-carboxylate (92).**

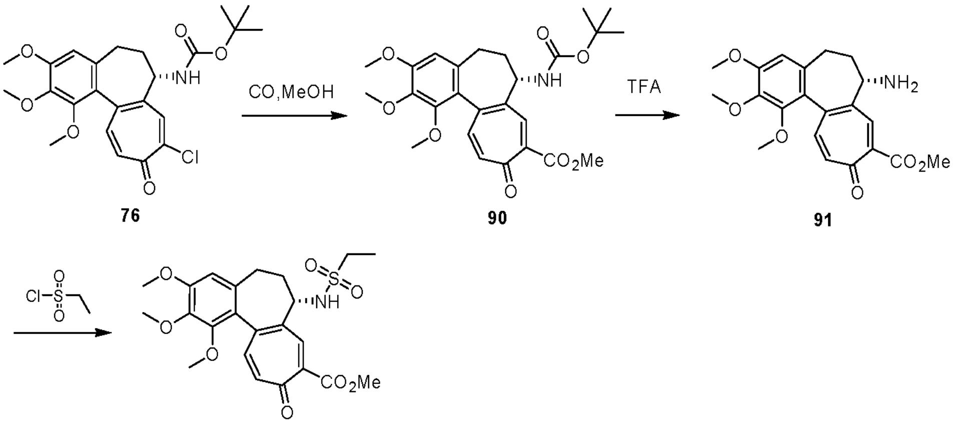


76 90 91

92

Experimental operations for the synthesis of compounds **90** and **92** using compound **76** as a raw material were described in turn in step B of Example 13 and steps C and D of Example 18. compound **90:** $^1$H NMR (DMSO-d6, 400 MHz) δ 7.71-7.69 (m, 2H), 7.22 (d, J = 12.8 Hz, 1H), 6.95 (d, J = 12.8 Hz, 1H), 6.84 (s, 1H), 4.01-3.94 (m, 1H), 3.86 (s, 3H), 3.79 (s, 3H), 3.78 (s, 3H), 3.65 (s, 3H), 2.57-2.55 (m, 1H), 2.21-2.03 (m, 3H), 1.33 (s, 9H). MS (ESI, m/z): 486.3 [M+H]$^+$. compound **92:** $^1$H NMR (DMSO-d6, 400 MHz) δ 8.10 (d, J = 7.6 Hz, 1H), 7.96 (s, 1H), 7.23 (d, J = 12.8 Hz, 1H), 6.97 (d, J = 12.8 Hz, 1H), 6.86 (s, 1H), 3.97-3.91 (m, 1H), 3.86 (s, 3H), 3.81 (s, 3H), 3.79 (s, 3H), 3.57 (s, 3H), 2.87-2.73 (m, 2H), 2.61-2.57 (m, 1H), 2.25-2.07 (m, 3H), 1.11 (t, J = 7.2 Hz, 3H). MS (ESI, m/z): 478.3 [M+H]$^+$.

**Example 36: Synthesis of methyl (S)-7-(3,3-dimethylureido)-1,2,3-trimethoxy-9-oxo-5,6,7,9-tetrahydrobenzo[a]heptalen-10-carboxylate (93) and methyl (S)-7-(3,3-dimethylureido)-1,2,3-trimethoxy-10-oxo-5,6,7,10-tetrahydrobenzo[a]heptahydrogen-9-carboxyla te (94)**

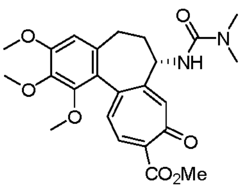

93

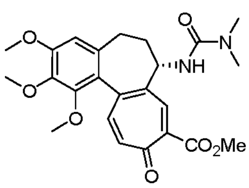

94

See Step D in Example 18 for experimental operations to synthesize compounds **93** or **94** using compound **78** (or 91) and dimethylcarbamoyl chloride as raw materials, compound **93:** $^1H$ NMR (DMSO-d6, 400 MHz) δ 7.63 (d, J = 9.6 Hz, 1H), 7.19 (s, 1H), 7.12 (d, J = 9.6 Hz, 1H), 6.86 (d, J = 7.2 Hz, 1H), 6.80 (s, 1H), 4.24-4.18 (m, 1H), 3.85 (s, 3H), 3.79 (s, 3H), 3.78 (s, 3H), 3.57 (s, 3H), 2.79 (s, 6H), 2.65-2.61 (m, 1H), 2.27-2.23 (m, 1H), 2.00-1.95 (m, 2H). MS (ESI, m/z): 457.4 $[M+H]^+$. compound 94: $^1H$ NMR (DMSO-d6, 400 MHz) δ 7.81 (s, 1H), 7.22 (d, J = 12.4 Hz, 1H), 6.93 (d, J = 12.4 Hz, 1H), 6.84-6.83 (m, 2H), 4.26-4.19 (m, 1H), 3.85 (s, 3H), 3.79 (s, 3H), 3.78 (s, 3H), 3.59 (s, 3H), 2.78 (s, 6H), 2.58-2.56 (m, 1H), 2.26-2.10 (m, 3H). MS (ESI, m/z): 457.4 $[M+H]^+$.

**Example 37: Synthesis of (S)-N-{1,2,3-trimethoxy-9-oxo-10-(1H-tetrazol-1-yl)-5,6,7,9-tetrahydrobenzo[a]heptalen-7-yl}acetamide (95)**

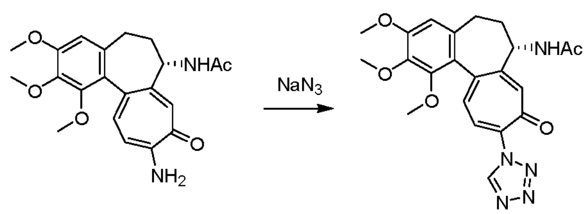


1 95

A mixture containing compound 1 (200 mg, 0.520 mmol), sodium azide (40.6 mg, 0.624 mmol), triethyl orthoformate (355 mg, 2.39 mmol) and acetic acid (5 mL) was stirred at 90°C for 2 h. The mixture was extracted with dichloromethane (30 mL ×2). After the mixture was cooled to room temperature, water (30 mL) was added and the pH was adjusted to 7 ~ 8 with saturated sodium bicarbonate solution. The product was extracted with dichloromethane (30 mL ×2), and the combined organic phases were washed with saturated brine (10 mL) and dried over anhydrous sodium sulfate. The solvent was evaporated under reduced pressure and the product was purified by preparative HPLC to give (S)-N-{1,2,3-trimethoxy-9-oxo-10-(1H-tetrazol-1-yl)-5,6,7,9-tetrahydrobenzo[a]heptalen-7-yl}acetamide **(95).** $^1H$ NMR (DMSO-d6, 400 MHz) δ 9.86 (s, 1H), 8.70 (d, J = 7.2 Hz, 1H), 7.25 (d, J = 10.0 Hz, 1H), 7.40 (s, 1H), 7.32 (d, J = 10.0 Hz, 1H), 6.84 (s, 1H), 4.39-4.35 (m, 1H), 3.86 (s, 3H), 3.81 (s, 3H), 3.61 (s, 3H), 2.70-2.66 (m, 1H), 2.35-2.32 (m, 1H), 2.30-2.05 (m, 1H), 1.92-1.86 (m, 4H). MS (ESI, m/z): 438.3 $[M+H]^+$.

**Example 38: Synthesis of (S)-N-{1,2,3-trimethoxy-9-oxo-10-(1H-1,2,4-triazol-1-yl)-5,6,7,9-tetrahydrobenzo[a]heptalen-7-yl}acetamide (96)**

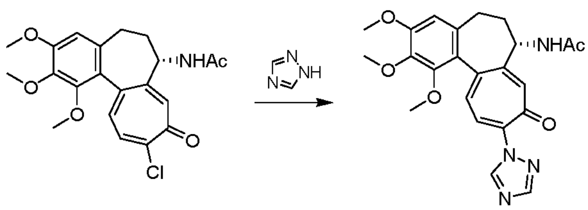

32 96

A mixture containing compound **32** (100 mg, 0.248 mmol), potassium carbonate (103 mg, 0.743 mmol), 1,2,4-triazole (34.2 mg, 0.495 mmol), cuprous iodide (12.7 mg, 0.0669 mmol) and DMSO (2 mL) was reacted for 30 min at 80°C in microwave. After cooling to room temperature, water (15 mL) was added and the mixture was extracted with dichloromethane (30 mL ×2). The combined organic phases were washed with saturated saline (10 mL) and dried over anhydrous sodium sulfate. The solvent was evaporated under reduced pressure and the product was purified by preparative HPLC to give (S)-N-{1,2,3-trimethoxy-9-oxo-10-(1H-1,2,4-triazol-1-yl)-5,6,7,9-tetrahydrobenzo[a]heptalen-7-yl}acetamide **(96).** $^1H$ NMR (DMSO-d6, 400 MHz) δ 9.33 (s, 1H), 8.68 (d, J = 7.2 Hz, 1H), 8.27 (s, 1H), 8.17 (d, J = 10.4 Hz, 1H), 7.40 (s, 1H), 7.34 (d, J = 10.4 Hz, 1H), 6.83 (s, 1H), 4.39-4.33 (m, 1H), 3.86 (s, 3H), 3.80 (s, 3H), 3.59 (s, 3H), 2.68-2.63 (m, 1H), 2.32-2.29 (m, 1H), 2.08-2.06 (m, 1H), 2.05-1.86 (m, 4H). MS (ESI, m/z): 437.2 $[M+H]^+$.

**Example 39: Synthesis of (S)-N-{10-(1H-imidazol-1-yl)-1,2,3-trimethoxy-9-oxo-5,6,7,9-tetrahydrobenzo[a]heptalen-7-yl}acetamide (97) and (S)-N-{1,2,3-trimethoxy-10-(3-methyl-1H-pyrazol-1-yl)-9-oxo-5,6,7,9-tetrahydrobenzo[a]heptalen-7-yl}aceta mide (98)**

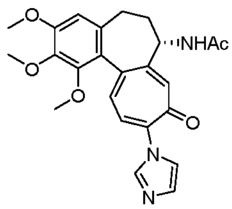
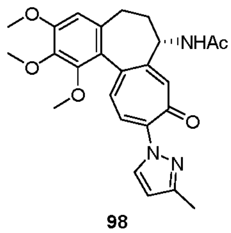

**97** **98**

See Example 38 for experimental operations to synthesize compounds **97** or **98** using compound **32** and imidazole (or 3-methyl-1H-pyrazole) as raw materials, compound **97:** $^1$H NMR (DMSO-d6, 400 MHz) δ 8.65 (d, J = 6.8 Hz, 1H), 8.12 (s, 1H), 7.72 (dd, J = 2.0, 10.0 Hz, 1H), 7.56 (s, 1H), 7.31 (s, 1H), 7.17 (dd, J = 2.0, 10.0 Hz, 1H), 7.05 (s, 1H), 6.82 (s, 1H), 4.38-4.32 (m, 1H), 3.85 (s, 3H), 3.80 (s, 3H), 3.59 (s, 3H), 2.68-2.63 (m, 1H), 2.32-2.29 (m, 1H), 2.07-2.04 (m, 1H), 1.87-1.85 (m, 4H). MS (ESI, m/z): 436.2 [M+H]$^+$. Compound **98:** $^1$H NMR (DMSO-d6, 400 MHz) δ 8.65-8.63 (m, 2H), 8.20 (d, J = 10.8 Hz, 1H), 7.34 (s, 1H), 7.30 (d, J = 10.8 Hz, 1H), 6.81 (s, 1H), 6.34 (d, J = 2.4 Hz, 1H), 4.38-4.32 (m, 1H), 3.85 (s, 3H), 3.80 (s, 3H), 3.58 (s, 3H), 2.66-2.61 (m, 1H), 2.29-2.25 (m, 4H), 2.07-2.04 (m, 1H), 1.90-1.86 (m, 4H). MS (ESI, m/z): 450.2 [M+H]$^+$.

**Example 40: Synthesis of (S)-N-(1,2,3-trimethoxy-9-oxo-10-(1H-1,2,3-triazol-1-yl)-5,6,7,9-tetrahydrobenzo[a]heptalen-7-yl)acetamide (99) and (S)-N-(1,2,3-trimethoxy-9-oxo-10-(2H-1,2,3-triazol-2-yl)-5,6,7,9-tetrahydrobenzo[a]heptalen-7-yl)acetamide (100)**

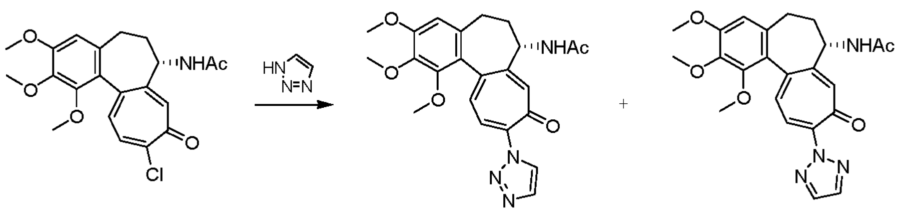

**32** **99** **100**

Experimental operations for the synthesis of compounds **99** and **100** were described in Example 38, wherein 1,2,4-triazole in Example 38 was replaced with 1,2,3-triazole. Compound **99:** $^1$H NMR (DMSO-d6, 400 MHz) δ 8.67 (d, J = 7.2 Hz, 1H), 8.64 (s, 1H), 7.08 (d, J = 10.0 Hz, 1H), 7.92 (s, 1H), 7.38 (s, 1H), 7.30 (d, J = 10.0 Hz, 1H), 6.83 (s, 1H), 4.40-4.33 (m, 1H), 3.86 (s, 3H), 3.81 (s, 3H), 3.61 (s, 3H), 2.70-2.65 (m, 1H), 2.35-2.32 (m, 1H), 2.08-2.05 (m, 1H), 1.89-1.86 (m, 4H). MS (ESI, m/z): 437.2 [M+H]$^+$. Compound **100:** $^1$H NMR (DMSO-d6, 400 MHz) δ 8.64 (d, J = 7.2 Hz, 1H), 8.08 (s, 2H), 7.83 (d, J = 10.0 Hz, 1H), 7.29 (s, 1H), 7.20 (d, J = 10.0 Hz, 1H), 6.82 (s, 1H), 4.36-4.30 (m, 1H), 3.86 (s, 3H), 3.80 (s, 3H), 3.61 (s, 3H), 2.69-2.64 (m, 1H), 2.38-2.33 (m, 1H), 2.07-2.03 (m, 1H), 1.90-1.86 (m, 4H). MS (ESI, m/z): 437.1 [M+H]$^+$.

**Example 41: Synthesis of methyl (S)-7-acetamido-1,2,3-trimethoxy-10-morpholino-9-oxo-5,6,7,9-tetrahydrobenzo[a]heptalen-11-carboxylate (101)**

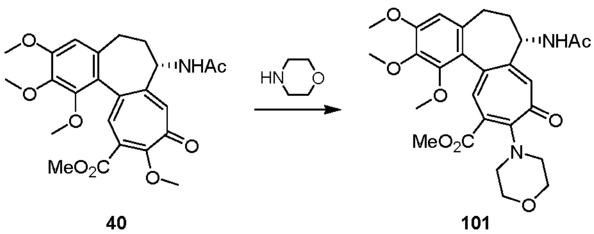

40 101

See Example 25 for experimental operations to synthesize compound **101** using compound **40** as a raw material. $^1$H NMR (DMSO-d6, 400 MHz) δ 8.48 (d, J = 8.4 Hz, 1H), 7.89 (s, 1H), 7.26 (s, 1H), 6.84 (s, 1H), 4.61-4.56 (m, 1H), 3.85 (s, 3H), 3.83 (s, 3H), 3.80 (s, 3H), 3.71-3.51 (m, 9H), 3.10 (s, 2H), 2.58-2.55 (m, 1H), 2.24-2.21 (m, 1H), 2.08-2.04 (m, 1H), 1.91-1.88 (m, 4H). MS (ESI, m/z): 513.4 [M+H]$^+$.

**Example 42: Synthesis of (S)-7-(3-cyclopropylcarbonylamino)-1,2,3-trimethoxy-N-methyl-9-oxo-5,6,7,9-tetrahydrobenzo[a]heptalen-1 0-carboxamide (102) and (S)-7-(3-cyclopropylcarbonylamino)-1,2,3-trimethoxy-N-methyl-10-oxo-5,6,7,10-tetrahydrobenzo[a]heptale n-9-carboxamide (103)**

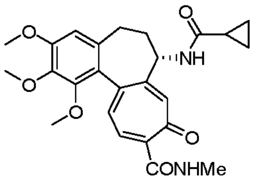

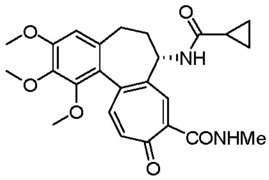

102 103

Compound **102** or **103** was obtained by synthesizing the corresponding amide using compound **78** (or **91)** and cyclopropanecarboxylic acid as raw materials, followed by a condensation reaction of the acid obtained by hydrolysis of the methyl ester with the methylamine, as described in the experimental procedures of Example 20. Compound **102:** $^1$H NMR (DMSO-d6, 400 MHz) δ 9.26 (q, J = 4.8 Hz, 1H), 8.85 (d, J = 7.6 Hz, 1H), 8.19 (d, J = 9.6 Hz, 1H), 7.30-7.26 (m, 2H), 6.81 (s, 1H), 4.36-4.29 (m, 1H), 3.85 (s, 3H), 3.79 (s, 3H), 3.55 (s, 3H), 2.81 (d, J = 4.8 Hz, 3H), 2.67-2.63 (m, 1H), 2.27-2.25 (m, 1H), 2.08-2.06 (m, 1H), 1.88-1.86 (m, 1H), 1.66-1.62 (m, 1H), 0.67-0.57 (m, 4H). MS (ESI, m/z): 453.3 [M+H]$^+$. compound **103:** $^1$H NMR (DMSO-d6, 400 MHz) δ 9.27 (q, J = 4.4 Hz, 1H), 8.92 (d, J = 6.8 Hz, 1H), 8.42 (s, 1H), 7.28 (d, J = 12.8 Hz, 1H), 7.07 (d, J = 12.8 Hz, 1H), 6.85 (s, 1H), 4.32-4.26 (m, 1H), 3.86 (s, 3H), 3.79 (s, 3H), 3.58 (s, 3H), 2.82 (d, J = 4.8 Hz, 3H), 2.62-2.60 (m, 1H), 2.20-2.09 (m, 3H), 1.66-1.63 (m, 1H), 0.65-0.57 (m, 4H). MS (ESI, m/z): 453.3 [M+H]$^+$.

**Example 43: Synthesis of (S)-N-(8-bromo-1,2,3,10-tetramethoxy-11-methyl-9-oxo-5,6,7,9-tetrahydrobenzo[a]heptalen-7-yl)acetamide (105) and (S)-N-(8-bromo-1,2,3,9-tetramethoxy-11-methyl-10-oxo-5,6,7,10-tetrahydrobenzo[a]heptalen-7-yl)acetamid e (106).**

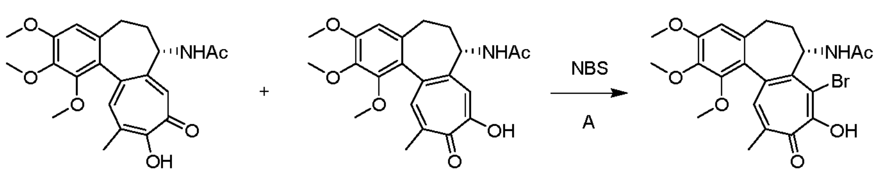


85 86 104

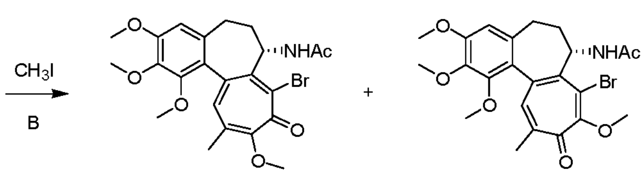


105 106

Step A: To a solution of a mixture of compounds **85** and **86** (1.0 g, 2.50 mmol) in DMF (10 mL) were added lithium bromide (220 mg, 2.53 mmol) and NBS (530 mg, 2.98 mmol), and after addition, the resulting mixture was stirred at room temperature overnight. Water (40 mL) was added and the product was extracted with ethyl acetate (30 mL ×3), the combined organic phases were washed with saturated saline (20 mL) and dried over anhydrous sodium sulfate. The solvent was evaporated under reduced pressure to afford (S)-N-(8-bromo-9-hydroxy-1,2,3 -trimethoxy-11 -methyl-10-oxo-5,6,7,10-tetrahydrobenzo [a]heptalen-7-yl)acetami de (104) as crude (3.60 g) (containing some DMF). The compound was used directly in the next step of the reaction without purification.

The experimental operations of Step B were described in Step C in Example 5, giving (S)-N-(1,2,3,10-tetramethoxy-11-methyl-9-oxo-5,6,7,9-tetrahydrobenzo[a]heptalen-7-yl)acetamide **(105)** and (S)-N-(1,2,3,9-tetramethoxy-11-methyl-9-oxo-5,6,7,10-tetrahydrobenzo[a]heptalen-7-yl)acetamide **(106).** MS (ESI, m/z) of compound **105:** 492.0 $[M+H]^+$. MS (ESI, m/z) of compound **106:** 492.0 $[M+H]^+$.

Example **44: Synthesis of methyl (S)-1,2,3-trimethoxy-9-oxo-7-(2-oxopiperidin-1-yl)-5,6,7,9-tetrahydrobenzo[a]heptalen-10-carboxylate (108)**

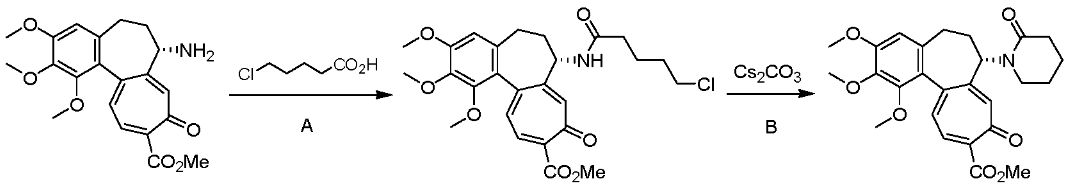


**78** **107** **108**

Step A: See Step B in Example 20 for experimental operations to synthesize Compound **107** using Compound **78** and 5-chlorovaleric acid as raw materials.

Step B: A mixture containing compound **107** (300 mg, 0.595 mmol), cesium carbonate (582 mg, 1.79 mmol), cuprous iodide (34.0 mg, 0.179 mmol) and DMSO (4 mL) was reacted at 80°C for 0.5 hours in microwave. After cooling to room temperature, water (20 mL) was added and the product was extracted with ethyl acetate (20 mL ×3), the combined organic phases were washed with saturated brine (20 mL) and dried over anhydrous sodium sulfate. The solvent was evaporated under reduced pressure and the product was isolated and purified by preparative HPLC and SFC to give methyl (S)-1,2,3-trimethoxy-9-oxo-7-(2-oxopiperidin-1-yl)-5,6,7,9-tetrahydrobenzo[a]heptalen-10-carboxylate **(108).** $^1$H NMR (DMSO-d6, 400 MHz) δ 7.62 (d, J = 9.2 Hz, 1H), 7.12 (d, J = 9.2 Hz, 1H), 6.81 (s, 1H), 6.77 (s, 1H), 4.67-4.63 (m, 1H), 3.84 (s, 3H), 3.77 (s, 6H), 3.67-3.64 (m, 1H), 3.59 (s, 3H), 3.45-3.41 (m, 1H), 2.76-2.71 (m, 1H), 2.35-2.19 (m, 4H), 2.05-1.99 (m, 1H), 1.91-1.87 (m, 1H), 1.78-1.74 (m, 2H), 1.63-1.59 (m, 1H). MS (ESI, m/z): 468.4 $[M+H]^+$.

**Example 45: Synthesis of methyl (S)-1,2,3-trimethoxy-10-oxo-7-(2-oxopiperidin-1-yl)-5,6,7,10-tetrahydrobenzo[a]heptalen-9-carboxylate (109)**

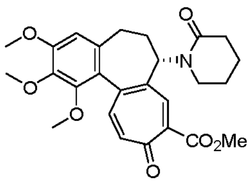

**109**

See Step B in Example 20 and Step B in Example 44 for experimental operations to synthesize Compound 109 using Compound 91 and 5-chlorovaleric acid as raw materials. $^1$H NMR (DMSO-d6, 400 MHz) δ 7.43 (s, 1H), 7.24 (d, J = 12.4 Hz, 1H), 6.92 (d, J = 12.4 Hz, 1H), 6.86 (s, 1H), 4.65-4.60 (m, 1H), 3.85 (s, 3H), 3.79 (s, 3H), 3.78 (s, 3H), 3.69-3.66 (m, 1H), 3.60 (s, 3H), 3.39-3.35 (m, 1H), 2.69-2.66 (m, 1H), 2.46-2.42 (m, 1H), 2.27-2.16 (m, 4H), 1.88-1.62 (m, 4H). MS (ESI, m/z): 468.4 $[M+H]^+$.

**Example 46: Synthesis of methyl (S)-1,2,3-trimethoxy-9-oxo-7-(3-oxomorpholinyl)-5,6,7,9-tetrahydrobenzo[a]heptalen-10-carboxylate (110)**

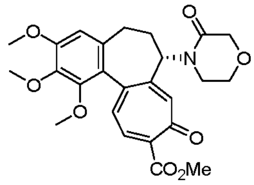

110

See Step B in Example 20 and Step B in Example 43 for experimental operations to synthesize Compound 110 using Compound **78** and 2-(2-chloroethoxy)acetic acid as raw materials. $^1$H NMR (DMSO-d6, 400 MHz) δ 7.64 (d, J = 9.2 Hz, 1H), 7.14 (d, J = 9.2 Hz, 1H), 6.83 (s, 1H), 6.82 (s, 1H), 4.62-4.60 (m, 1H), 4.11-3.93 (m, 4H), 3.85 (s, 3H), 3.78-3.71 (m, 7H), 3.59-3.56 (m, 4H), 2.76-2.73 (m, 1H), 2.33-2.31 (m, 1H), 2.23-2.21 (m, 1H), 2.10-2.08 (m, 1H). MS (ESI, m/z): 470.1 [M+H]$^+$.

**Example 47: Synthesis of methyl (S)-1,2,3-trimethoxy-7-(N-methylacetamido)-9-oxo-5,6,7,9-tetrahydrobenzo[a]heptalen-10-carboxylate (111)**

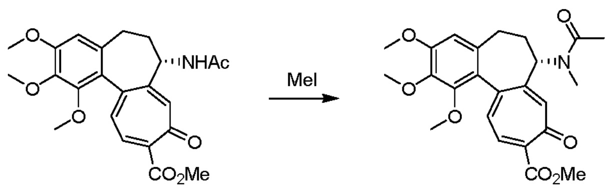


34 111

60% sodium hydride (93.6 mg, 2.34 mmol) was added to a solution of compound **34** (500 mg, 1.17 mmol) in DMF (7 mL) in an ice-water bath, and stirring was continued at this temperature for 0.5 h before iodomethane (664 mg, 4.68 mmol) was added. After addition, the resulting mixture was stirred at room temperature for 1.5 hours. Saturated ammonium chloride solution (10 mL) was added, extraction was carried out with ethyl acetate (10 mL ×3), and the combined organic phases were washed with saturated saline (15 mL ×3) and dried over anhydrous sodium sulfate. The solvent was evaporated under reduced pressure and the product was purified by preparative HPLC to give methyl (S)-1,2,3-trimethoxy-7-(N-methylacetamido)-9-oxo-5,6,7,9-tetrahydrobenzo[a]heptalen-10-carboxylate (111). $^1$H NMR (DMSO-d6, 400 MHz) δ 7.62 (d, J = 9.6 Hz, 1H), 7.11 (d, J = 9.6 Hz, 1H), 6.81 (s, 1H), 6.79 (s, 1H), 4.64-4.60 (m, 1H), 3.84 (s, 3H), 3.77 (s, 6H), 3.58 (s, 3H), 3.17 (s, 3H), 2.75-2.70 (m, 1H), 2.34-2.26 (m, 2H), 2.02-1.95 (m, 4H). MS (ESI, m/z): 442.4 [M+H]$^+$.

**Example 48: Synthesis of methyl (S)-7-[(N-ethylsulfonyl)amino]-1,2,3-trimethoxy-9-oxo-5,6,7,9-tetrahydrobenzo[a]heptalen-10-carboxylate (112) and methyl (S)-7-[(N-ethylsulfonyl)amino]-1,2,3-trimethoxy-10-oxo-5,6,7,10-tetrahydrobenzo[a]heptalen-9-carboxylate (113)**

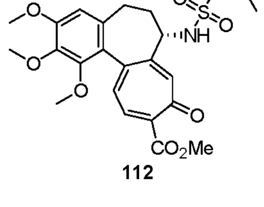

112

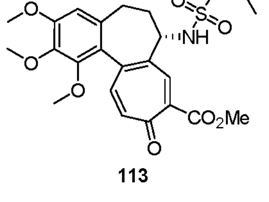

113

See Step D in Example 18 for experimental operations to synthesize compounds **112** or **113** using Compound

78 (or 91) and N-ethylaminosulfonyl chloride as raw materials. Compound **112:** $^1H$ NMR (DMSO-d6, 400 MHz) δ 7.77 (d, J = 8.4 Hz, 1H), 7.64 (d, J = 9.6 Hz, 1H), 7.41 (s, 1H), 7.11 (d, J = 9.6 Hz, 1H), 6.80 (s, 1H), 6.77 (t, J = 6.0 Hz, 1H), 3.87-3.79 (m, 10H), 3.53 (s, 3H), 2.73-2.59 (m, 3H), 2.24-2.22 (m, 1H), 2.09-1.99 (m, 1H), 1.80-1.78 (m, 1H), 0.86 (t, J = 7.2 Hz, 3H). MS (ESI, m/z): 493.4 $[M+H]^+$. Compound 113: $^1H$ NMR (DMSO-d6, 400 MHz) δ 7.96 (d, J = 8.4 Hz, 1H), 7.77 (d, J = 7.2 Hz, 1H), 7.22 (d, J = 12.8 Hz, 1H), 6.95 (d, J = 12.8 Hz, 1H), 6.86 (s, 1H), 6.78 (t, J = 6.0 Hz, 1H), 3.86-3.79 (m, 10H), 3.58 (s, 3H), 2.73-2.58 (m, 3H), 2.19-2.16 (m, 3H), 0.83 (t, J = 7.2 Hz, 3H). MS (ESI, m/z): 493.4 $[M+H]^+$.

**Example 49: Synthesis of methyl (S)-7-acrylamido-1,2,3-trimethoxy-9-oxo-5,6,7,9-tetrahydrobenzo[a]heptalen-10-carboxylate (114) and methyl (S)-7-acrylamido-1,2,3-trimethoxy-10-oxo-5,6,7,10-tetrahydrobenzo[a]heptalen-9-carboxylate (115)**

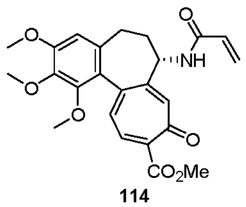

114

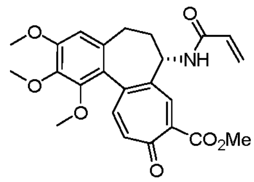

115

See Step D in Example 18 for experimental operations to synthesize compounds **114** or **115** using compound 78 (or 91) and acryloyl chloride as raw materials. Compound **114:** $^1H$ NMR (DMSO-d6, 400 MHz) δ 8.84 (d, J = 7.2 Hz, 1H), 7.65 (d, J = 9.6 Hz, 1H), 7.15 (d, J = 9.6 Hz, 1H), 7.05 (s, 1H), 6.81 (s, 1H), 6.34-6.27 (m, 1H), 6.08-6.03 (m, 1H), 5.67-5.62 (m, 1H), 4.38-4.32 (m, 1H), 3.85 (s, 3H), 3.79 (s, 3H), 3.77 (s, 3H), 3.59 (s, 3H), 2.69-2.64 (m, 1H), 2.34-2.30 (m, 1H), 2.08-2.05 (m, 1H), 1.89-1.86 (m, 1H). MS (ESI, m/z): 440.3 $[M+H]^+$. Compound **115:** $^1H$ NMR (DMSO-d6, 400 MHz) δ 8.82 (d, J = 6.8 Hz, 1H), 7.62 (s, 1H), 7.25 (d, J = 12.8 Hz, 1H), 6.96 (d, J = 12.8 Hz, 1H), 6.86 (s, 1H), 6.32-6.25 (m, 1H), 6.07-6.03 (m, 1H), 5.65-5.62 (m, 1H), 4.36-4.29 (m, 1H), 3.86 (s, 3H), 3.80 (s, 3H), 3.77 (s, 3H), 3.63 (s, 3H), 2.63-2.60 (m, 1H), 2.28-2.13 (m, 3H). MS (ESI, m/z): 440.2 $[M+H]^+$.

**Example 50: Synthesis of methyl (S)-7-acetamido-4-chloro-1,2,3-trimethoxy-9-oxo-5,6,7,9-tetrahydrobenzo[a]heptalen-10-carboxylate (116)**

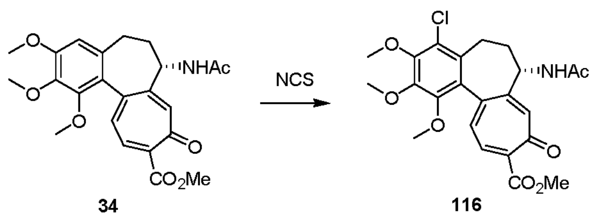


34 116

The experimental operations for the synthesis of compound **116** were described in step A of Example 2, wherein the colchicine in step A of Example 2 was replaced with compound **34.** $^1H$ NMR (DMSO-d6, 400 MHz) δ 8.65 (d, J = 7.2 Hz, 1H), 7.66 (d, J = 9.2 Hz, 1H), 7.13 (d, J = 9.2 Hz, 1H), 7.07 (s, 1H), 4.18-4.15 (m, 1H), 3.91 (s, 3H), 3.88 (s, 3H), 3.79 (s, 3H), 3.58 (s, 3H), 3.16-3.11 (m, 1H), 2.19-2.17 (m, 1H), 1.96-1.93 (m, 1H), 1.85-1.80 (m, 4H). MS (ESI, m/z): 462.3 $[M+H]^+$.

**Example 51: Synthesis of (S)-N-{1,2,3-trimethoxy-9-oxo-10-(2-oxooxazolidin-3-yl]-5,6,7,9-tetrahydrobenzo[a]heptalen-7-yl}acetamide (118)**

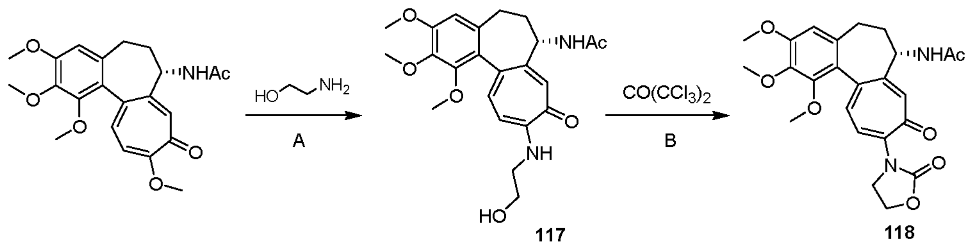


117 118

Step A: A mixture containing colchicine (3.0 g, 7.51 mmol), ethanolamine (56.9 g, 932 mmol) and methanol (30 mL) was stirred at 30°C for 5 hours. Water (20 mL) was added and the mixture was extracted with ethyl acetate (50 mL ×3). The combined organic phases were washed with saturated saline (50 mL) and dried over anhydrous sodium sulphate. The solvent was evaporated under reduced pressure and the product was purified by column chromatography (200 ~ 300 mesh silica gel, eluting with ethyl acetate:methanol = 9:1) to afford (S)-N-{10-[(2-hydroxyethyl)amino]-1,2,3-trimethoxy-9-oxo-5,6,7,9-tetrahydrobenzo[a]heptalen-7-yl}acetamide (117) (2.70 g). The yield was 83.9 %. $^1$H NMR (400 MHz, DMSO) δ 8.64 (d, J = 7.6 Hz, 1H), 7.61-7.58 (m, 1H), 7.20 (d, J = 11.2 Hz, 1H), 7.11(s, 1H), 6.75-6.69 (m, 2H), 5.00-4.97 (m, 1H), 4.41-4.34 (m, 1H), 3.83 (s, 3H), 3.78 (s, 3H), 3.69-3.65 (m, 2H), 3.48 (s, 3H), 3.43-3.41 (m, 2H), 2.57-2.55 (m, 1H), 2.22-2.14 (m, 1H), 2.07-1.98 (m, 1H), 1.89-1.82 (m, 4H). MS (ESI, m/z): 429.1 $[M+H]^+$.

Step B: Compound **117** (1.0 g, 2.33 mmol) and triethylamine (708 mg, 7.0 mmol) were added to a solution of triphosgene (370 mg, 1.25 mmol) in dichloromethane (60 mL) under an ice water bath. After addition, the resulting mixture was stirred at room temperature for 30 minutes. Water (100 mL) was added and the mixture was extracted with dichloromethane (50 mL ×2). The combined organic phases were washed with saturated saline (30 mL) and dried over anhydrous sodium sulfate. The solvent was evaporated under reduced pressure and the product was purified by preparative HPLC to give (S)-N-{1,2,3-trimethoxy-9-oxo-10-(2-oxooxazolidin-3-yl]-5,6,7,9-tetrahydrobenzo[a]heptalen-7-yl}acetamide (118). $^1$H NMR (400 MHz, DMSO) δ 8.60 (d, J = 7.6 Hz, 1H), 7.69 (d, J = 10.4 Hz, 1H), 7.17 (s, 1H), 7.13(d, J = 10.4 Hz, 1H), 6.80 (s, 1H), 4.47-4.43 (m, 2H), 4.36-4.29 (m, 1H), 4.10-4.04 (m, 1H), 3.94-3.90 (m, 1H), 3.85 (s, 3H), 3.80 (s, 3H), 3.57 (s, 3H), 2.67-2.61 (m, 1H), 2.31-2.22 (m, 1H), 2.08-2.00 (m, 1H), 1.86-1.80 (m, 4H). MS (ESI, m/z): 455.4 $[M+H]^+$.

**Example 52: Synthesis of (S)-N-{1,2,3-trimethoxy-10-(1-methyl-1H-pyrazol-4-yl)-9-oxo-5,6,7,9-tetrahydrobenzo[a]heptalen-7-yl}aceta mide (119)**

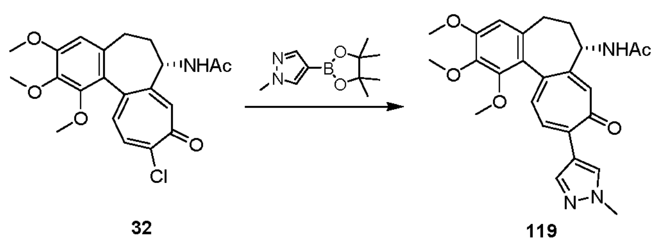

32 119

To a mixture containing Compound **32** (300 mg, 0.743 mmol), 1-methyl-4-(4,4,5,5-tetramethyl-1,3,2-dioxaborolan-2-yl)-1H-pyrazole (232 mg, 1.11 mmol), potassium carbonate (308 mg, 2.23 mmol), water (3 mL) and dioxane (6 mL), were added 1,1'-Bis(diphenylphosphino)ferrocene]dichloropalladium(II) (54 mg, 0.0743 mmol), and after addition, the resulting mixture was stirred under nitrogen at 80°C for 4 hours. Water (20 mL) was added and the mixture was extracted with ethyl acetate (30 mL ×3). The combined organic phases were washed with saturated saline (20 mL) and dried over anhydrous sodium sulfate. The solvent was evaporated under reduced pressure and the product was purified by preparative HPLC to give (S)-N-{1,2,3-trimethoxy-10-(1-methyl-1H-pyrazol-4-yl)-9-oxo-5,6,7,9-tetrahydrobenzo[a]heptalen-7-yl}acetamid e (119). $^1$H NMR (DMSO-d6, 400 MHz) δ 8.60-8.57 (m, 2H), 8.11 (s, 1H), 7.92 (d, J = 10.4 Hz, 1H), 7.16-7.13 (m, 2H), 6.79 (s, 1H), 4.36-4.29 (m, 1H), 3.90 (s, 3H), 3.84 (s, 3H), 3.79 (s, 3H), 3.56 (s, 3H), 2.64-2.59 (m, 1H), 2.30-2.25 (m, 1H), 2.05-2.01 (m, 1H), 1.88-1.84 (m, 4H). MS (ESI, m/z): 450.2 $[M+H]^+$.

**Example 53: Synthesis of (S)-7-acetamido-1,2,3-trimethoxy-9-oxo-N-(pyridin-3-yl)-5,6,7,9-tetrahydrobenzo[a]heptalen-10-carboxami de (120)**

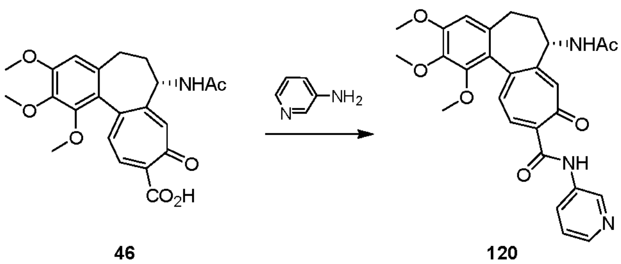

46 120

The experimental procedures for the synthesis of compound **120** using compound **46** and 3-aminopyridine as raw materials were described in step B of Example 20. $^1$H NMR (DMSO-d6, 400 MHz) δ 11.36 (s, 1H), 8.84 (d, J = 2.0 Hz, 1H), 8.68 (d, J = 7.2 Hz, 1H), 8.33 (dd, J = 1.2, 4.4 Hz, 1H), 8.19-8.16 (m, 1H), 8.08 (d, J = 10.0 Hz, 1H), 7.43-7.40 (m, 1H), 7.33-7.30 (m, 2H), 6.83 (s, 1H), 4.36-4.30 (m, 1H), 3.86 (s, 3H), 3.81 (s, 3H), 3.61 (s, 3H), 2.70-2.65 (m, 1H), 2.29-2.24 (m, 1H), 2.09-2.03 (m, 1H), 1.90-1.82 (m, 4H). MS (ESI, m/z): 490.3 [M+H]$^+$.

**Example 54: Synthesis of (S)-7-acetamido-2-hydroxy-1,3-dimethoxy-N-methyl-9-oxo-5,6,7,9-tetrahydrobenzo[a]heptalen-10-carboxa mide (121) and (S)-7-acetamido-1,3-dimethoxy-2-trideutero-methoxy-N-methyl-9-oxo-5,6,7,9-tetrahydrobenzo[a]heptalen-1 0-carboxamide (122)**

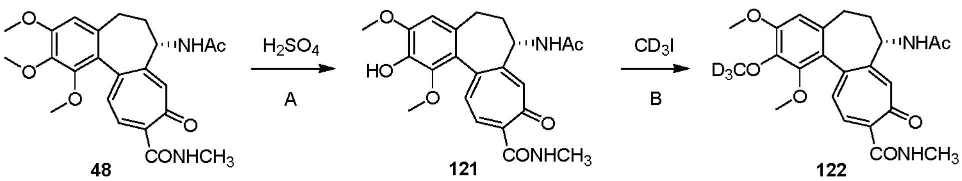


48 121 122

Step A: A mixture containing Compound 48 (200 mg, 0.469 mmol) and sulfuric acid (2 mL) was stirred at 45°C for 7 hours. Isopropanol (5 mL) was added and the mixture was poured into ice water (10 mL). Extraction was performed with dichloromethane (20 mL ×3). The combined organic phases were washed with saturated sodium bicarbonate solution (20 mL) and dried over anhydrous sodium sulfate. The solvent was evaporated under reduced pressure and the product was purified by preparative HPLC to give (S)-7-acetamido-2-hydroxy-1,3-dimethoxy-N-methyl-9-oxo-5,6,7,9-tetrahydrobenzo [a]heptalen-10-carboxamide (121) (50.8 mg). The yield was 26.2 %. $^1$H NMR (DMSO-d6, 400 MHz) δ 9.29 (q, J = 4.4 Hz, 1H), 8.65 (d, J = 7.6 Hz, 1H), 8.21 (d, J = 10.0 Hz, 1H), 7.30 (d, J = 10.0 Hz, 1H), 7.23 (s, 1H), 6.70 (s, 1H), 4.35-4.28 (m, 1H), 3.82 (s, 3H), 3.50 (s, 3H), 2.81 (d, J = 4.8 Hz, 3H), 2.59-2.55 (m, 1H), 2.20-2.19 (m, 1H), 2.02-1.99 (m, 1H), 1.84-1.79 (m, 4H). MS (ESI, m/z): 413.3 [M+H]$^+$.

Step B: A mixture containing compound **121** (100 mg, 0.242 mmol), Iodomethane-d3 (1.40 g, 9.65 mmol), potassium carbonate (101 mg, 0.731 mmol) and acetone (3 mL) was stirred at 60°C for 1 hour. Water (10 mL) was added, and the mixture was extracted with dichloromethane (10 mL ×3) and dried over anhydrous sodium sulfate. The solvent was evaporated under reduced pressure and the product was purified by preparative HPLC to give (S)-7-acetamido-1,3-dimethoxy-2-trideutero-methoxy-N-methyl-9-oxo-5,6,7,9-tetrahydrobenzo [a]heptalen-10-car boxamide (122). $^1$H NMR (DMSO-d6, 400 MHz) δ 9.25 (q, J = 4.4 Hz, 1H), 8.63 (d, J = 7.2 Hz, 1H), 8.19 (d, J = 10.0 Hz, 1H), 7.29 (d, J = 10.0 Hz, 1H), 7.23 (s, 1H), 6.81 (s, 1H), 4.33-4.27 (m, 1H), 3.85 (s, 3H), 3.58 (s, 3H), 2.81 (d, J = 4.8 Hz, 3H), 2.66-2.62 (m, 1H), 2.26-2.24 (m, 1H), 2.05-2.02 (m, 1H), 1.86-1.82 (m, 4H). MS (ESI, m/z): 430.4 [M+H]$^+$.

**Example 55: Synthesis of (S)-7-acetamido-2,3-dihydroxy-1-methoxy-N-methyl-9-oxo-5,6,7,9-tetrahydrobenzo[a]heptalene-10-carboxa mide (123) and (S)-7-acetamido-1-methoxy-2,3-bis(trideuteromethoxy)-N-methyl-9-oxo-5,6,7,9-tetrahydrobenzo[a] heptalen-10-carboxamide (124)**

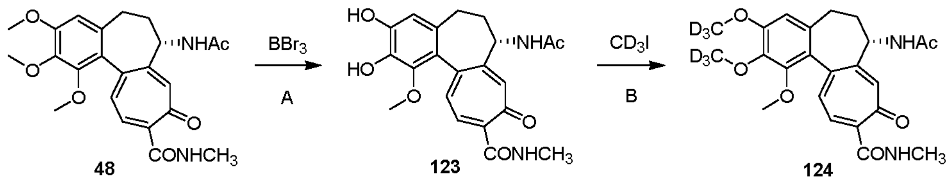

48 123 124

Step A: 1 M boron tribromide dichloromethane solution (16.8 mL) was added dropwise to a solution of compound **48** (1.20 g, 2.81 mmol) in dichloromethane (138 mL) in an ice water bath. After addition, the resulting mixture was stirred at 10°C for 4 hours. The reaction was quenched by batchwise addition of methanol (10 mL). The solvent was evaporated under reduced pressure and the product was purified by preparative HPLC to afford (S)-7-acetamido-2,3-dihydroxy-1-methoxy-N-methyl-9-oxo-5,6,7,9-tetrahydrobenzo[a]heptahydrotropene-10-car boxamide **(123)** (300 mg). The yield was 26.8 %. $^1$H NMR (DMSO-d6, 400 MHz) δ 9.32 (q, J = 4.4 Hz, 1H), 8.62 (d, J = 7.2 Hz, 1H), 8.22 (d, J = 10.0 Hz, 1H), 7.30 (d, J = 10.0 Hz, 1H), 7.23 (s, 1H), 6.47 (s, 1H), 4.36-4.30 (m, 1H), 3.49 (s, 3H), 2.81 (d, J = 4.8 Hz, 3H), 2.46-2.43 (m, 1H), 2.16-2.12 (m, 1H), 2.02-1.99 (m, 1H), 1.85-1.77 (m, 4H). MS (ESI, m/z): 399.0 [M+H]$^+$.

The experimental procedures of Step B were described in Step B of Example 54 to afford (S)-7-acetamido-1-methoxy-2,3-bis(trideuteromethoxy)-N-methyl-9-oxo-5,6,7,9-tetrahydrobenzo [a]heptalen-10-c arboxamide (124). $^1$H NMR (DMSO-d6, 400 MHz) δ 9.25 (q, J = 4.4 Hz, 1H), 8.63 (d, J = 7.6 Hz, 1H), 8.19 (d, J = 10.0 Hz, 1H), 7.29 (d, J = 10.0 Hz, 1H), 7.23 (s, 1H), 6.80 (s, 1H), 4.33-4.27 (m, 1H), 3.58 (s, 3H), 2.81 (d, J = 4.8 Hz, 3H), 2.66-2.62 (m, 1H), 2.26-2.24 (m, 1H), 2.05-2.02 (m, 1H), 1.87-1.82 (m, 4H). MS (ESI, m/z): 433.2 [M+H]$^+$.

**Example 56: Synthesis of (S)-6-acetamido-13-methoxy-N-methyl-4-oxo-4,6,7,8-tetrahydroheptalen[1',2':4,5]benzo[1,2-d][1,3]dioxolan e-3-carboxamide (125)**

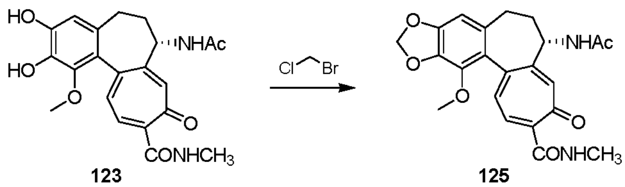
123 125

A mixture containing compound 123 (150 mg, 0.377 mmol), chlorobromomethane (341 mg, 2.64 mmol), potassium carbonate (416 mg, 3.01 mmol) and N-methylpyrrolidone (3 mL) was stirred at 70°C for 1 hour. After cooling to room temperature, water (5 mL) was added, and the mixture was extracted with dichloromethane (10 mL ×3) and dried over anhydrous sodium sulfate. The solvent was evaporated under reduced pressure and the product was purified by preparative HPLC to give (S)-6-acetamido-13-methoxy-N-methyl-4-oxo-4,6,7,8-tetrahydroheptalen[1',2':4,5]benzo[1,2-d][1,3]dioxolane-3-c arboxamide **(125)** (69 mg ). The yield was 44.6 %. $^1$H NMR (DMSO-d6, 400 MHz) δ 9.28 (q, J = 4.4 Hz, 1H), 8.60 (d, J = 7.6 Hz, 1H), 8.22 (d, J = 10.0 Hz, 1H), 7.29 (d, J = 10.0 Hz, 1H), 7.23 (s, 1H), 6.69 (s, 1H), 6.09 (dd, J = 0.8, 6.8 Hz, 2H), 4.35-4.29 (m, 1H), 3.82 (s, 3H), 2.82 (d, J = 4.8 Hz, 3H), 2.62-2.57 (m, 1H), 2.24-2.16 (m, 1H), 2.06-1.96 (m, 1H), 1.89-1.75 (m, 4H). MS (ESI, m/z): 411.4 [M+H]$^+$.

**Example 57: Synthesis of (S)-6-acetamido-N-ethyl-13-methoxy-4-oxo-4,6,7,8-tetrahydroheptalen[1',2':4,5]benzo[1,2-d][1,3]dioxane-3-carboxamide (126) and (S)-6-acetamido-13-methoxy-4-oxo-N-phenyl-4,6,7,8-tetrahydroheptalen[1',2':4,5]benzo[1,2-d] [1,3]dioxolan e-3-carboxamide (127)**

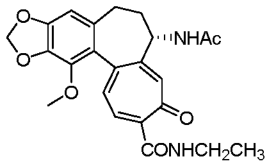
126

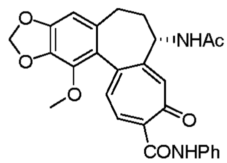
127

The experimental procedures for the synthesis of compound **126** (or **127)** using compound **55** (or **58**) as a raw

material were described in turn in step A of Example 55 and Example 56. compound **126:** $^1$H NMR (DMSO-d6, 400 MHz) δ 9.37 (t, J = 5.6 Hz, 1H), 8.61 (d, J = 7.2 Hz, 1H), 8.20 (d, J = 10.0 Hz, 1H), 7.28 (d, J = 10.0 Hz, 1H), 7.22 (s, 1H), 6.92 (s, 1H), 6.10 (d, J = 5.6 Hz, 2H), 4.34-4.28 (m, 1H), 3.81 (s, 3H), 3.32-3.27 (m, 2H), 2.62-2.57 (m, 1H), 2.24-2.15 (m, 1H), 2.06-1.98 (m, 1H), 1.86-1.75 (m, 4H), 1.12 (t, J = 7.2 Hz, 3H). MS (ESI, m/z): 425.4 $[M+H]^+$. compound **127:** $^1$H NMR (DMSO-d6, 400 MHz) δ 11.34 (s, 1H), 8.64 (d, J = 7.6 Hz, 1H), 8.13 (d, J = 10.0 Hz, 1H), 7.70 (d, J = 7.6 Hz, 2H), 7.39-7.29 (m, 4H), 7.14-7.11 (m, 1H), 6.71 (s, 1H), 6.10 (d, J = 5.2 Hz, 2H), 4.37-4.31 (m, 1H), 3.83 (s, 3H), 2.64-2.59 (m, 1H), 2.27-2.18 (m, 1H), 2.08-2.00 (m, 1H), 1.88-1.78 (m, 4H). MS (ESI, m/z): 473.4 $[M+H]^+$.

**Example 58: Synthesis of (S)-7-acetamido-1,2-dihydroxy-3-methoxy-N-methyl-9-oxo-5,6,7,9-tetrahydrobenzo[a]heptalen-10-carboxa mide (128) and (S)-8-acetamido-4-methoxy-N-methyl-10-oxo-6,7,8,10-tetrahydroheptalen[1', 2':3,4]benzo[1,2-d][1,3]dioxolane-11-carboxamide (129)**

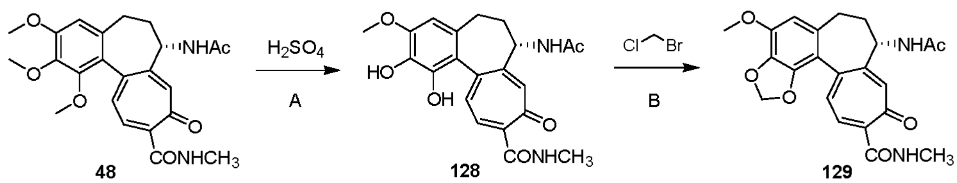


48 128 129

Step A: A mixture containing compound **48** (800 mg, 1.88 mmol) and sulfuric acid (8 mL) was stirred at 60°C for 0.5 h, then warmed to 75 °C and continued stirring for 1.5 h. The mixture was then stirred for 1.5 h. The mixture was then heated to 75 °C and continued stirring. (S)-7-acetamido-1,2-dihydroxy-3-methoxy-N-methyl-9-oxo-5,6,7,9-tetrahydrobenzo[a]heptalen-10-carboxamide **(126)** was obtained. After cooled to room temperature, the reaction mixture was poured batchwise into ice water (20 mL) and the pH was adjusted to 5 ~ 6 with 20% sodium hydroxide solution. The mixture was extracted with a solvent mixture of dichloromethane/methanol (20 mL ×3) and dried over anhydrous sodium sulfate. The solvent was evaporated under reduced pressure and the product was purified by preparative HPLC to give (S)-7-acetamido-1,2-dihydroxy-3-methoxy-N-methyl-9-oxo-5,6,7,9-tetrahydrobenzo[a]heptalen-10-carboxamide **(128)** (370 mg). The yield was 49.4%. MS (ESI, m/z): 399.1 $[M+H]^+$.

The experimental procedures of Step B were described in Example 56 to afford (S)-8-acetamido-4-methoxy-N-methyl-10-oxo-6,7,8,10-tetrahydroheptaleno[1',2':3,4]benzo[1,2-d][1,3]dioxolane-11-carboxamide (129). $^1$H NMR (DMSO-d6, 400 MHz) δ 9.16 (q, J = 4.8 Hz, 1H), 8.60 (d, J = 7.6 Hz, 1H), 8.17 (d, J = 9.6 Hz, 1H), 7.50 (d, J = 9.6 Hz, 1H), 7.22 (s, 1H), 6.67 (s, 1H), 6.14 (s, 1H), 6.08 (s, 1H), 4.40-4.34 (m, 1H), 3.88 (s, 3H), 2.81 (d, J = 4.8 Hz, 3H), 2.70-2.63 (m, 1H), 2.31-2.26 (m, 1H), 2.11-2.08 (m, 1H), 1.92-1.85 (m, 4H). MS (ESI, m/z): 411.4 $[M+H]^+$.

**Example 59: Synthesis of (S)-7-acetamido-1-hydroxy-2,3-dimethoxy-N-methyl-9-oxo-5,6,7,9-tetrahydrobenzo[a]heptalen-10-carboxa mide (131) and (S)-7-acetamido-2,3-dimethoxy-1-trideuteromethoxy-N-methyl-9-oxo-5,6,7,9-tetrahydrobenzo[a]heptalen-1 0-carboxamide (132)**

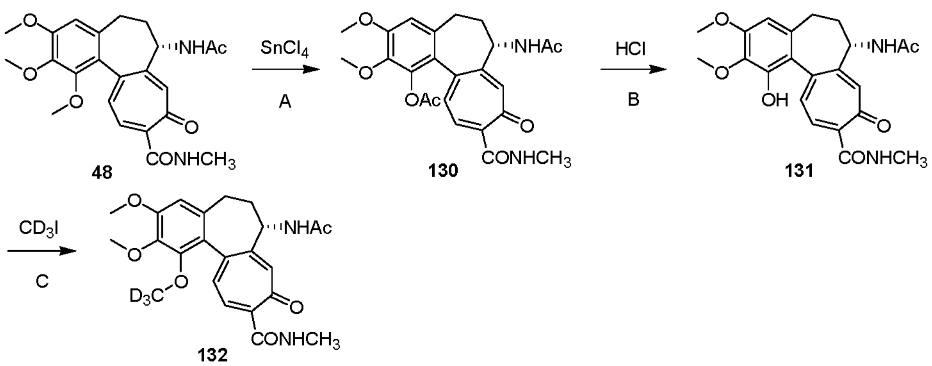


48 130 131 132

Step A: Tin tetrachloride (11.2 g, 42.8 mmol) was added dropwise to a solution of compound 48 (500 mg, 1.17 mmol) and acetyl chloride (3.30 g, 42.0 mmol) in dichloromethane (5 mL) in an ice water bath. After addition, the resulting mixture was stirred at 30°C for 12 hours. Water (20 mL) was added, extraction was performed with dichloromethane

(30 mL ×3), and the combined organic phases were washed with saturated saline (30 mL) and dried over anhydrous sodium sulfate. The solvent was evaporated under reduced pressure to give a crude product (900 mg) of acetic acid {(S)-7-acetamido-2,3-dimethoxy-10-methylcarbamoyl-9-oxo-5,6,7,9-tetrahydrobenzo[a]heptalen-1-yl}ester (130). This compound was used directly in the next step of the reaction without purification.

Step B: A mixture containing compound **130** crude (900 mg) and 4 M hydrochloric acid (10 mL) was stirred at 25 °C for 12 hours. Water (30 mL) was added, extraction was performed with dichloromethane (50 mL ×3), and the combined organic phases were washed with saturated saline (50 mL) and dried over anhydrous sodium sulfate. The solvent was evaporated under reduced pressure and the product was purified by preparative HPLC to afford (S)-7-acetamido-1-hydroxy-2,3-dimethoxy-N-methyl-9-oxo-5,6,7,9-tetrahydrobenzo [a]heptalen-10-carboxamide **(131)** (220 mg). The overall yield of the two-step reaction in steps A and B was 45.6%. MS (ESI, m/z): 413.2 $[M+H]^+$.

The experimental procedures of Step C were described in Step B of Example 54 to give (S)-7-acetamido-2,3-dimethoxy-1-trideutero-methoxy-N-methyl-9-oxo-5,6,7,9-tetrahydrobenzo [a]heptalen-10-car boxamide **(132).** $^1H$ NMR (DMSO-d6, 400 MHz) δ 9.25 (q, J = 4.8 Hz, 1H), 8.64 (d, J = 7.2 Hz, 1H), 8.19 (d, J = 10.0 Hz, 1H), 7.29 (d, J = 10.0 Hz, 1H), 7.22 (s, 1H), 6.80 (s, 1H), 4.31-4.28 (m, 1H), 3.84 (s, 3H), 3.79 (s, 3H), 2.80 (d, J = 4.8 Hz, 3H), 2.66-2.62 (m, 1H), 2.29-2.20 (m, 1H), 2.08-2.00 (m, 1H), 1.84-1.79 (m, 4H). MS (ESI, m/z): 430.4 $[M+H]^+$.

**Example 60: Synthesis of (S)-7-acetamido-2-difluoromethoxy-1,3-dimethoxy-N-methyl-9-oxo-5,6,7,9-tetrahydrobenzo[a]heptalen-10-carboxamide (133)**

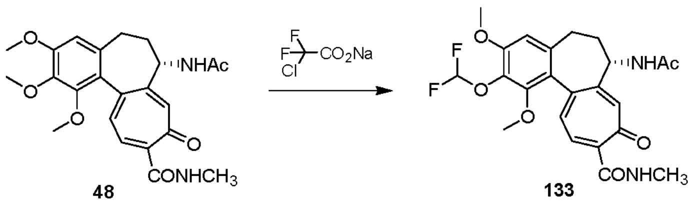


48

133

A mixture containing compound **48** (200 mg, 0.485 mmol), sodium difluorochloroacetate (74 mg, 0.485 mmol), cesium carbonate (316 mg, 0.970 mmol) and DMF (2 mL) was stirred at 70°C for 2 hours. After cooled to room temperature, water (15 mL) was added, extraction was performed with dichloromethane (30 mL ×3), and the combined organic phases were washed with saturated brine (15 mL ×2) and dried over anhydrous sodium sulfate. The solvent was evaporated under reduced pressure and the product was purified by preparative HPLC to give (S)-7-acetamido-2-difluoromethoxy-1,3-dimethoxy-N-methyl-9-oxo-5,6,7,9-tetrahydrobenzo [a]heptalen-10-carbo xamide **(133).** $^1H$ NMR (DMSO-d6, 400 MHz) δ 9.19 (q, J = 4.4 Hz, 1H), 8.68 (d, J = 7.2 Hz, 1H), 8.16 (d, J = 10.0 Hz, 1H), 7.32 (d, J = 10.0 Hz, 1H), 7.23 (s, 1H), 7.18 (s, 0.25H), 7.00 (d, J = 2.8 Hz, 0.5H), 6.96 (s, 1H), 6.81 (s, 0.25H), 4.34-4.28 (m, 1H), 3.88 (s, 3H), 3.54 (s, 3H), 2.81 (d, J = 4.8 Hz, 3H), 2.74-2.69 (m, 1H), 2.34-2.26 (m, 1H), 2.10-2.01 (m, 1H), 1.90-1.83 (m, 4H). MS (ESI, m/z): 463.3 $[M+H]^+$.

**Example 61: Synthesis of (S)-7-acetamido-2-hydroxy-1-methoxy-3-trideutero-methoxy-N-methyl-9-oxo-5,6,7,9-tetrahydrobenzo [a]he ptalen-10-carboxamide (134) and (S)-7-acetamido-3-hydroxy-1-methoxy-2-trideutero-methoxy-N-methyl-9-oxo-5,6,7,9-tetrahydrobenzo [a]he ptalen-10-carboxamide (135) and (S)-7-acetamido-1,2-dimethoxy-3-trideutero-methoxy-N-methyl-9-oxo-5,6,7,9-tetrahydrobenzo[a]heptalene-10-carboxamide (136), and (S)-7-acetamido-3-difluoromethoxy-1-methoxy-2-trideutero-methoxy-N-methyl-9-oxo-5,6,7,9-tetrahydrobenzo[a]heptalen-10-carboxamide (137)**

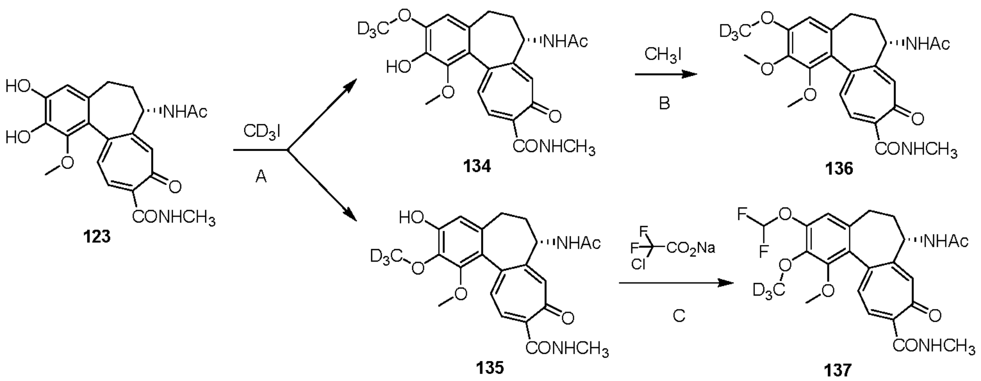


123 134 135 136 137

Step A: To a solution of compound **123** (500 mg, 1.25 mmol) in DMF (5 mL) were added potassium carbonate (520 mg, 3.76 mmol) and iodomethane-d3 (200 mg, 1.38 mmol), and after addition, the resulting mixture was stirred at 50°C for 0.5 hr. Water (20 mL) was added and extraction was performed with ethyl acetate (20 mL ×3). The combined organic phases were washed sequentially with water (10 mL × 2) and saturated saline (10 mL) and dried over anhydrous sodium sulfate. The solvent was evaporated under reduced pressure and the product was separated by preparative HPLC and SFC to give (S)-7-acetamido-2-hydroxy-1-methoxy-3-trideutero-methoxy-N-methyl-9-oxo-5,6,7,9-tetrahydrobenzo[a]heptalen -10-carboxamide (134) and (S)-7-acetamido-3-hydroxy-1-methoxy-2-trideutero-methoxy-N-methyl-9-oxo-5,6,7,9-tetrahydrobenzo[a]heptalen-10-carboxamide **(135).** Compound **134:** $^1$H NMR (DMSO-d6, 400 MHz) δ 9.28 (q, J = 4.4 Hz, 1H), 8.79 (s, 1H), 8.63 (d, J = 7.6 Hz, 1H), 8.20 (d, J = 10.0 Hz, 1H), 7.30 (d, J = 10.0 Hz, 1H), 7.23 (s, 1H), 6.70 (s, 1H), 4.39-4.26 (m, 1H), 3.50 (s, 3H), 2.81 (d, J = 4.8 Hz, 3H), 2.60-2.55 (m, 1H), 2.22-2.20 (m, 1H), 2.03-1.99 (m, 1H), 1.84-1.77 (m, 4H). MS (ESI, m/z): 416.4 [M+H]$^+$. Compound **135:** $^1$H NMR (DMSO-d6, 400 MHz) δ 9.77 (s, 1H), 9.28 (q, J = 4.4 Hz, 1H), 8.62 (d, J = 7.6 Hz, 1H), 8.20 (d, J = 10.0 Hz, 1H), 7.29 (d, J = 10.0 Hz, 1H), 7.22 (s, 1H), 6.58 (s, 1H), 4.34-4.28 (m, 1H), 3.57 (s, 3H), 2.81 (d, J = 4.8 Hz, 3H), 2.60-2.55 (m, 1H), 2.19-2.17 (m, 1H), 2.02-1.99 (m, 1H), 1.84-1.75 (m, 4H). MS (ESI, m/z): 416.4 [M+H]$^+$.

The experimental procedures of step B were described in Example 54, wherein the Iodomethane-d3 in step B of Example 54 was replaced with iodomethane to give (S)-7-acetamido-1,2-dimethoxy-3-trideutero-methoxy-N-methyl-9-oxo-5,6,7,9-tetrahydrobenzo [a]heptalen-10-car boxamide (136). $^1$H NMR (DMSO-d6, 400 MHz) δ 9.25 (q, J = 4.4 Hz, 1H), 8.64 (d, J = 7.6 Hz, 1H), 8.20 (d, J = 9.6 Hz, 1H), 7.29(d, J = 9.6 Hz, 1H), 7.23 (s, 1H), 6.80 (s, 1H), 4.33-4.27 (m, 1H), 3.79 (s, 3H), 3.58 (s, 3H), 2.81 (d, J = 4.8 Hz, 3H), 2.66-2.62 (m, 1H), 2.26-2.24 (m, 1H), 2.05-2.02 (m, 1H), 1.87-1.82(m, 4H). MS (ESI, m/z): 430.4 [M+H]$^+$.

The experimental procedures of Step C were described in Example 60 to afford (S)-7-acetamido-3-difluoromethoxy-1-methoxy-2-trideutero-methoxy-N-methyl-9-oxo-5,6,7,9-tetrahydrobenzo [a] heptalen-10-carboxamide (137). $^1$H NMR (DMSO-d6, 400 MHz) δ 9.17 (q, J = 4.4 Hz, 1H), 8.66 (d, J = 7.2 Hz, 1H), 8.16 (d, J = 10.0 Hz, 1H), 7.41 (s, 0.25H), 7.31 (d, J = 10.0 Hz, 1H), 7.22-7.21 (m, 1.5H), 7.04 (s, 0.25H), 6.94 (s, 1H), 4.30-4.24 (m, 1H), 3.59 (s, 3H), 2.81 (d, J = 4.8 Hz, 3H), 2.70-2.65 (m, 1H), 2.27-2.23 (m, 1H), 2.03-2.00 (m, 1H), 1.87-1.82 (m, 4H). MS (ESI, m/z): 466.0 [M+H]$^+$.

### Example 62: Pharmacodynamics experiment A of compounds on gouty arthritis in rats

1. Experimental materials

(1) Test drug

Compounds **2, 17, 26** and **27** were prepared by grinding with 0.5% CMC-Na into a suspension of the corresponding concentration (0.06 mg/mL) for gavage before administration, and the volume of administration was 10 mL/kg. Colchicine was purchased from Sarn Chemical Technology (Shanghai) Co. with a batch number 49ETRRAS. Before use, 0.5% CMC-Na was used for grinding and prepared into a suspension of the corresponding concentration (0.06 mg/mL) for gavage, and the volume of drug administered was 10 mL/kg.

(2) Experimental animals

Sprague Dawley (SD) rats, SPF grade, male, weighing 180-240 g, were purchased from Zhejiang Vital River Laboratory Animal Technology Co., Ltd., under License No.: SCXK (Zhejiang)2019-0001; Animal Qualification Certificate No.: 20210119Aazz0619000545 (Zhejiang).

2 Experimental methods

(1) Grouping method

70 SD rats, males, weighing 180-240 g, were randomly divided into 7 groups of 10 animals each: blank control group (0.5% CMC-Na), model group (0.5% CMC-Na), positive control colchicine group, compound **2** group, compound **17** group, compound **26** group, and compound **27** group, and the administered dose was 0.6 mg/kg. Each group of subjects was prepared into a suspension of the corresponding concentration, and the administration volume was 10 mL/kg.

(2) Modeling and drug administration

The crystalline arthritis model was prepared by intra-articular injection of urate crystals (40 mg/mL, in the left ankle joint of each rat). Different doses of the test drug were given separately by gavage administration. The day of administration was defined as day 1 of the experiment, and the drug was administered once a day for 10 consecutive days (pre-administration of the drug for 7 days prior to modeling, modeling on day 8, and continuation of the drug for 3 days). The experiment ended on day 10.

(3) Test indicators

a. Paw volume

Paw volume was measured before administration, before urate injection, and 2, 4, 8, 10, 24, and 48 h after injection.

b. Gait score

The degree of lameness of the modeled joints was observed and scored at the same time point.

c. Pain score

The pain level was scored using the flexion and extension joint pain test scoring method at the same time point.

(4) Data processing and statistical methods

The data obtained from the experiment were statistically analyzed using IBM SPSS Statistics 22.0. All measurements were expressed as Mean±S.E.M s. The data were analyzed using SPSS 22.0 statistical software and variance chi-square test was performed on the parameters.

3. Experimental results

(1) Paw volume

The paw volume data of rats in each group are shown in Table 1. At each time point after modeling, the paw volume of rats in the model group was significantly larger than that of the blank control group ($P < 0.001$). The paw volumes of rats in the colchicine group were significantly smaller than those in the model group at 2 h, 4 h, 8 h, 10 h, and 48 h after modeling ($P < 0.01$). Each test compound group could reduce the paw volume of rats at different time points after modeling, among which, compounds **2** and **26** could significantly reduce the paw volume at 2 h, 4 h, and 48 h, compound **17** could significantly reduce the paw volume at 2 h, 8 h, 24 h, and 48 h, and compound **27** could significantly reduce the paw volume at 2 h, 4 h, 8 h, 10 h, and 48 h.

**Table 1. Paw volume ($cm^3$) for each group (Mean±SEM)**

| Compound name or No. | Dose (mg/kg) | Before modeling | 2h | 4h | 8 h | 10 h | 24 h | 48 h |
|---|---|---|---|---|---|---|---|---|
| **blank control group** | - | 2.18±0.04 | 2.15±0.02 | 2.15±0.01 | 2.14±0.02 | 2.13±0.02 | 2.15±0.02 | 2.14±0.03 |
| **model group** | - | 2.22±0.03 | 2.71±0.05### | 2.67±0.04### | 2.66±0.04### | 2.64±0.03### | 2.60±0.03### | 2.52±0.04### |
| **colchicine** | 0.6 | 2.18±0.03 | 2.44±0.02*** | 2.45±0.03** | 2.44±0.04*** | 2.46±0.04** | 2.46±0.05 | 2.24±0.03*** |
| **2** | 0.6 | 2.18±0.02 | 2.51±0.06* | 2.54±0.04* | 2.63±0.06 | 2.59±0.07 | 2.52±0.08 | 2.35±0.06* |
| **17** | 0.6 | 2.19±0.03 | 2.54±0.02** | 2.58±0.02 | 2.54±0.04* | 2.55±0.03 | 2.41±0.05** | 2.23±0.03*** |
| **26** | 0.6 | 2.19±0.04 | 2.53±0.04* | 2.54±0.03* | 2.55±0.04 | 2.57±0.05 | 2.47±0.06 | 2.34±0.07* |
| **27** | 0.6 | 2.16±0.04 | 2.51±0.04** | 2.54±0.03* | 2.57±0.04* | 2.55±0.04* | 2.50±0.07 | 2.29±0.04** |

Note: ### P<0.001, compared with the blank control group at the same time point; *P<0.05, **P<0.01, ***P<0.001, compared with the model group at the same time point.

(2) Gait score

The gait scores of rats in each group were shown in Table 2. At each time point after modeling, the gait scores of rats in the model group were significantly higher than those in the blank control group ($P<0.001$). The colchicine group improved the gait of rats at 2 h, 24 h, and 48 h after modeling ($P < 0.05$ or $P < 0.01$). Each tested compound group could improve the gait of rats at different time points after modeling, among which, compound 2 significantly improved the gait at 2 h, 8 h, 24 h, and 48 h, compounds **17** and **26** significantly improved the gait at 2 h, 8 h, and 24 h, and compound **27** significantly improved the gait at 2 h, 24 h, and 48 h ($P < 0.05$ or $P < 0.01$).

**Table 2. Effect of compounds on gait scores in rats with gouty arthritis (Mean±SEM)**

| Compound name or No. | Dose (mg/kg) | Before modeling | 2h | 4h | 8 h | 10 h | 24 h | 48 h |
|---|---|---|---|---|---|---|---|---|
| **blank control group** | - | 0.00±0.00 | 0.00±0.00 | 0.00±0.00 | 0.00±0.00 | 0.00±0.00 | 0.00±0.00 | 0.00±0.00 |
| **model group** | - | 0.00±0.00 | 1.60±0.16### | 1.30±0.1### | 1.70±0.1### | 1.40±0.1### | 1.60±0.1### | 1.50±0.17### |
| **colchicine** | 0.6 | 0.00±0.00 | 1.10±0.10* | 1.10±0.10 | 1.30±0.15 | 1.30±0.21 | 1.00±0.00** | 1.00±0.00* |
| **2** | 0.6 | 0.00±0.00 | 1.10±0.10* | 1.10±0.10 | 1.10±0.10** | 1.20±0.13 | 1.00±0.00** | 1.00±0.00* |
| **17** | 0.6 | 0.00±0.00 | 1.00±0.00** | 1.20±0.13 | 1.00±0.00** | 1.10±0.10 | 1.10±0.10* | 1.10±0.10 |
| **26** | 0.6 | 0.00±0.00 | 1.00±0.00** | 1.10±0.10 | 1.20±0.13* | 1.30±0.15 | 1.10±0.10** | 1.10±0.10 |
| **27** | 0.6 | 0.00±0.00 | 1.00±0.00** | 1.10±0.10 | 1.40±0.22 | 1.20±0.20 | 1.00±0.00** | 1.00±0.00* |

Note: ####P<0.001, compared with the normal control group at the same time point; *P<0.05, **P<0.01, compared with the model group at the same time point.

(3) Pain scores

The pain scores of rats in each group were shown in Table 3. At each time point after modeling, the pain scores of rats in the model group were significantly higher than those in the blank control group ($P<0.001$). The colchicine group improved the pain at 2 h, 8 h, 24 h, and 48 h ($P < 0.05$ or $P < 0.01$). Each test compound group could improve rat pain at different time points after modeling, among which, compound 2 significantly improved pain at 2 h, 4 h, 8 h, 10 h, 24 h, and 48 h, compound **17** significantly improved gait at 2 h, 8 h, and 24 h, compound **26** significantly improved gait at 2 h, and compound **27** significantly improved gait at 2 h, 8 h, 10 h, 24 h, and 48 h ($P < 0.05$ or $P < 0.01$).

Table 3. Pain scores of rats in each group (Mean±SEM)

| Compound name or No. | Dose (mg/kg) | Before modeling | 2h | 4 h | 8 h | 10 h | 24 h | 48 h |
|---|---|---|---|---|---|---|---|---|
| **blank control group** | - | 0.00±0.00 | 0.00±0.00 | 0.00±0.00 | 0.00±0.00 | 0.00±0.00 | 0.00±0.00 | 0.00±0.00 |
| **model group** | - | 0.00±0.00 | 4.80±0.13### | 4.20±0.33### | 4.80±0.13### | 4.20±0.36### | 3.60±0.56### | 3.50±0.62### |
| **colchicine** | 0.6 | 0.00±0.00 | 3.30±0.60* | 2.90±0.71 | 3.20±0.61* | 2.60±0.70 | 1.40±0.58* | 0.80±0.44** |
| **2** | 0.6 | 0.00±0.00 | 2.70±0.68* | 1.30±0.52*** | 3.30±0.54* | 2.20±0.68* | 1.30±0.62* | 1.00±0.67* |
| **17** | 0.6 | 0.00±0.00 | 3.10±0.64* | 3.20±0.66 | 3.00±0.68* | 3.30±0.54 | 1.30±0.65* | 1.60±0.70 |
| **26** | 0.6 | 0.00±0.00 | 3.00±0.56* | 2.90±0.67 | 4.30±0.40 | 3.80±0.59 | 3.20±0.51 | 2.20±0.73 |
| **27** | 0.6 | 0.00±0.00 | 2.00±0.67** | 2.80±0.68 | 3.30±0.47* | 2.50±0.65* | 1.50±0.60* | 1.00±0.60** |

Note: ###P<0.001, compared with blank control group at the same time point; *P<0.05, **P<0.01, compared with model group at the same time point.

**Example 63: Pharmacodynamics experiment B of compounds on gouty arthritis rats**

1. Experimental materials

(1) Test drug

Compounds **12, 16, 19, 30, 34, 36, 38, 40, 44, 48, 49, 52, 58, 69, 83, 98, 102, 119, 121,** and **122** were prepared by grinding with with 0.5% CMC-Na prior to administration and formulated into suspensions of appropriate concentration (0.12 mg/mL) for gavage, with an administered dose of 0.5 mL/100 g. Colchicine, purchased from MCE Company, with Lot No. 41632, before use, was prepared by grinding with 0.5% CMC-Na and prepared into a suspension of corresponding concentration (0.12 and 0.24 mg/mL) for gavage, and the volume of administration was 0.5 mL/100 g.

(2) Experimental animals

Sprague Dawley (SD) rats, SPF grade, male, weighing 180-220 g, were purchased from slac laboratory animal Co. Ltd, Production License No.: SCXK (Shanghai) 2017-0005, Quality Certificate No.: 20170005054931.

(3) Experimental reagents

Sodium urate, purchased from Sigma, saline containing 10% Tween-80 was used to formulate a suspension with a concentration of 25 mg/mL.

2. Experimental methods

(1) Grouping method

240 SD rats, males, were acclimatized for one week and weighed 180-220 g. They were randomly divided into 24 groups of 10 rats each: blank control group (0.5% CMC-Na), model group (0.5% CMC-Na), positive control colchicine low-dose group (0.6 mg/kg), positive control colchicine high-dose group (1.2 mg/kg), and each compound groups. The dose of each compound group was 0.6 mg/kg. The compounds in each group were prepared into the corresponding concentration suspension, and the volume of administration was 10 mL/kg.

(2) Modeling and dosing regimen

250 mg of sodium urate were added to 9 mL of saline, and 1 mL of Tween-80 solution was added, the resulting mixture was heated and stirred to formulate 10 mL of sodium urate solution. The rats were grasped and fixed, and 0.2 mL of sodium urate solution was injected into the right ankle and knee joint cavities respectively to establish gouty arthritis rat model.

Before modeling, positive drug and tested compounds were pre-administered for 7 days, once a day, and modeling was performed on day 8, and drug administration by gavage continued for 3 days after modeling.

3. Test indicators

(1) Gait score

Gait was scored according to the modified gait grading criteria introduced by Coderre et al. The gait of each rat was observed before and 2 h, 4 h, 8 h, 10 h, 24 h and 48 h after modeling and graded scoring was performed. The gait grading criteria were as follows: 0 point, normal walking; 1 point, slight lameness, the lower limbs were slightly bent; 2 points: moderate lameness, the lower limbs just touched the ground; 3 points, severe lameness, the lower limbs left the ground, and walked on three feet. The mean score of each group was calculated and compared with that of the model group.

(2) Ankle swelling rate

Before modeling and 2 h, 4 h, 8 h, 10 h, 24 h and 48 h after modeling, the circumference of the right ankle joint was measured using a soft ruler or the volume change at the ankle was measured (volumetric method), and the swelling degree and swelling rate of ankle joint were calculated:

$$\text{Swelling degree (mm)} = \text{measured circumference (mm)} - \text{baseline circumference (mm)}$$

$$\text{Swelling rate (\%)} = \text{swelling degree (mm)/baseline circumference (mm)} \times 100\%$$

(3) Data processing and statistical methods

Each experimental data was expressed as the mean, and intergroup comparisons were examined for significance using the ANOVA-Dunnett T-test, with P<0.05 as the significance indicator.

4. Experimental results

(1) Gait score

The gait scores of rats in each group were shown in Table 4. Compared with the control group, the gait scores of the model group were significantly higher at 8 h and 10 h after modeling (P<0.05), but the gait began to recover at 24 h after modeling. At 4 h, 8 h, 10 h and 24 h after modeling, compared with the model group, colchicine low-dose and high-dose groups significantly improved the gait of rats (P<0.05), and significant improvement in gait occurred at different time points in compound **12, 16, 19, 30, 34, 36, 38, 40, 44, 48, 49, 52, 58, 69, 83** and **98** groups (P<0.05 or P <0.01).

**Table 4. Effect of compounds on gait scores in gouty arthritis rats**

| Compound name or No. | Dose (mg/kg) | Gait Score | | | | | | |
|---|---|---|---|---|---|---|---|---|
| | | 0 h | 2h | 4h | 8 h | 10 h | 24 h | 48 h |
| **Blank control group** | / | 0 | 0 | 0 | 0 | 0 | 0 | 0 |
| **model group** | / | 0 | 0 | 0.17 | 0.45# | 0.53# | 0.20 | 0.08 |
| **colchicine low dose group** | 0.6 | 0 | 0 | 0.02* | 0.24* | 0.16** | 0.02* | 0.02 |
| **colchicine high dose group** | 1.2 | 0 | 0 | 0** | 0.1** | 0.18** | 0* | 0 |
| **12** | 0.6 | 0 | 0 | 0 | 0* | 0* | 0 | 0 |
| **16** | 0.6 | 0 | 0 | 0.1 | 0* | 0* | 0 | 0 |
| **19** | 0.6 | 0 | 0.0 | 0 | 0** | 0** | 0.2 | 0.1 |
| **30** | 0.6 | 0 | 0 | 0 | 0* | 0* | 0.3 | 0 |
| **34** | 0.6 | 0 | 0 | 0 | 0** | 0.1** | 0 | 0 |
| **36** | 0.6 | 0 | 0 | 0 | 0* | 0* | 0.2 | 0.1 |
| **38** | 0.6 | 0 | 0 | 0 | 0* | 0* | 0 | 0 |
| **40** | 0.6 | 0 | 0 | 0 | 0* | 0* | 0.4 | 0 |
| **44** | 0.6 | 0 | 0 | 0 | 0* | 0* | 0 | 0 |
| **48** | 0.6 | 0 | 0 | 0.1 | 0.1** | 0** | 0* | 0 |
| **49** | 0.6 | 0 | 0 | 0 | 0* | 0.* | 0.2 | 0 |
| **52** | 0.6 | 0 | 0 | 0 | 0* | 0* | 0 | 0 |
| **58** | 0.6 | 0 | 0 | 0* | 0.3 | 0.2** | 0 | 0 |
| **69** | 0.6 | 0 | 0 | 0 | 0* | 0* | 0 | 0 |
| **83** | 0.6 | 0 | 0 | 0 | 0* | 0* | 0 | 0 |
| **98** | 0.6 | 0 | 0 | 0 | 0* | 0* | 0.1 | 0 |
| **102** | 0.6 | 0 | 0 | 0 | 0.27 | 0.09* | 0 | 0 |
| **119** | 0.6 | 0 | 0 | 0 | 0.1* | 0.4 | 0. | 0 |

(continued)

| Compound name or No. | Dose (mg/kg) | Gait Score | | | | | | |
|---|---|---|---|---|---|---|---|---|
| | | 0 h | 2h | 4h | 8 h | 10 h | 24 h | 48 h |
| **121** | 0.6 | 0 | 0 | 0 | 0.2 | 0.2 | 0 | 0 |
| **122** | 0.6 | 0 | 0 | 0.2 | 0.1 | 0.2 | 0.1 | 0 |
| Note: #P<0.05, compared with the blank control group at the same time point; *P<0.05, **P<0.01, compared with the model group at the same time point. | | | | | | | | |

(2) Ankle joint swelling rate

The ankle joint swelling rate of rats in each group were shown in Table 5. Compared with the blank control group, the ankle joint swelling rate was significantly higher in the model group at each time point after modeling (P<0.01). The ankle swelling rate of rats in colchicine low and high dose groups were significantly (P<0.01) reduced after modeling compared with the model group at the same time point. The ankle swelling rate of rats in all the tested compounds groups at different time points were significantly reduced after modeling (P<0.05 or P<0.01).

**Table 5. Effect of compounds on ankle swelling rate in gouty arthritis rats ($\bar{x}$)**

| Compound name or No. | Dose (mg/kg) | Swelling rate | | | | | |
|---|---|---|---|---|---|---|---|
| | | 2h | 4h | 8 h | 10 h | 24 h | 48 h |
| **blank control group** | / | 1.66% | 1.28% | 2.24% | 2.39% | 2.05% | 1.29% |
| **model group** | / | 15.72%## | 19.75%## | 27.05%## | 32.66%## | 31.26%## | 19.07%## |
| **colchicine low dose group** | 0.6 | 10.49%** | 15.73%* | 20.61%** | 23.39%** | 24.38%** | 12.11%** |
| **colchicine high dose group** | 1.2 | 8.97%* * | 14.92%** | 17.18%** | 19.37%* * | 19.82%** | 9.29%** |
| **12** | 0.6 | 10.77% | 16.53% | 18.70%* | 31.35% | 22.77%* | 15.01% |
| **16** | 0.6 | 9.71% | 12.97%* | 17.78%** | 17.38%** | 18.59%** | 6.17%** |
| **19** | 0.6 | 8.77%* | 10.88%** | 17.09%* * | 14.05%** | 19.45%** | 7.77%** |
| **30** | 0.6 | -4.04%** | 1.20%** | 0.57%** | 6.69%** | 9.77%** | -1.34%** |
| **34** | 0.6 | 3.98%** | 8.32%** | 16.22%** | 17.30%** | 21.05%** | 9.96%** |
| **36** | 0.6 | -3.59%** | 2.37%** | 2.62%** | 10.45%** | 10.03%** | -2.54%** |
| **38** | 0.6 | 6.34%** | 12.63%* | 10.41%** | 17.87%** | 16.90%** | 6.71%** |
| **40** | 0.6 | 5.81%** | 7.96%** | 11.21%** | 9.90%** | 21.73%* | 9.33%* |
| **44** | 0.6 | -9.72%** | -8.92%** | -6.13%** | -1.47%** | 3.17%** | -6.20%** |
| **48** | 0.6 | 9.42%** | 15.52%* | 21.03%* | 21.47%** | 22.39%** | 13.60%* |
| **49** | 0.6 | -1.02%** | 7.38%** | 8.12%** | 16.54%** | 12.89%** | 1.43%** |
| **52** | 0.6 | 10.98% | 9.83%** | 11.79%** | 30.20% | 21.98%* | 7.88%* |
| **58** | 0.6 | 6.48%** | 13.85%** | 17.19%* * | 20.43%** | 18.13%** | 8.42%** |
| **69** | 0.6 | 5.65%** | 7.01%** | 14.66%** | 26.82%* | 19.00%* | 5.21%** |
| **83** | 0.6 | 8.31%** | 10.38%** | 16.57%** | 21.13%* | 26.26% | 15.28% |
| **98** | 0.6 | 7.9%* * | 13.4%* | 16.4%** | 17.7%** | 19.7%* | 8.9%* |
| **102** | 0.6 | 14.1% | 13.6%** | 14.4%** | 19.3%** | 16.7%** | 9.4%* |
| **119** | 0.6 | 10.4% | 14.1%** | 19.4%* | 22.2%* | 15.8%** | 5.3%** |

(continued)

| Compound name or No. | Dose (mg/kg) | Swelling rate | | | | | |
|---|---|---|---|---|---|---|---|
| | | 2h | 4h | 8 h | 10 h | 24 h | 48 h |
| **121** | 0.6 | 11.8% | 15.6%* | 20.9% | 22.5%* | 16.3%** | 9.4%* |
| **122** | 0.6 | 7.9%* * | 12.1%** | 15.8%** | 15.3%** | 12.5%** | 4.7%** |
| Note: ##P<0.01, compared with blank control group at same time point; *P<0.05, **P<0.01, compared with model group at same time point. | | | | | | | |

**Example 64: Pharmacodynamics experiment A of compounds on inflammatory factor levels**

1. Experimental materials

(1) Test drugs

Compounds **14, 17** and **26** were prepared by grinding with 0.5% CMC-Na before administration and into a suspension for gavage at the corresponding concentration (0.06 mg/mL), with a dosage volume of 10 mL/kg. Colchicine, purchased from Sun Chemical Technology (Shanghai) Co., Ltd. with batch no. 49ETRRAS, and was prepared by grinding with 0.5% CMC-Na before administration and into a suspension for gavage at the corresponding concentration (0.06 mg/mL), with a dosage volume of 10 mL/kg.

(2) Experimental animals

Sprague Dawley (SD) rats, SPF grade, male, weighing 180-240 g, were purchased from Zhejiang Vital River Laboratory Animal Technology Co., Ltd., with License No.: SCXK (Zhe)2019-0001; Animal Qualification Certificate No.: 20210119Aazz0619000545 (Zhejiang).

2. Experimental methods

(1) Grouping method

Sixty SD rats, males, weighing 180-240 g, were randomly divided into six groups of 10 animals each: blank control group (0.5% CMC-Na), model group (0.5% CMC-Na), positive control colchicine group, compound 14 group, compound **17** group, and compound **26** group, and the administered dose was all 0.6 mg/kg. The compounds in each group were prepared into a suspension of the corresponding concentration, and the dosage volume was 10 mL/kg.

(2) Modeling and dosing

The crystalline arthritis model was prepared by intra-articular injection of urate crystals (40 mg/mL in the left ankle joint of each rat). Different doses of the test compounds were given separately by gavage administration. The day of administration was defined as day 1 of the experiment, and the compounds were administered once a day for 10 consecutive days (pre-administration of the drug for 7 days prior to modeling, modeling on day 8, and continuation of the drug for 3 days). The experiment ended on day 10.

(3) Test indicators

a. Inflammatory factor

At the end of the experiment (day 10), animals were dissected, and the synovial leachate from the ankle joint cavity at the modeling site was taken to test the levels of IL-6 and TNF-$\alpha$.

(4) Data processing and statistical methods

The data obtained from the experiment were statistically analyzed using IBM SPSS Statistics 22.0. The data were analyzed using SPSS 22.0 statistical software, and the parameters were subjected to variance chi-square test.

3. Experimental results

(1) TNF-α

The results of the decreased percentage of TNF-α in the synovial tissue of the knee joint of rats in each group are shown in Table 6. Colchicine can reduce the TNF-α level in the synovial tissue of rat knee joints after modeling. All compound could reduce the TNF-α level in the synovial membrane of rat knee joints after modeling.

**Table 6. Effect of compounds on TNF-α in synovial tissue of knee joints of gouty arthritis rats**

| Compound name or No. | Dose (mg/kg) | Decreased TNF-α percentage % |
|---|---|---|
| **Colchicine** | 0.6 | 93.73 |
| **14** | 0.6 | 125.78 |
| **17** | 0.6 | 107.16 |
| **26** | 0.6 | 160.36 |

(2) IL-6

The results of the decreased percentage of IL-6 in the synovial tissue of the knee joint of rats in each group were shown in Table 7. Colchicine reduced the IL-1β level in the synovial tissue of the knee joint of rats after modeling. All compounds decreased the IL-6 level in the synovial tissue of the knee joint of rats after modeling.

**Table 7. Effect of compounds on IL-6 in synovial tissue of knee joints of gouty arthritis rats**

| Compound name or No. | Dose (mg/kg) | Decreased IL-6 percentage % |
|---|---|---|
| **colchicine** | 0.6 | 107.14 |
| **14** | 0.6 | 119.58 |
| **17** | 0.6 | 132.54 |
| **26** | 0.6 | 113.37 |

**Example 65: Pharmacodynamics experiment B of compounds on levels of inflammatory factors**

1. Experimental materials

(1) Test drugs

Compounds **19, 34, 38, 44, 48, 49, 55, 58, 60, 61, 69, 81, 95, 97, 98, 99, 100, 108, 112,119, 121, and 122** were prepared by grinding with 0.5% CMC-Na prior to administration and formulated into suspensions of appropriate concentration (0.12 mg/mL) for gavage, with the administration volume of 0.5 mL/100 g. Colchicine, purchased from MCE Company with Lot No. 41632, was prepared by grinding with 0.5% CMC-Na and prepared into a suspension of the corresponding concentration (0.12 and 0.24 mg/mL) for gavage before use, and the volume of administration was 0.5 mL/100 g.

(2) Experimental animals

Sprague Dawley (SD) rats, SPF grade, male, weighing 180-220 g, were purchased from slac laboratory animal co. ltd with Production License No.: SCXK (Shanghai) 2017-0005, Quality Certificate No.: 20170005054931.

(3) Experimental reagents

Sodium urate, purchased from Sigma, was used to prepare a suspension with a concentration of 25 mg/mL using saline containing 10% Tween-80. IL-1β and TNF-α detection kits (ELISA method) were purchased from Wuhan Elabscience Biotechnology Co.,Ltd. IL-6 detection kits (ELISA method) were purchased from Hangzhou MultiSciences Biotech Co., Ltd.

2. Experimental methods

(1) Grouping method

260 SD rats, male, one week after acclimatization, weighing 180-220 g, were randomly divided into 26 groups of 10 rats each: blank control group (0.5% CMC-Na), model group (0.5% CMC-Na), positive control colchicine low-dose group (0.6 mg/kg), positive control colchicine high-dose group (1.2 mg/kg), and the test Compound group. The dose of compound group was 0.6 mg/kg. The compound in each group were prepared into the corresponding concentration suspension, and the dosage volume was 10 mL/kg.

(2) Modeling and dosing regimen

250 mg of sodium urate were added to 9 mL of saline, and 1 mL of Tween-80 solution was added, and the resulting mixture was heated and stirred to formulate 10 mL of sodium urate solution. The rats were grasped and fixed, and 0.2 mL of sodium urate solution was injected into the right ankle and knee joint cavities respectively to establish gouty arthritis rat model.

Positive drug and test compounds were pre-administered for 7 days before modeling, once a day, modeling was performed on day 8, and administration by gavage continued for 3 days after modeling.

(3) Test indicators

a. Inflammatory factors

Measurement of IL-1β, IL-6 and TNF-α in synovial tissue: 48 h after modeling, rats were killed by decapitation, synovial tissues of the knee joints were dissected to prepare tissue homogenates, and ELISA kit was used to measure the level of IL-1β, IL-6 and TNF-α in synovial tissues, and the percentage decrease of each inflammatory factor was calculated.

(4) Data processing and statistical methods

The data of each experimental measure were expressed as percentage decrease.

3. Experimental results

(1) TNF-α

The results of the decreased percentage of TNF-α in the synovial tissue of the knee joint of rats in each group were shown in Table 8. Both low-dose and high-dose groups of colchicine could reduce the TNF-α lelvel of synovial tissue of rat knee joints after modeling. All compound groups could reduce the TNF-α lelvel in the synovial tissue of the knee joint of rats after modeling, and the inhibition of the TNF-α lelvel in the synovial tissue of the knee joint of rats after modeling was significantly better than that of colchicine.

**Table 8. Effect of compounds on TNF-α in synovial tissue of knee joints of gouty arthritis rats**

| Compound name or No. | Dose (mg/kg) | TNF-α percentage decrease % | Compound name or No. | Dose (mg/kg) | Decreased TNF-α percentage % |
|---|---|---|---|---|---|
| **colchicine low dose group** | 0.6 | 57.97 | **69** | 0.6 | 76.08 |
| **colchicine high dose group** | 1.2 | 52.46 | **81** | 0.6 | 77.98 |
| **19** | 0.6 | 84.70 | **95** | 0.6 | 70.03 |
| **34** | 0.6 | 82.86 | **97** | 0.6 | 92.04 |
| **38** | 0.6 | 86.57 | **98** | 0.6 | 98.89 |
| **44** | 0.6 | 72.57 | **99** | 0.6 | 91.73 |
| **48** | 0.6 | 81.58 | **100** | 0.6 | 119.69 |

(continued)

| Compound name or No. | Dose (mg/kg) | TNF-α percentage decrease % | Compound name or No. | Dose (mg/kg) | Decreased TNF-α percentage % |
|---|---|---|---|---|---|
| **49** | 0.6 | 79.18 | **108** | 0.6 | 103.68 |
| **55** | 0.6 | 86.42 | **112** | 0.6 | 97.82 |
| **58** | 0.6 | 80.45 | **119** | 0.6 | 104.77 |
| **60** | 0.6 | 85.83 | **121** | 0.6 | 119.21 |
| **61** | 0.6 | 71.23 | **122** | 0.6 | 98.89 |

(2) IL-1β

The results of the decreased percentage of IL-1β in the synovial tissue of the knee joint of rats in each group were shown in Table 9. Colchicine low-dose and high-dose groups reduced IL-1β in synovial tissue of rat knee joints after modeling. All compound groups could reduce the IL-1β lelvel in the synovial tissue of the knee joint of rats after modeling, and the inhibition of IL-1β lelvel in the synovial tissue of the knee joint of rats by most compounds was significantly better than that of colchicine.

**Table 9. Effect of compounds on IL-1β in synovial tissue of knee joints of gouty arthritis rats**

| Compound name or No. | Dose (mg/kg) | IL-1β percentage decrease % | Compound name or No. | Dose (mg/kg) | Decreased IL-1β percentage % |
|---|---|---|---|---|---|
| **Colchicine low dose group** | 0.6 | 74.20 | 69 | 0.6 | 109.93 |
| **colchicine high dose group** | 1.2 | 63.07 | 81 | 0.6 | 86.60 |
| **19** | 0.6 | 81.53 | 95 | 0.6 | 40.40 |
| **34** | 0.6 | 93.07 | **97** | 0.6 | 20.24 |
| **38** | 0.6 | 102.09 | **98** | 0.6 | 64.98 |
| **44** | 0.6 | 104.38 | **99** | 0.6 | 66.42 |
| **48** | 0.6 | 82.15 | **100** | 0.6 | 65.22 |
| **49** | 0.6 | 69.07 | **108** | 0.6 | 92.74 |
| **55** | 0.6 | 132.97 | **112** | 0.6 | 91.96 |
| **58** | 0.6 | 87.55 | **119** | 0.6 | 85.49 |
| **60** | 0.6 | 76.08 | **121** | 0.6 | 67.70 |
| **61** | 0.6 | 97.21 | **122** | 0.6 | 86.87 |

(3) IL-6

The results of the decreased percentage of IL-6 in the synovial tissue of the knee joint of rats in each group were shown in Table 10. Colchicine low-dose and high-dose groups could reduce the IL-6 lelvel of knee synovial tissue after modeling. All compound groups could reduce the IL-6 lelvel in the synovial tissue of the knee joint of rats after modeling, and the inhibition of IL-6 lelvel in the synovial tissue of the knee joint of rats after modeling by most of the compounds was significantly better than that of colchicine.

**Table 10. Effect of compounds on IL-6 in synovial tissue of knee joints of gouty arthritis rats**

| Compound name or No. | Dose (mg/kg) | IL-6 percentage decrease % | Compound name or No. | Dose (mg/kg) | Decreased IL-6 percentage % |
|---|---|---|---|---|---|
| **Colchicine low dose group** | 0.6 | 83.54 | **69** | 0.6 | 83.05 |
| **Colchicine high dose group** | 1.2 | 96.32 | **81** | 0.6 | 70.64 |
| **19** | 0.6 | 98.08 | **95** | 0.6 | 109.36 |
| **34** | 0.6 | 115.12 | **97** | 0.6 | 97.81 |
| **38** | 0.6 | 68.98 | **98** | 0.6 | 167.41 |
| **44** | 0.6 | 19.72 | **99** | 0.6 | 79.57 |
| **48** | 0.6 | 109.78 | **100** | 0.6 | 168.80 |
| **49** | 0.6 | 78.58 | **108** | 0.6 | 207.01 |
| **55** | 0.6 | 128.11 | **112** | 0.6 | 165.11 |
| 58 | 0.6 | 86.72 | **119** | 0.6 | 169.36 |
| 60 | 0.6 | 59.51 | **121** | 0.6 | 231.24 |
| **61** | 0.6 | 52.45 | **122** | 0.6 | 220.07 |

**Example 66: Acute toxicity test of compounds 34, 48, 55 and 58 in mice by single administration**

1. Experimental materials

Compounds **34, 48, 55** and **58** were ground with 0.5% CMC-Na before administration, and prepared into a suspension of the corresponding concentration for gavage, with an administration volume of 0.1 mL/10 g. Colchicine, purchased from Sun Chemical Technology (Shanghai) Co., Ltd. with batch number 49ETRRAS, was ground using 0.5% CMC-Na before use, and formulated into appropriate concentration of suspension for gavage with an administration volume of 0.1 mL/10 g.

2. Test animals and feeding conditions

ICR mice, SPF grade, weighing 18-20 g, 6-8 weeks old, were provided by Nantong University. The Laboratory Animal Production License was SCXK (Su) 2016-0010 and Laboratory Animal Use License was SYXK (Su) 2017-0035.

2. Experimental methods and results

ICR mice were randomly divided into 18 groups, i.e., normal group, colchicine group (50 mg/kg), 50 mg/kg dose group, 100 mg/kg dose group, 400 mg/kg dose group and 600 mg/kg dose group of compound **34,** 50 mg/kg dose group, 100 mg/kg dose group, 400 mg/kg dose group and 600 mg/kg dose group of compound **48,** 50 mg/kg dose group, 100 mg/kg dose group, 400 mg/kg dose group and 600 mg/kg dose group of Compound **55,** 50 mg/kg dose group, 100 mg/kg dose group, 400 mg/kg dose group and 600 mg/kg dose group of Compound **58,** with three females and three males in each group. After overnight fasting, the drug was administered once by gavage in a volume of 0.1 mL/10 g. The normal group was given the corresponding volume of 0.5% CMC-Na solution according to body weight. The state of the animal was observed after administration, the animals were weighed daily and the number of deaths was recorded for 14 consecutive days.

The dose and mortality rate of mice in each group were shown in Table 11. No immediate toxic reaction was observed in each compound group, no delayed toxic reaction was observed from 24 hours to 14 days, the animals were in good condition with weight gain and all the mice survived. The maximum tolerated dose (MTD) for acute toxicity testing in mice was greater than 600 mg/kg for compounds **34, 48, 55** and **58,** whereas the MTD for colchicine was less than 50 mg/kg.

**Table 11: Doses administered to ICR mice and mortality rate**

| Group | Dose (mg/kg) | Animal No. | Mortality rate |
|---|---|---|---|
| normal group | / | 6 | 0/6 |
| colchicine group | 50 | 6 | 3/6 |
| compound 34 | 50 | 6 | 0/6 |
| | 100 | 6 | 0/6 |
| | 400 | 6 | 0/6 |
| | 600 | 6 | 0/6 |
| compound 48 | 50 | 6 | 0/6 |
| | 100 | 6 | 0/6 |
| | 400 | 6 | 0/6 |
| | 600 | 6 | 0/6 |
| compound 55 | 50 | 6 | 0/6 |
| | 100 | 6 | 0/6 |
| | 400 | 6 | 0/6 |
| | 600 | 6 | 0/6 |
| compound 58 | 50 | 6 | 0/6 |
| | 100 | 6 | 0/6 |
| | 400 | 6 | 0/6 |
| | 600 | 6 | 0/6 |

**Example 67: In vivo pharmacokinetics of compounds 48, 55, 56, 58, 69,124,125 and 132 in SD rats**

1. Experimental materials

(1) Test drugs

Compound stock solution preparation: an appropriate amount of solid powder of the compounds were weighed separately, a certain amount of DMSO was added and 20 mg/mL of stock solution was obtained under vortex ultrasound.

Preparation of test compound for gavage: an appropriate amount of compound stock solution was taken by pipette, a certain amount of Solutol HS15 solution was added, the mixture was vortexed for 1 minute, then a certain amount of saline was added, and 1 mg/mL of solution was obtained after mixing well.

Preparation of test compounds for intravenous injection: an appropriate amount of compound stock solution was taken by pipette separately, a certain amount of Solutol HS15 solution was added, the mixture was vortexed for 1 minute, then a certain amount of physiological saline was added and a solution of 0.5 mg/mL was obtained after mixing well.

(2) Experimental animals

SD rats, male, SPF grade, 6-8 weeks old, were purchased from JH Laboratory Animal Co. LTD. The license No. was SCXK (SH) 2017-0012 and the Certificate of Conformity No. was 20170012022077.

2. Experimental methods

(1) Dose and mode of administration

The animals were fasted overnight before administration by gavage, and food was given 4 hours after administration, during which they were free to drink. Two groups were set for each test compound, intravenous injection group and oral administration group, and the dose and mode of administration were shown in Table 12 below.

**Table 12: Dose and mode of administration of compounds to SD rats**

| Group | Dose (mg/kg) | Volume (mL/kg) | Concentration (mg/mL) | Mode of administration |
|---|---|---|---|---|
| intravenous injection group | 1 | 2 | 0.5 | intravenous |
| oral administration group | 10 | 10 | 1 | oral |

(2) Experimental operations

Blood samples (150 μL/sample) were collected from the jugular vein of SD rats before and 5 min (intravenous injection group only), 15 min, 30 min, 1 h, 2 h, 4 h, 6 h, 8 h, and 24 h after administration, respectively, and were placed in centrifuge tubes containing the anticoagulant sodium heparin. The blood samples were centrifuged at 4°C for 5 min at 2,000 g to separate the plasma. The plasma samples were analyzed by LC/MS/MS to detect the concentration of each test compound in the plasma samples.

(3) Pharmacokinetic analysis

The parameters related to the non-atrial model were obtained by calculations with WinNonlin® Professional software.

$$\text{Bioavailability F\%} = (\text{Dose}_{(IV)} \times \text{AUC}_{(0\text{-}t)(PO)}) / (\text{Dose}_{(PO)} \times \text{AUC}_{(0\text{-}t)(IV)}) \times 100\%$$

3. Experimental results

The pharmacokinetic parameters of SD rats obtained for each of the test compounds based on the above method were shown in Table 13. The compounds of the present invention have better pharmacokinetic parameters, higher bioavailability than those of colchicine.

**Table 13: Pharmacokinetic parameters of SD rats given each compound orally or intravenously**

| Gavage -10 mg/kg | | | | | | |
|---|---|---|---|---|---|---|
| **Compound No.** | **$t_{1/2}$ (h)** | **Tmax (h)** | **Cmax (ng/mL)** | **$AUC_{INF}$ (hr*ng/mL)** | **F* (%)** | **$MRT_{INF}$ (h)** |
| **colchicine** | / | 0.417 | 175 | 735 | 8.73 | 25.7 |
| **48** | 1.61 | 0.25 | 1933 | 3305 | 57.0 | 1.83 |
| **55** | 2.02 | 0.333 | 2043 | 6257 | 115 | 2.72 |
| **56** | 1.27 | 0.417 | 1640 | 3716 | 62.3 | 2.20 |
| **58** | 2.22 | 2.67 | 2043 | 15113 | 109 | 4.58 |
| **122** | 1.79 | 0.250 | 1153 | 3129 | 50.1 | 2.47 |
| **124** | 1.38 | 0.333 | 2257 | 3379 | 44.7 | 1.71 |
| **125** | 3.33 | 0.25 | 4153 | 5459 | 93.0 | 1.8 |
| **132** | 1.39 | 0.25 | 1783 | 3335 | 47.5 | 2.10 |

| Intravenous injection - 1 mg/kg | | | | | |
|---|---|---|---|---|---|
| **Compound No.** | **$t_{1/2}$ (h)** | **$AUC_{INF}$ (hr*ng/mL)** | **Vss (L/kg)** | **CL (L/hr/kg)** | **$MRT_{INF}$ (h)** |
| **colchicine** | / | 567 | 17.6 | 1.91 | 11.0 |
| **48** | 0.536 | 580 | 1.11 | 1.75 | 0.641 |
| **55** | 0.6 | 543 | 1.10 | 1.87 | 0.592 |
| **56** | 0.590 | 597 | 0.977 | 1.72 | 0.582 |

(continued)

| Intravenous injection - 1 mg/kg | | | | | |
|---|---|---|---|---|---|
| **Compound No.** | **$t_{1/2}$ (h)** | **$AUC_{INF}$ (hr*ng/mL)** | **Vss (L/kg)** | **CL (L/hr/kg)** | **$MRT_{INF}$ (h)** |
| **58** | 1.92 | 1388 | 1.44 | 0.728 | 2.04 |
| **122** | 1.57 | 624 | 1.99 | 1.63 | 1.34 |
| **124** | 0.644 | 756 | 0.864 | 1.34 | 0.652 |
| **125** | 1.19 | 587 | 1.15 | 1.71 | 0.675 |
| **132** | 2.12 | 702 | 1.39 | 1.46 | 1.06 |
| *: Calculated by $AUC_{INF}$ | | | | | |

The foregoing are only better specific embodiments of the present invention, but the scope of protection of the present invention is not limited thereto, and any equivalent substitution or change made by a person skilled in the art familiar with the technical field of the present invention in accordance with the technical solution of the invention and its inventive concept shall be covered by the scope of protection of the invention.

## Claims

**1.** Compounds, isomers or pharmaceutically acceptable salts of the structure shown in formula (1) or formula (II),

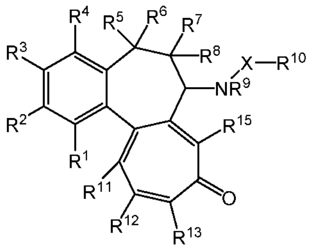

( I )

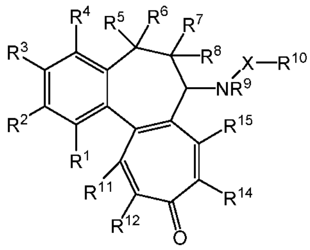

( II )

Wherein,

$R^1$, $R^2$, $R^3$, or $R^4$ are independently hydrogen, deuterium, halogen, cyano, $C_{1-3}$ alkoxy, hydroxyl, aldehyde group, carboxyl, $C_{2-6}$ ester, amide, substituted amide, $C_{1-3}$ alkyl, substituted $C_{1-3}$ alkoxy, or substituted $C_{1-3}$ alkyl, respectively, the said substituent is one or more selected from the group consisting of deuterium, halogen, hydroxyl, carboxyl, $C_{1-3}$ alkyl or cyano;

Alternatively, two adjacent groups in $R^1$, $R^2$, $R^3$ and $R^4$ are joined together to form -O-$R^{17}$-O- group, wherein $R^{17}$ is C1-6 alkyl;

$R^5$, $R^6$, $R^7$ or $R^8$ are independently hydrogen, deuterium, halogen, hydroxyl, cyano, carboxyl, $C_{2-6}$ ester group or substituted or non-substituted groups of amino, amide, $C_{1-3}$ alkoxy or $C_{1-3}$ alkyl, the said substituent is one or more selected from the group consisting of deuterium, halogen, cyano, $C_{1-3}$ alkyl or $C_{1-3}$ haloalkyl;

$R^9$ is hydrogen, $C_{1-4}$ alkyl, or $C_{1-4}$ substituted alkyl, the said substituent is one or more selected from the group consisting of deuterium, hydroxyl, $C_{1-2}$ alkoxy, or cyano;

X is -C(=O)-, -S(=O)$_2$-, -S(=O)-, -C(=NH)- or substituted -C(=NH)-, the substituent is selected from CN and $SO_2NH_2$;

$R^{10}$ is $C_{1-4}$ alkyl, substituted $C_{1-4}$ alkyl, $C_{3-6}$ cycloalkyl, substituted $C_{3-6}$ cycloalkyl, amino, $C_{1-4}$ alkylamino, substituted $C_{1-4}$ alkylamino, $C_{3-6}$ cycloalkylamino, $C_{1-4}$ alkoxy, substituted $C_{1-4}$ alkoxy, or vinyl; or $R^9$ is attached to $R^{10}$ so that $R^9$-N-X-$R^{10}$ forms a ring group together; the said substituent is selected from one or more of deuterium, halogen and cyano;

R" is hydrogen, deuterium, halogen, cyano, $C_{1-2}$ alkyl, $C_{1-2}$ alkoxy, carboxyl, or $C_{1-3}$ alkyl substituted amide.

$R^{12}$ is a hydrogen, deuterium, halogen, hydroxyl, cyano, carboxyl, or substituted or non-substituted group of the following: $C_{1-4}$ alkyl, $C_{3-6}$ cycloalkyl, $C_{1-4}$ alkoxy, morpholinyl, aminocarbonyl, $C_{1-4}$ alkylamino carbonyl, or

$C_{1-4}$ alkoxy carbonyl; the substituent is selected from one or more of deuterium, halogen or cyano; and

(a) when $R^{12}$ is hydrogen, $R^{13}$ is cyano, aldehyde group, carboxyl, borono, substituted $C_{1-4}$ alkoxy, or substituted or non-substituted group of $C_{1-4}$ alkoxy carbonyl, amino carbonyl, phenyl amino carbonyl, pyridinyl amino carbonyl, $C_{1-4}$ alkylamino carbonyl, $C_{1-4}$ alkoxy carbonyl, $C_{1-4}$ alkylamino carbonyl amino, $C_{2-6}$ heterocycloalkyl carbonyl, $C_{3-6}$ heterocycloalkyl ketone group, $C_{1-2}$ alkyl carbonyl, sulfonamido, sulfonyloxy, amino sulfonyl, amino sulfonyloxy, $C_{1-4}$ alkyl sulfonamido, $C_{1-4}$ alkyl amino sulfonyl, $C_{1-4}$ alkyl amino sulfonamido, $C_{1-4}$ alkyl sulfonyloxy, $C_{1-4}$ alkyl amino sulfonyloxy, $C_{1-4}$ alkyl amino carbonyloxy, tetrazolyl, triazolyl, imidazolyl, morpholinyl, $C_{1-4}$ alkyl thio, $C_{3-6}$ cycloalkoxy or $C_{3-6}$ heterocycloalkyloxy, the substituent is selected from one or more of deuterium, halogen, cyano, hydroxyl, phenyl, pyridyl, $C_{1-3}$ alkyl, $C_{1-3}$ deuteroalkyl or $C_{1-3}$ haloalkyl;

(b) when $R^{12}$ is not hydrogen, $R^{13}$ is cyano, aldehyde group, carboxyl, borono, or substituted or non-substituted group of $C_{1-4}$ alkoxy carbonyl, amino carbonyl, phenyl amino carbonyl, pyridinyl amino carbonyl, $C_{1-4}$ alkylamino carbonyl, $C_{1-4}$ alkoxy carbonyl amino, $C_{1-4}$ alkylamino carbonyl amino, $C_{2-6}$ heterocycloalkyl carbonyl, $C_{3-6}$ heterocycloalkyl ketone group, $C_{1-4}$ alkyl carbonyl, sulfonamido, sulfonyloxy, sulfamoyl, sulfamoyloxy, $C_{1-4}$ alkyl sulfonamido, $C_{1-4}$ alkyl amino sulfonyl, $C_{1-4}$ alkyl amino sulfonamido, $C_{1-4}$ alkyl sulfonyloxy, $C_{1-4}$ alkyl amino sulfonyloxy, $C_{1-4}$ alkyl amino carbonyloxy, heterocyclic group, $C_{1-4}$ alkyl amino, $C_{3-6}$ cycloalkyl amino, $C_{3-6}$ heterocycloalkyloxy, $C_{3-6}$ heterocycloalkyl amino, $C_{1-4}$ alkyl carbonyl amino, $C_{1-4}$ alkoxy, $C_{1-4}$ alkylthio, $C_{3-6}$ cycloalkoxy, $C_{3-6}$ heterocyclo alkyl or $C_{1-4}$ alkyl, the substituent is selected from one or more of deuterium, halogen, cyano, hydroxyl, phenyl, pyridyl, $C_{1-3}$ alkyl, $C_{1-3}$ deuteroalkyl and $C_{1-3}$ haloalkyl;

$R^{14}$ is a cyano, carboxyl, or substituted or non-substituted group of $C_{1-4}$ alkoxy carbonyl, amino carbonyl, $C_{1-4}$ alkyl amino carbonyl, $C_{1-4}$ alkoxy carbonyl amino, $C_{1-4}$ alkyl amino carbonyl amino, $C_{2-6}$ heterocycloalkyl carbonyl, sulfamoyl, $C_{1-4}$ alkylaminosulfonyl, heterocyclic, $C_{3-6}$ heterocycloalkyl, $C_{3-6}$ heterocycloalkyloxy, $C_{1-4}$ alkoxy, $C_{3-6}$ cycloalkoxy, $C_{3-6}$ heterocycloalkoxy or $C_{1-4}$ alkyl, the substituent is selected from one or more of deuterium, halogen, cyano, hydroxyl, $C_{1-3}$ alkyl and $C_{1-3}$ haloalkyl;
$R^{15}$ is hydrogen, deuterium, halogen, cyano, $C_{1-2}$ alkyl, $C_{1-2}$ alkoxy, carboxyl, or $C_{1-3}$ alkyl substituted amide.
Provided that the following conditions are excluded:

(i) In formula (1), when $R^1$-$R^3$ is methoxy, $R^4$-$R^9$ is hydrogen, $R^{10}$ is methyl, $R^{11}$-$R^{12}$ is hydrogen, and $R^{15}$ is hydrogen, $R^{13}$ is selected from the following groups: $C_{1-4}$ alkoxy, $C_{1-4}$ alkylthio, deutero $C_{1-4}$ alkylthio, $C_{1-4}$-alkoxy carbonyl amino, butyl carbonyl, or morpholinyl;
(ii) In formula (1), $R^1$-$R^3$ is methoxy, $R^4$-$R^9$ is hydrogen, $R^{10}$ is deuteromethyl, R" is hydrogen, $R^{12}$ is deuterium, $R^{13}$ is methoxy, and $R^{15}$ is deuterium;
(iii) In formula (1), $R^1$-$R^3$ is methoxy, $R^4$-$R^9$ is hydrogen, $R^{10}$ is methyl, $R^{11}$ is hydrogen, $R^{12}$ is bromine, $R^{13}$ is methoxy, and $R^{15}$ is bromine.

2. The compounds, isomers or pharmaceutically acceptable salts according to claim 1, wherein the compounds are selected from the compoundes in formula (III) or formula (IV),

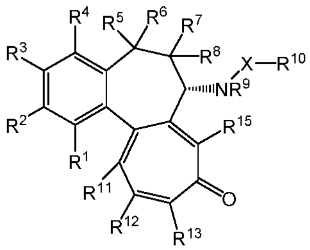
( III )

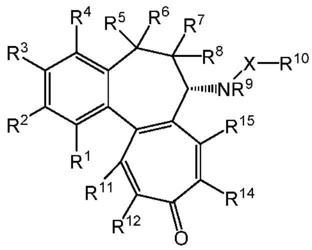
( IV )

In the above formulae,

$R^1$, $R^2$, $R^3$, or $R^4$ are independently hydrogen, deuterium, halogen, cyano, $C_{1-3}$ alkoxy, aldehyde group, $C_{1-3}$ alkyl, substituted $C_{1-3}$ alkoxy, or substituted $C_{1-3}$ alkyl, respectively, wherein the substituent is one or more selected from the group consisting of deuterium, halogen, $C_{1-3}$ alkyl, and cyano;
Alternatively, $R^1$ and $R^2$, $R^2$ and $R^3$, or $R^3$ and $R^4$ are joined together to form the -O-$R^{17}$-O- group, wherein $R^{17}$

is $C_{1-3}$ alkyl;
$R^5$, $R^6$, $R^7$, or $R^8$ is independently hydrogen, deuterium, halogen, cyano, carboxyl, hydroxyl, or amino, respectively;
$R^9$ is hydrogen, $C_{1-4}$ alkyl, or $C_{1-4}$ substituted alkyl, wherein the substituent is one or more selected from the group consisting of deuterium, hydroxyl, $C_{1-2}$ alkoxy, and cyano;
X is -C(=O)- or -S(=O)$_2$- group;
$R^{10}$ is a $C_{1-4}$ alkyl, substituted $C_{1-4}$ alkyl, $C_{3-6}$ cycloalkyl, substituted $C_{3-6}$ cycloalkyl, amino, $C_{1-4}$-alkyl amino, substituted $C_{1-4}$-alkyl amino, $C_{3-6}$ cycloalkyl amino, $C_{1-4}$ alkoxy, substituted $C_{1-4}$ alkoxy, or vinyl, or $R^9$-N-X-$R^{10}$ forms

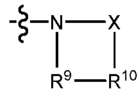,

the substituent is selected from one or more of deuterium and cyano;
R" is hydrogen, deuterium, halogen, cyano, $C_{1-2}$ alkyl, $C_{1-2}$ alkoxy, carboxyl, or $C_{1-3}$ alkyl substituted amide.
$R^{12}$ is hydrogen, deuterium, halogen, hydroxyl, cyano, or substituted or non-substituted group of the following: $C_{1-4}$ alkyl, $C_{3-6}$ cycloalkyl, $C_{1-4}$ alkoxy, morpholinyl, $C_{1-4}$ alkylamino carbonyl or $C_{1-4}$-alkoxy carbonyl; the substituent is selected from one or more of deuterium, halogen and cyano; and

(a) when $R^{12}$ is hydrogen, $R^{13}$ is a cyano, aldehyde group, carboxyl, borono, substituted $C_{1-4}$ alkoxy, or substituted or non-substituted group of $C_{1-4}$ alkoxy carbonyl, amino carbonyl, phenyl amino carbonyl, pyridinyl amino carbonyl, $C_{1-4}$ alkyl amino carbonyl, $C_{1-4}$ alkoxy carbonyl amino, $C_{1-4}$ alkyl amino carbonyl amino, pyrrolidyl carbonyl, piperazinyl carbonyl, morpholinyl carbonyl, pyrrolidonyl, oxazolidinonyl, morpholinonyl, $C_{1-2}$ alkyl carbonyl, sulfonamido, sulfonyloxy, amino sulfonyl, amino sulfonyloxy, $C_{1-4}$ alkyl sulfonamido, $C_{1-4}$ alkyl amino sulfonyl, $C_{1-4}$ alkyl amino sulfonamido, $C_{1-4}$ alkyl amino sulfonyloxy, $C_{1-4}$ alkyl amino carbonyloxy, tetrazolyl, triazolyl, imidazolyl, morpholinyl, $C_{1-4}$ alkyl thio, $C_{3-6}$ cycloalkyloxy or tetrahydrofuranyloxy, the substituent is one or more selected from the group consisting of deuterium, halogen, cyano, hydroxyl, phenyl, $C_{1-3}$ alkyl, $C_{1-3}$ deuteroalkyl or $C_{1-3}$ haloalkyl.
(b) when $R^{12}$ is not hydrogen, $R^{13}$ is cyano, aldehyde group, carboxyl, borono, or substituted or non-substituted group of $C_{1-4}$ alkoxy carbonyl, amino carbonyl, phenyl amino carbonyl, pyridinyl amino carbonyl, $C_{1-4}$ alkyl amino carbonyl, $C_{1-4}$ alkoxy carbonyl amino, $C_{1-4}$ alkyl amino carbonyl amino, $C_{2-6}$ heterocycloalkyl carbonyl, $C_{3-6}$ heterocycloalkylketone group, $C_{1-4}$ alkyl carbonyl, sulfonamido, sulfonyloxy, amino sulfonyl, amino sulfonyloxy, $C_{1-4}$ alkyl sulfonamido, $C_{1-4}$ alkyl amino sulfonyl, $C_{1-4}$ alkyl amino sulfonamido, $C_{1-4}$ alkyl sulfonyloxy, $C_{1-4}$ alkyl amino sulfonyloxy, $C_{1-4}$ alkyl amino carbonyloxy, heterocyclic, $C_{1-4}$ alkyl amino, $C_{3-6}$ cycloalkyl amino, tetrahydrofuranyloxy, tetrahydrofuranyl amino, $C_{1-4}$ alkyl carbonyl amino, $C_{1-4}$ alkoxy, $C_{1-4}$ alkylthio, $C_{3-6}$ cycloalkoxy, $C_{3-6}$ heterocycloalkyl or $C_{1-4}$ alkyl, wherein the substituent is one or more selected from the group consisting of deuterium, halogen, cyano, hydroxyl, phenyl, $C_{1-3}$ alkyl, $C_{1-3}$ deuteroalkyl or $C_{1-3}$ haloalkyl;

$R^{14}$ is a cyano, carboxyl, or substituted or non-substituted group of $C_{1-4}$ alkoxy carbonyl, aminocarbonyl, $C_{1-4}$ alkyl amino carbonyl, $C_{1-4}$ alkoxy carbonyl amino, $C_{1-4}$ alkyl amino carbonyl amino, *N*-pyrrolidinyl carbonyl, amino sulfonyl, $C_{1-4}$ alkyl amino sulfonyl, heterocyclic group, tetrahydrofuranyloxy, $C_{1-4}$ alkoxy, $C_{3-6}$ cycloalkyloxy, or $C_{1-4}$ alkyl, wherein the substituent is one or more selected from the group consisting of deuterium, halogen, cyano, hydroxyl, $C_{1-3}$ alkyl or $C_{1-3}$ haloalkyl.
$R^{15}$ is hydrogen, deuterium, halogen, cyano, $C_{1-2}$ alkyl, $C_{1-2}$ alkoxy, carboxyl, or $C_{1-3}$ alkyl substituted amide.

**3.** The compounds, isomers or pharmaceutically acceptable salts according to claim 2, wherein the compounds are selected from the structure shown in formula (V) or formula (VI),

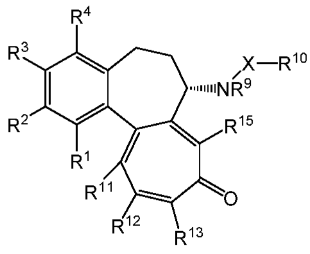

( V )

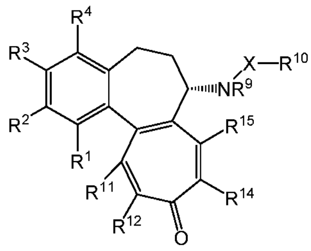

( VI )

in the formulae,

$R^1$, $R^2$ or $R^3$ is independently $C_{1-3}$ alkoxy respectively; or, $R^1$ and $R^2$ or $R^2$ and $R^3$ are joined together to form -O-$R^{17}$-O- group, wherein $R^{17}$ is $C_{1-2}$ alkyl;
$R^4$ is hydrogen, deuterium, halogen, or cyano.
$R^9$ is hydrogen or $C_{1-3}$ alkyl;
X is -C(=O)- or -S(=O)$_2$- group;
$R^{10}$ is $C_{1-3}$ alkyl, substituted $C_{1-3}$ alkyl, $C_{3-6}$ cycloalkyl, amino, $C_{1-4}$ alkyl amino, $C_{1-3}$ alkoxy or vinyl, or $R^9$ forms

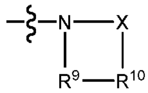

group with N-X-$R^{10}$; wherein the substituent is selected from one or more of deuterium and cyano;
$R^{11}$ is hydrogen, deuterium or halogen;
$R^{12}$ is hydrogen, deuterium, halogen, cyano, or substituted or non-substituted group of the following: $C_{1-3}$ alkyl, C3-5 cycloalkyl, $C_{1-3}$ alkoxy, morpholinyl, $C_{1-4}$ alkylamino carbonyl or $C_{1-4}$-alkoxy carbonyl; wherein the substituent is selected from one or more of deuterium and halogen; and

(a) when $R^{12}$ is hydrogen, $R^{13}$ is a cyano, aldehyde group, carboxyl, borono, substituted $C_{1-4}$ alkoxy, or substituted or non-substituted group of $C_{1-4}$ alkoxy carbonyl, amino carbonyl, phenyl amino carbonyl, pyridinyl amino carbonyl, $C_{1-4}$ alkyl amino carbonyl, $C_{1-4}$ alkoxy carbonyl amino, $C_{1-4}$ alkyl amino carbonyl amino, N-pyrrolidyl carbonyl, piperazinyl carbonyl, morpholinyl carbonyl, pyrrolidonyl, oxazolidinonyl, 3-morpholinonyl, $C_{1-2}$ alkyl carbonyl, sulfonamido, sulfonyloxy, amino sulfonyl, amino sulfonyloxy, $C_{1-4}$ alkyl sulfonamido, $C_{1-4}$ alkyl amino sulfonyl, $C_{1-4}$ alkyl amino sulfonamido, $C_{1-4}$ alkyl amino sulfonyloxy, $C_{1-4}$ alkyl amino carbonyloxy, tetrazolyl, triazolyl, imidazolyl, pyrazolyl, morpholinyl, $C_{1-4}$ alkyl thio, $C_{3-6}$ cycloalkyloxy or tetrahydrofuranyloxy, the substituent is one or more selected from the group consisting of deuterium, halogen, cyano, hydroxyl, phenyl, pyridyl, $C_{1-3}$ alkyl, $C_{1-3}$ deuteroalkyl or $C_{1-3}$ haloalkyl.
(b) when $R^{12}$ is not hydrogen, $R^{13}$ is cyano, aldehyde group, carboxyl, borono, or substituted or non-substituted group of $C_{1-4}$ alkoxy carbonyl, amino carbonyl, phenyl amino carbonyl, pyridinyl amino carbonyl, $C_{1-4}$ alkyl amino carbonyl, $C_{1-4}$ alkoxy carbonyl amino, $C_{1-4}$ alkyl amino carbonyl amino, pyrrolidonyl, 2-oxazolidinonyl, 3-morpholinonyl, pyrrolidinyl carbonyl, methylpiperazinyl carbonyl, morpholinyl carbonyl, $C_{1-4}$ alkyl carbonyl, sulfonamido, sulfonyloxy, amino sulfonyl, amino sulfonyloxy, $C_{1-4}$ alkyl sulfonamido, $C_{1-4}$ alkyl amino sulfonyl, $C_{1-4}$ alkyl amino sulfonamido, $C_{1-4}$ alkyl sulfonyloxy, $C_{1-4}$ alkyl amino sulfonyloxy, $C_{1-4}$ alkyl amino carbonyloxy, heterocyclic, $C_{1-4}$ alkyl amino, $C_{3-6}$ cycloalkyl amino, tetrahydrofuranyloxy, tetrahydrofuranyl amino, $C_{1-4}$ alkyl carbonyl amino, $C_{1-4}$ alkoxy, $C_{1-4}$ alkylthio, $C_{3-6}$ cycloalkoxy, $C_{3-6}$ heterocycloalkyl or $C_{1-4}$ alkyl, wherein the substituent is one or more selected from the group consisting of deuterium, halogen, cyano, hydroxyl, phenyl, pyridyl, $C_{1-3}$ alkyl, $C_{1-3}$ deuteroalkyl or $C_{1-3}$ haloalkyl;

$R^{14}$ is a cyano, carboxyl, or substituted or non-substituted group of $C_{1-4}$ alkoxy carbonyl, aminocarbonyl, $C_{1-4}$ alkyl amino carbonyl, $C_{1-4}$ alkoxy carbonyl amino, $C_{1-4}$ alkyl amino carbonyl amino, *N*-pyrrolidinyl carbonyl, amino sulfonyl, $C_{1-4}$ alkyl amino sulfonyl, pyrrolidyl, oxazolidyl, isooxazolidyl, piperazinyl, morpholinyl, piperidyl, tetrahydrofuranyl, tetrahydrothiophenyl, hexahydropyrimidyl, tetraazolidyl, triazolidyl, tetrahydrofuranyloxy, $C_{1-4}$ alkoxy, $C_{3-6}$ cycloalkyloxy, or $C_{1-4}$ alkyl, wherein the substituent is one or more selected from the group consisting of deuterium, halogen, cyano, hydroxyl, $C_{1-3}$ alkyl or $C_{1-3}$ haloalkyl.
$R^{15}$ is hydrogen, deuterium, halogen, methyl, ethyl, halomethyl or haloethyl.

**4.** The compounds, isomers or pharmaceutically acceptable salts according to claim 1, 2 or 3, wherein,

$R^9$ is hydrogen, methyl or ethyl;
X is -C(=O)- or -S(=O)$_2$- group;
$R^{10}$ is methyl, ethyl, cyclopropyl, amino, methylamino, ethylamino, dimethylamino, diethylamino, methoxy, ethoxy, cyanomethyl, or cyanoethyl, or $R^9$ is joined together with N-X-$R^{10}$ to form

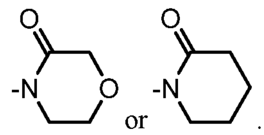

**5.** The compounds, isomers or pharmaceutically acceptable salts according to claim 1, 2 or 3, wherein, $R^{12}$ is hydrogen, deuterium, methyl, ethyl, halogen, cyano, methoxy, ethoxy, morpholinyl, aminocarbonyl, phenyl aminocarbonyl, methyl aminocarbonyl, methyl formate or ethyl formate; and

(a) when $R^{12}$ is hydrogen, $R^{13}$ is cyano, aldehyde group, carboxyl, borono, halomethoxyl, haloethoxyl, deutero methoxyl, deuteroethoxyl, methoxy carbonyl, ethoxy carbonyl, aminocarbonyl, phenyl aminocarbonyl, pyridinyl aminocarbonyl, methyl aminocarbonyl, deuteromethylaminocarbonyl, halomethyl aminocarbonyl, ethyl aminocarbonyl, dimethylaminocarbonyl, methoxy carbonyl amino, ethoxy carbonyl amino, (methoxy carbonyl)(methyl) amino, methyl amino carbonyl amino, ethyl amino carbonyl amino, dimethyl amino carbonyl, pyrrolidyl carbonyl, cyanopyrrolidyl carbonyl, piperazinyl carbonyl, methyl piperazinyl carbonyl, morpholinyl carbonyl, pyrrolidonyl, 2-oxazolidonyl, 3-morpholinonyl, methyl carbonyl, ethyl carbonyl, sulfonyl amino, sulfonyloxy, methyl sulfonyloxy, ethyl sulfonyloxy, hydroxyl sulfonyloxy, amino sulfonyl, amino sulfonyloxy, methyl sulfonyl amino, ethyl sulfonyl amino, methyl amino sulfonyl, methyl amino sulfonyl amino, methyl amino sulfonyloxy, N-ethyl methyl amino carbonyloxy, tetrazolyl, triazolyl, imidazolyl, pyrazolyl, morpholinyl, methylthio, ethylthio, halomethylthio, haloethylthio, deuteromethylthio, deuteroethylthio or tetrahydrofuranyloxy;
(b) when $R^{12}$ is not hydrogen, $R^{13}$ is cyano, aldehyde group, carboxyl, borono, methoxy carbonyl, ethoxy carbonyl, methylaminocarbonyl, dimethylaminocarbonyl, aminocarbonyl, phenylamino carbonyl, pyridyl amnio carbonyl, ethyl aminocarbonyl, halomethyl aminocarbonyl, deuteromethyl aminocarbonyl, (methoxycarbonyl)(methyl) amino, methyl aminocarbonyl amino, ethyl aminocarbonyl amino, dimethyl amino carbonyl amino, pyrrolidyl carbonyl, cyanopyrrolidyl carbonyl, piperazinyl carbonyl, methyl piperazinyl carbonyl, morpholinyl carbonyl, pyrrolidonyl, 2-oxazolidinonyl, 3-morpholinonyl, methyl carbonyl, ethyl carbonyl, sulfonyl amino, sulfonyloxy, hydroxyl sulfonyloxy, amino sulfonyl, amino sulfonyloxy, methyl sulfonyl amino, ethyl sulfonyl amino, methyl amino sulfonyl, ethyl amino sulfonyl, methyl amino sulfonyl amino, methyl amino sulfonyloxy, N-ethyl methyl amino carboxyloxy, tetrazolyl, triazolyl, methyl amino, ethyl amino, tetrahydrofuranyloxy, tetrahydrofuranyl amino, methyl carbonyl amino, methoxy, halomethoxy, deuteromethoxy, ethoxy, haloethoxy, deuteroethoxy, methylthio, halomethylthio, deuteromethylthio, ethylthio, haloethylthio, deuteroethylthio, tetrahydrofuranyl, piperazinyl, piperidyl, methylpiperidyl, imidazolyl, pyrazolyl, morpholinyl, pyrrolidinyl, tetrahydrocarbazolyl, morpholinyl, methyl, ethyl, propyl, hydroxymethyl, hydroxyethyl, halomethyl or haloethyl.

**6.** The compounds, isomers or pharmaceutically acceptable salts according to claim 1, 2 or 3, wherein,

R" is hydrogen or deuterium;
$R^{14}$ is methylaminocarbonyl, dimethylaminocarbonyl, cyano, methoxy carbonyl, ethoxy carbonyl, carboxyl, N-pyrrolidyl carbonyl, tetrahydrofuranyloxy, methoxy, ethoxy, cyclopropoxy, halomethoxy, haloethoxy, deuteromethoxy, deuteroethoxy, methyl, ethyl, propyl, hydroxymethyl, hydroxyethyl, tetraazolyl, triazolyl, morpholinyl, halomethyl or haloethyl;
$R^{15}$ is hydrogen, deuterium, methyl, ethyl, halomethyl, or haloethyl.

**7.** The compounds, isomers or pharmaceutically acceptable salts according to claim 1, wherein the compounds are selected from:

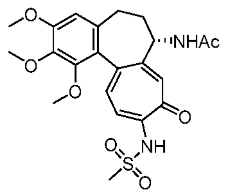 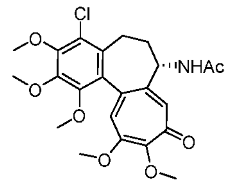 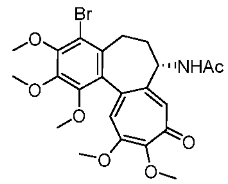 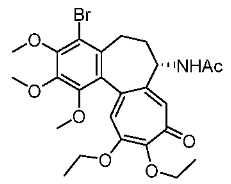

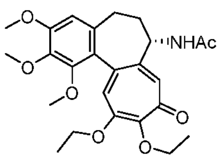 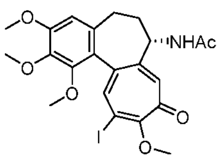 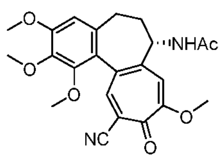 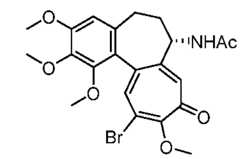

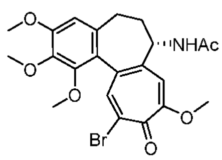 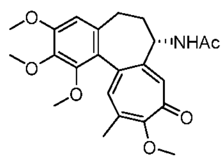 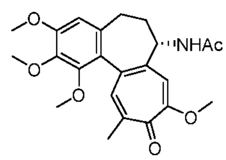 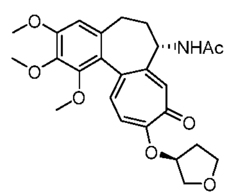

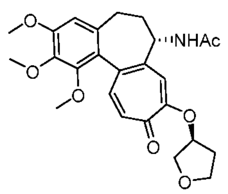 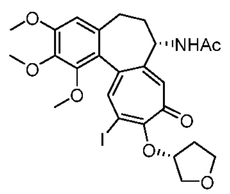 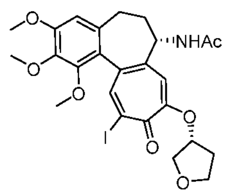 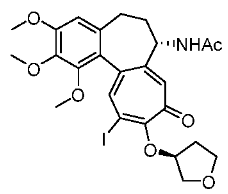

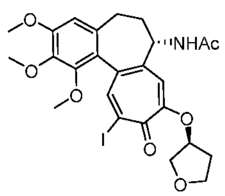 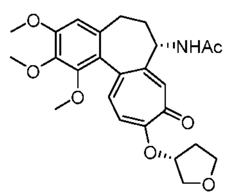 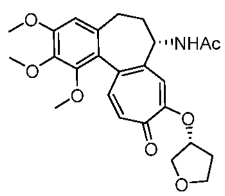 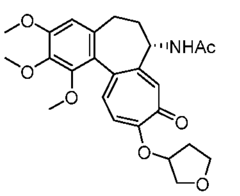

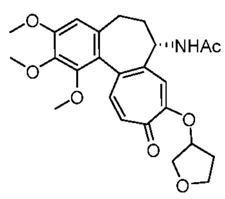 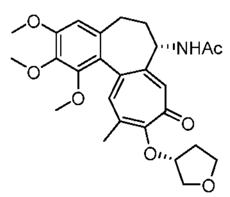 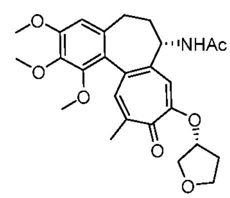 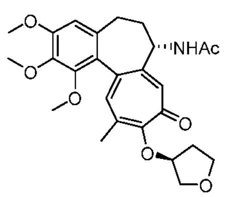

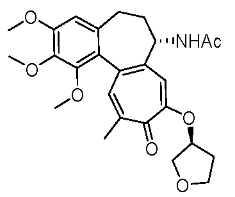 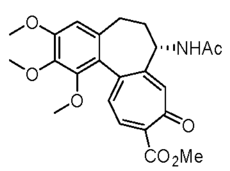 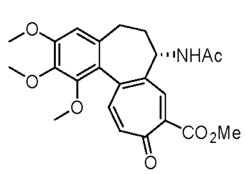 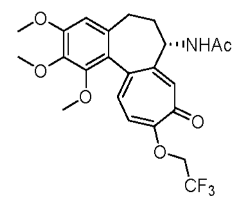

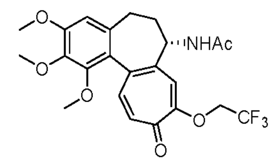
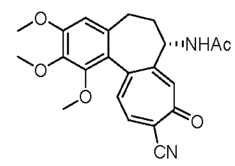
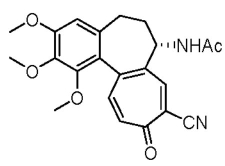
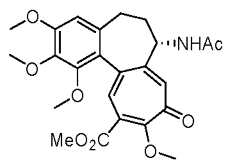

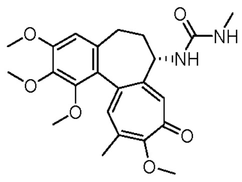
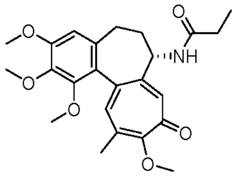
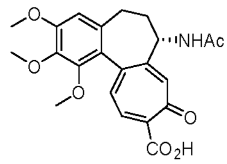
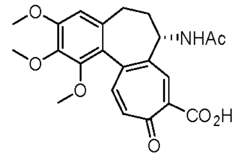

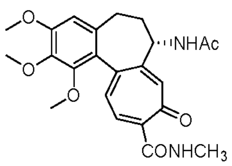
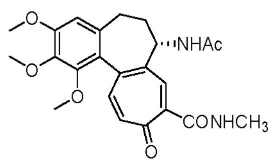
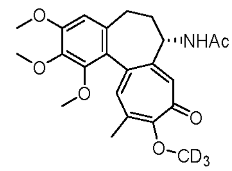
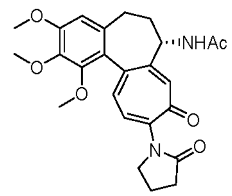

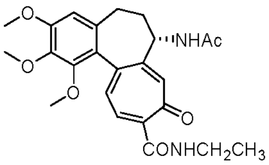
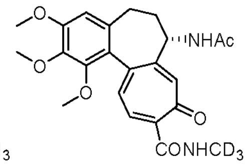
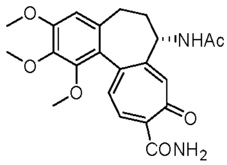
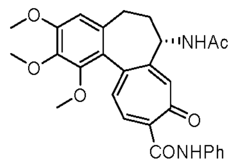

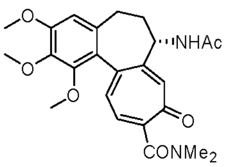
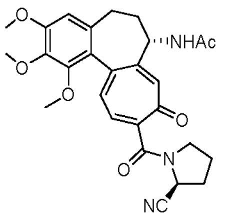
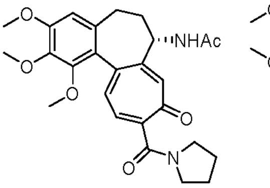
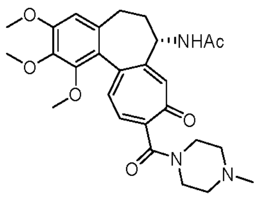

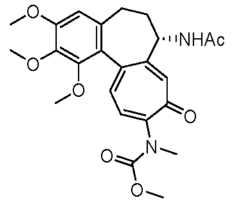
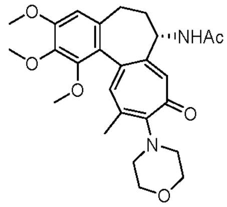
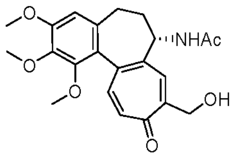
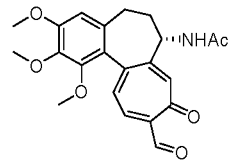

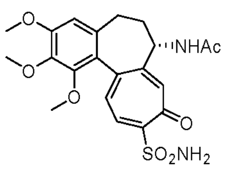
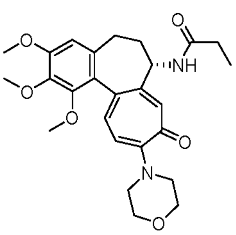
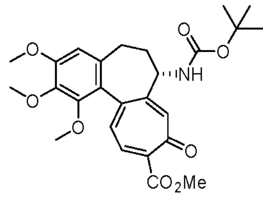
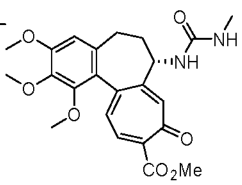

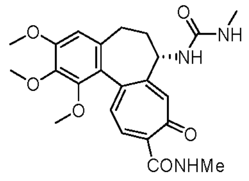
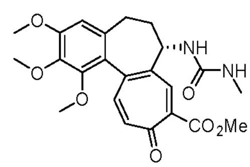
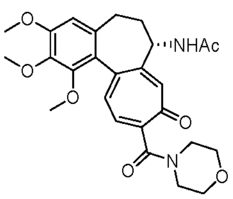
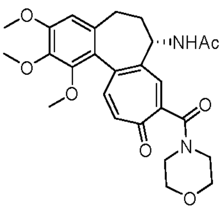

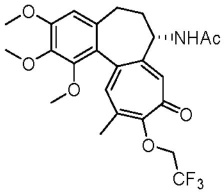
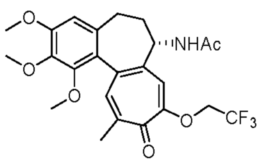
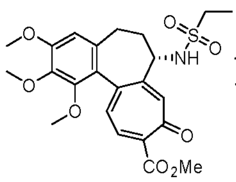
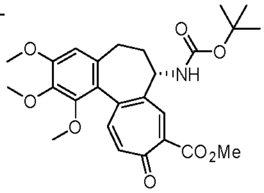

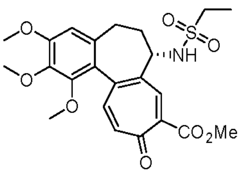
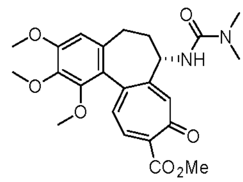
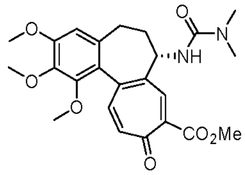
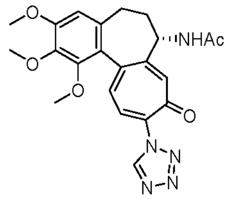

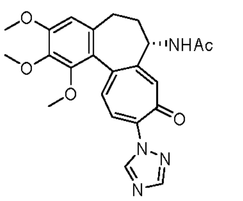
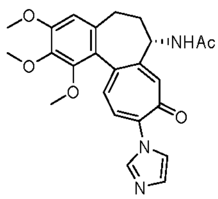
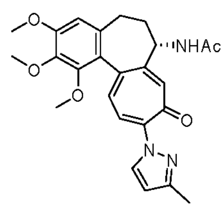
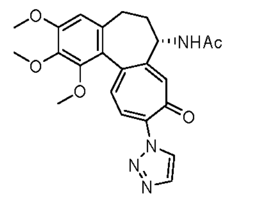

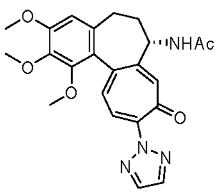
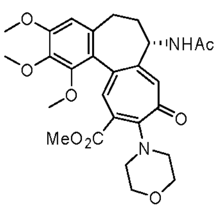
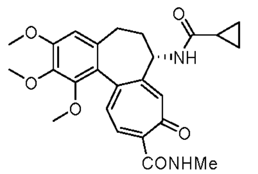
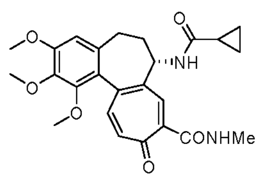

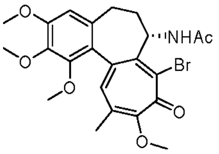
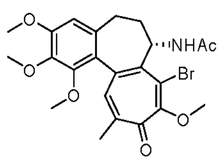
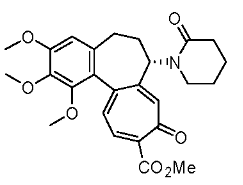
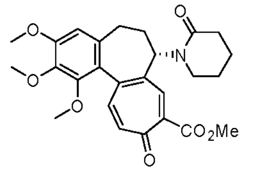

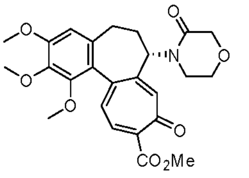
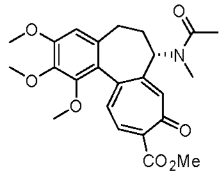
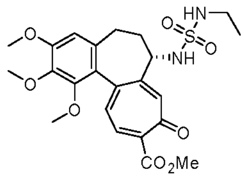
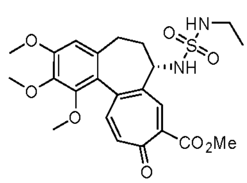

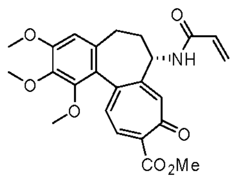 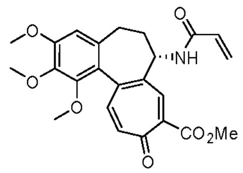 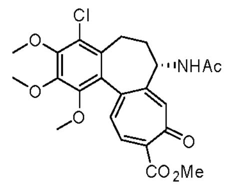 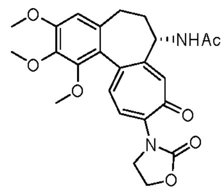

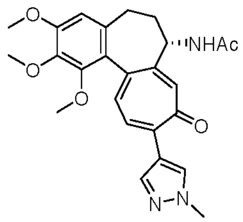 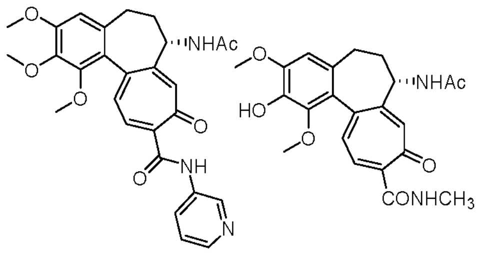 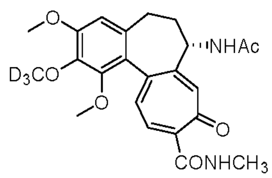

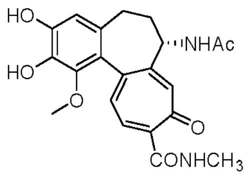 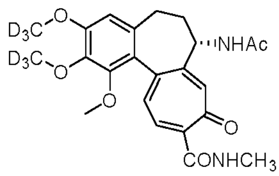 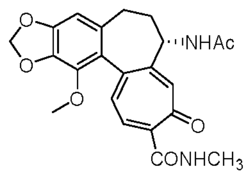 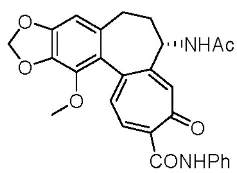

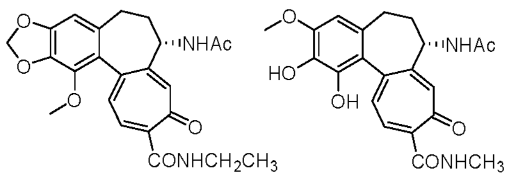 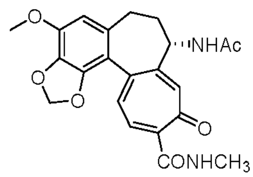 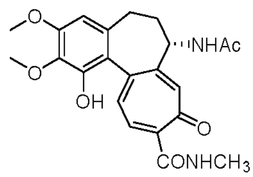

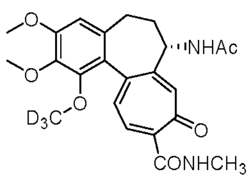 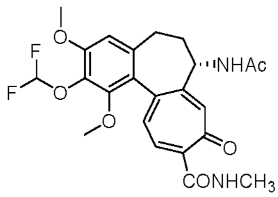 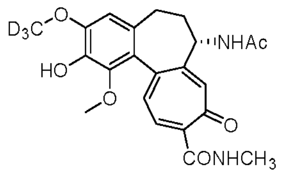

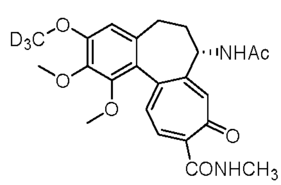 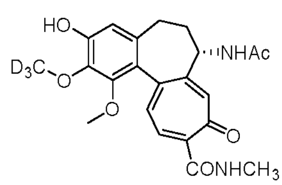 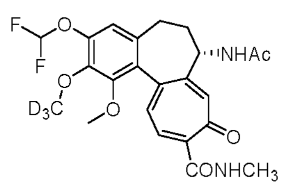

8. A pharmaceutical composition comprising the compounds, isomers or pharmaceutically acceptable salts according to any of claims 1 to 7 as active ingredient, supplemented by pharmaceutically acceptable excipients.

9. A use of any of the compounds, isomers or pharmaceutically acceptable salts according to any of claims 1 to 7 in the preparation of anti-inflammatory or analgesic drugs.

10. A use of any of the compounds, isomers or pharmaceutically acceptable salts according to any of claims 1 to 7 in the preparation of drugs for the treatment of arthritis, or in the preparation of drugs for treating acute gouty arthritis, rheumatoid arthritis, reducing inflammatory factors, treating inflammatory storms or coronavirus pneumonia.

**INTERNATIONAL SEARCH REPORT**

International application No.
**PCT/CN2022/090947**

**A. CLASSIFICATION OF SUBJECT MATTER**

C07D 307/20(2006.01)i; C07D 295/182(2006.01)i; C07D 265/32(2006.01)i; C07D 295/155(2006.01)i; C07D 257/04(2006.01)i; C07F 5/02(2006.01)i; C07D 207/16(2006.01)i; C07D 249/08(2006.01)i; C07D 207/27(2006.01)i; C07D 263/24(2006.01)i; C07C 311/07(2006.01)i; C07C 311/46(2006.01)i; C07C 307/02(2006.01)i; C07C 305/20(2006.01)i; C07C 307/08(2006.01)i; C07C 233/32(2006.01)i; C07C 233/52(2006.01)i; C07C 255/47(2006.01)i; C07C 237/24(2006.01)i; C07C 255/19(2006.01)i; C07C 275/26(2006.01)i; C07C 271/24(2006.01)i; C07C 271/34(2006.01)i; A61P 29/00(2006.01)i; A61P 19/02(2006.01)i; A61P 31/14(2006.01)i; A61K 31/5375(2006.01)i; A61K 31/421(2006.01)i; A61K 31/41(2006.01)i; A61K 31/27(2006.01)i; A61K 31/277(2006.01)i; A61K 31/255(2006.01)i; A61K 31/18(2006.01)i; A61K 31/196(2006.01)i; A61K 31/165(2006.01)i; A61K 31/40(2006.01)i; A61K 31/495(2006.01)i; A61K 31/64(2006.01)i; A61K 31/69(2006.01)i; A61K 31/341(2006.01)i; A61K 31/17(2006.01)i

According to International Patent Classification (IPC) or to both national classification and IPC

**B. FIELDS SEARCHED**

Minimum documentation searched (classification system followed by classification symbols)

C07D C07F C07C A61P A61K

Documentation searched other than minimum documentation to the extent that such documents are included in the fields searched

Electronic data base consulted during the international search (name of data base and, where practicable, search terms used)

CNABS; DWPI; VEN; CJFD; CNKI; CNTXT; USTXT; WOTXT; EPTXT; STN-REG; STN-CAPLUS; ISI-Web of Science; 万方, WANFANG; 超星读秀, DUXIU: 江苏新元素医药科技有限公司, 史东方, 傅长金, 杨艳, 李海明, 秋水仙碱, 炎症, 抗炎, 关节炎, 肺炎, 镇痛, 止痛, 结构式(I), structural formula (I), 结构式(II), structural formula (II), inflammatory, +inflammatory, analgesi+, gout

**C. DOCUMENTS CONSIDERED TO BE RELEVANT**

| Category* | Citation of document, with indication, where appropriate, of the relevant passages | Relevant to claim No. |
|---|---|---|
| PX | CN 114656429 A (JIANGSU ATOM BIOSCIENCE & PHARMACEUTICAL CO., LTD.) 24 June 2022 (2022-06-24)<br>claims 1-10 | 1-10 |
| X | KUREK, J. et al. "Cytotoxic, Analgesic and Anti-Inflammatory Activity of Colchicine and its C-10 Sulfur Containing Derivatives"<br>*Scientific Reports,* Vol. 11, No. 1, 27 April 2021 (2021-04-27),<br>see abstract, and figure 1, Results | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 July 2022** | **27 July 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/090947**

**C. DOCUMENTS CONSIDERED TO BE RELEVANT**

| Category* | Citation of document, with indication, where appropriate, of the relevant passages | Relevant to claim No. |
|---|---|---|
| X | PARE, G. et al. "The Development of A Targeted and More Potent, Anti-Inflammatory Derivative of Colchicine: Implications for Gout" *Biochemical Pharmacology,* Vol. 180, 26 June 2020 (2020-06-26), see pp. 3-11 | 1-10 |
| A | CN 102741223 A (MUTUAL PHARMACEUTICAL COMPANY, INC.) 17 October 2012 (2012-10-17) entire document | 1-10 |
| A | GHAWANMEH, A. A. et al. "Recent Developments on (-)-Colchicine Derivatives: Synthesis and Structure-Activity Relationship" *European Journal of Medicinal Chemistry,* Vol. 185, 18 October 2019 (2019-10-18), pp. 1-16 | 1-10 |

Form PCT/ISA/210 (second sheet) (January 2015)

**INTERNATIONAL SEARCH REPORT**
**Information on patent family members**

International application No.
**PCT/CN2022/090947**

| Patent document cited in search report | Publication date (day/month/year) | Patent family member(s) | Publication date (day/month/year) |
| --- | --- | --- | --- |
| CN 114656429 A | 24 June 2022 | None | |
| CN 102741223 A | 17 October 2012 | US 2011184061 A1 | 28 July 2011 |
| | | WO 2011091114 A2 | 28 July 2011 |
| | | WO 2011091114 A3 | 10 November 2011 |
| | | EP 2526086 A2 | 28 November 2012 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ZHU Y ; PANDYA BJ ; CHOI HK.** Prevalence of Gout and Hyperuricemia in the US General Population: The National Health and Nutrition Examination Survey 2007-2008[J]. *Arthritis Rheum,* 2011, vol. 63 (10), 3136-3141
- **LIU R ; HAN C ; WU D et al.** Prevalence of hyperuricemia and gout in mainland China from 2000 to 2014: A systematic review and meta-analysis[J]. *Biomed Research International,* 2015, 1-12
- **KHANNA D ; FITZGERALD JD ; KHANNA PP et al.** American College of Rheumatology Guidelines for Management of Gout. Part 1: Systematic Nonpharmacologic and Pharmacologic Therapeutic Approaches to Hyperuricemia[J]. *Arthritis Care & Research,* 2012, vol. 64 (10), 1432-1446
- **RICHETTE P ; BARDIN T.** *Gout. Lancet,* 2010, vol. 375 (9711), 318-328
- **SCHLESINGER N.** Difficult-to-treat gouty arthritis: a disease warranting better management. *Drugs,* 2011, vol. 71 (11), 1413-1439
- Chinese Society of Endocrinology. Guidelines for the diagnosis and management of hyperuricemia and gout in China 2019. *Chinese Journal Endocrinology and Metabolism,* 2019, vol. 36 (1
- **KEENAN RT ; O'BRIEN WR ; LEE KH et al.** Prevalence of contraindications and prescription of pharmacologic therapies for gout[J. *American Journal of Medicine,* 2011, vol. 124 (2), 155-163
- **KHANNA D ; KHANNA PP ; FITZGERALD JD et al.** American College of Rheumatology guidelines for management of gout. Part 2: therapy and antiinflammatory prophylaxis of acute gouty arthritis [J]. *Arthritis care & research,* 2012, vol. 64 (10), 1447-1461
- **WANG SIQI ; YU BIN ; ZHANG XIAOHAI et al.** Correlation analysis of pain and inflammatory factors in acute gout patients [J]. *Medical Theory & Practice,* 2021, vol. 34 (3), 371-373
- **HE Y ; GAN W ; ZHANG M et al.** Evaluation of clinical biochemical indexes in the progression and treatment of COVID-19[J]. *Journal of Laboratory Medicine,* 2020, vol. 41, 906-909
- **ZHANG XIMU ; LI TANSHI.** Cytokine storm and immunotherapy in post-influenza pneumonia [J]. *Medical Review,* 2020, vol. 26 (6), 1142-1146
- **WILK AJ ; RUSTAGI A ; ZHAO NQ et al.** A single cell atlas of the peripheral immune response in patients with severe COVID-19[J]. *Nature medicine,* 2020, vol. 26 (7), 1070-1076
- **HUANG C ; WANG Y ; LI X et al.** Clinical features of patients infected with 2019 novel coronavirus in Wuhan, China[J]. *Lancet,* 2020, vol. 395 (10223), 497-506
- **CLARK DR ; JONATHAN EM ; KENNETH JB et al.** Clinical evidence does not support corticosteroid treatment for 2019-nCoV lung injury[J]. *Lancet,* 2020, vol. 395, 473-475
- **TARDIF JC ; BOUABDALLAOUI N ; L 'ALLIER PL et al.** Efficacy of Colchicine in Non-Hospitalized Patients with COVID-19[J]. *MedRxiv,* 2021